# EUROPEAN PATENT SPECIFICATION

(11) **EP 3 472 147 B1**
(45) Date of publication and mention of the grant of the patent: **17.06.2020**
(21) Application number: 17729500.3
(22) Date of filing: 15.06.2017
(51) Int. Cl.: C07D 401/04, A61K 31/496, A61P 35/00

(54) **AZABENZIMIDAZOLE DERIVATIVES AS PI3K BETA INHIBITORS**
AZABENZIMIDALZOLDERIVATE ALS PI3K-BETA-HEMMER
DÉRIVÉS D'AZABENZIMIDAZOLE EN TANT QU'INHIBITEURS DE PI3K BETA

(30) Priority: 16.06.2016 EP 16174712
(43) Date of publication of application: 24.04.2019
(73) Proprietor: Janssen Pharmaceutica NV, 2340 Beerse (BE)
(72) Inventor: PILATTE, Isabelle, Noëlle, Constance, 92787 Issy-les Moulineaux Cedex 9 (FR); QUEROLLE, Olivier, Alexis, Georges, 92787 Issy-les Moulineaux Cedex 9 (FR); ANGIBAUD, Patrick, René, 92787 Issy-les Moulineaux Cedex 9 (FR); BERTHELOT, Didier, Jean-Claude, 92787 Issy-les Moulineaux Cedex 9 (FR); COUPA, Sophie, 92787 Issy-les Moulineaux Cedex 9 (FR); DEMESTRE, Christophe, Gabriel, Marcel, 92787 Issy-les Moulineaux Cedex 9 (FR); MEERPOEL, Lieven, 2340 Beerse (BE); MERCEY, Guillaume, Jean, Maurice, 92787 Issy-les Moulineaux Cedex 9 (FR); MEVELLEC, Laurence, Anne, 92787 Issy-les Moulineaux Cedex 9 (FR); MEYER, Christophe, 92787 Issy-les Moulineaux Cedex 9 (FR); PASQUIER, Elisabeth, Thérèse, Jeanne, 92787 Issy-les Moulineaux Cedex 9 (FR); PONCELET, Virginie, Sophie, 92787 Issy-les Moulineaux Cedex 9 (FR)
(74) Representative: Lenaerts, Philip
(86) International application number: PCT/EP2017/064672
(87) International publication number: WO 2017/216293

(56) References cited:
- WO-A1-2009/045175
- WO-A1-2013/095761
- EUNA YOO ET AL: "Structure-activity relationships in Toll-like receptor 7 agonistic 1H-imidazo[4,5-c]pyridines", ORGANIC & BIOMOLECULAR CHEMISTRY, vol. 11, no. 38, 15 August 2013 (2013-08-15), page 6526, XP055165875, ISSN: 1477-0520, DOI: 10.1039/c3ob40816g

## Description

### Field of the Invention

The present invention relates to azabenzimidazole derivatives useful as PI3Kβ inhibitors. The invention further relates to pharmaceutical compositions comprising said compounds as an active ingredient as well as the use of said compounds as a medicament.

### Background of the invention

There are three classes of phosphoinositide-3-kinases (PI3Ks): class I, class II and class III. Class I PI3Ks are the most associated with human cancer [K.D Courtney, R.B. Corcoran and J.A. Engelman (2010), Journal of Clinical Oncology., 28; 1075]. The class I phosphoinositide-3-kinases (PI3Ks) are divided into 2 subclasses: class I_{A}, composed of a p110 catalytic subunit (p110a, p110b or p110d) and a p85 regulatory subunit (p85a, p55a and p50a, p85b or p55g) and class 1_{B} PI3K represented by the p110g catalytic subunit and the p101 and p84 regulatory subunits [B. Vanhaesebroeck and M.D. Waterfield (1999) Experimental Cell Research., 253, 239-254]. The class I_{A} PI3Ks are activated in a variety of solid and non-solid tumors via mutation or deletion of the tumor suppressor PTEN (phosphatase and tensin homolog) or in the case of p110a by activating mutations [K.D Courtney, R.B. Corcoran and J.A. Engelman (2010), Journal of Clinical Oncology., 28; 1075]. PI3Ks can also be activated by receptor tyrosine kinases (RTKs); p110b can be activated by G-protein coupled receptors [K.D Courtney, R.B. Corcoran and J.A. Engelman (2010), Journal of Clinical Oncology., 28; 1075]. Once activated the phosphoinositide-3-kinases catalyze the phosphorylation of phosphatidyl 4,5-diphosphate leading to the generation of phosphatidyl, 3, 4, 5-triphosphate (PIP3) [Zhao L., Vogt P. K.(2008) Oncogene 27, 5486-5496]. PTEN antagonizes the activity of the PI3Ks through the dephosphorylation of PIP3 [Myers M. P., Pass I., Batty I. H., Van der Kaay J., Stolarov J. P., Hemmings B. A., Wigler M. H., Downes C. P., Tonks N. K.(1998) Proc. Natl. Acad. Sci. U.S.A. 95, 13513-13518]. The PIP3 generated by activation of PI3K or sustained by the inactivation of PTEN binds to a subset of lipid-binding domains in downstream targets such as the pleckstrin homology domain of the oncogene Akt thereby recruiting it to the plasma membrane [Stokoe D., Stephens L. R., Copeland T., Gaffney P. R., Reese C. B., Painter G. F., Holmes A. B., McCormick F., Hawkins P. T. (1997) Science 277, 567-570]. Once at the plasma membrane Akt phosphorylates several effector molecules that are involved in numerous biologically relevant processes such as metabolism, differentiation, proliferation, longevity and apoptosis [D. R. Calnan and A. Brunet (2008) Oncogene 27; 2276)].

Several studies suggest a key role for p110b in PTEN-deficient tumors. For example the genetic knockout of p110b, but not p110a, is able to block tumor formation and Akt activation driven by Pten loss in the anterior prostate in a mouse model [Jia S, Liu Z, Zhang S, Liu P, Zhang L, Lee SH, Zhang J, Signoretti S, Loda M, Roberts TM, Zhao JJ. Nature 2008; 454:776-9]. Furthermore other studies have shown that a subset of PTEN-deficient human tumor cell lines is sensitive to inactivation of p110b rather than p110a [Wee S, Wiederschain D, Maira SM, Loo A, Miller C, deBeaumont R, Stegmeier F, Yao YM, Lengauer C (2008) Proc. Natl. Acad. Sci (USA); 105 13057]. PTEN deficiency either by genetic inactivation or reduced expression frequently occurs in human cancers such as GBM, endometrial, lung, breast cancers and prostate cancer among others [K.D Courtney, R.B. Corcoran and J.A. Engelman (2010), Journal of Clinical Oncology., 28; 1075].

These studies suggest that treatment of PTEN-deficient cancer with agents that inhib p110b may be therapeutically beneficial. In addition to its role in cancer, p110b may be a target for antithrombotic therapy. It has been reported in mouse models that PI3Kb inhibition can prevent stable integrin a_{IIb}b₃ adhesion contacts that eliminates occulusive thrombus formation without prolongation of bleed time [S. P. Jackson et al. (2005) Nature Medicine., 11, 507-514].

Furthermore, the phosphatidylinositol-4,5-bisphosphate 3-kinase (PI3K)/AKT pathway is frequently activated during prostate cancer (PCa) progression through loss or mutation of the phosphatase and tensin homolog (PTEN) gene. Following the androgen receptor (AR)pathway, it is the second major driver of PCa growth. Combination with hormonal therapy improved efficacy of PI3K/AKT-targeted agents in PTEN-negative PCa models. Upregulation of AR-target genes upon PI3K/AKT inhibition suggests a compensatory crosstalk between the PI3K-AR pathways which, for optimal efficacy treatment, could require cotargeting of the AR axis [Marques RB, et al., High Efficacy of Combination Therapy Using PI3K/AKT Inhibitors with Androgen Deprivation in Prostate Cancer Preclinical Models. Eur Urol (2014), http://dx.doi.org/10.1016/j.eururo.2014.08.053]. Therefore PI3Kβ inhibitors can be advantageously combined with anti-androgen therapies including androgen receptor antagonists and inhibitors of androgen biosynthesis in PTEN-negative prostate cancers.

WO 2012/116237 discloses heterocyclic entitites that modulate PI3 kinase activity.

WO 2011/123751 describes heterocyclic compounds as selective inhibitors of PI3K activity.

WO 2011/022439 discloses heterocyclic entities that modulate PI3 kinase activity.

WO 2008/014219 describes thiozolidinedione derivatives as PI3 kinase inhibitors.

WO 2013/028263 relates to pyrazolopyrimidine derivatives as PI3 kinase inhibitors.

WO 2012/047538 relates to benzimidazole derivatives as PI3 kinase inhibitors.

WO 2013/095761 relates to imidazopyridine derivatives as PI3 kinase inhibitors.

US 2013/0157977 relates to benzimidazole boronic acid derivatives as PI3 kinase inhibitors.

WO 2009/021083 describes quinoxaline derivatives as PI3 kinase inhibitors.

WO 2007/103756 describes the preparation of thiazolones for use as PI3 kinase inhibitors.

WO 2011/041399 describes benzimidazolyl (morpholinyl)purines and related compounds as PI3Kδ inhibitors and their preparation and use for the treatment of PI3K-mediated diseases.

WO 2009/088990 describes the preparation of pyrazolo pyrimidines and other heterocyclic compounds as therapeutic PI3 kinase modulators.

Euna Yoo, et al, Organic & and Biomolecular Chemistry, vol 11(38),1.1. 2013 pp 6526-6545 disclose azabenzimidazole derivatives similar to the present ones which however concern TLR7 agonistic agents instead of the present PI3K beta inhibitors.

There is thus a strong need for novel PI3Kβ kinase inhibitors thereby opening new avenues for the treatment or prevention of cancer, in particular PTEN-deficient cancers, more in particular prostate cancer. It is accordingly an object of the present invention to provide such compounds.

### Summary of the invention

It has been found that the compounds of the present invention are useful as PI3Kβ inhibitors. The compounds according to the invention and compositions thereof, may be useful for the treatment or prevention, in particular for the treatment, of diseases such as cancer, autoimmune disorders, cardiovascular diseases, inflammatory diseases, neurodegenerative diseases, allergy, pancreatitis, asthma, multiorgan failure, kidney diseases, platelet aggregation, sperm motility, transplantation rejection, graft rejection, lung injuries and the like.

This invention concerns compounds of Formula (I) 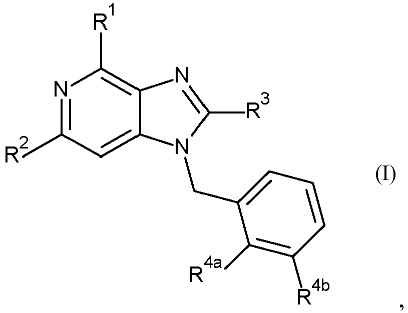 tautomers and stereoisomeric forms thereof, wherein
R¹ represents hydrogen, -C=OOH, -C=ONH₂, -NH₂, 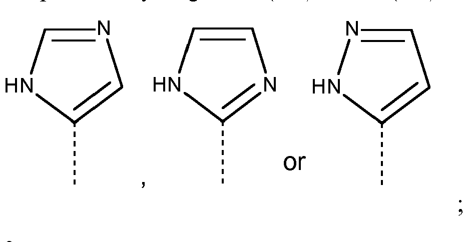
R² represents 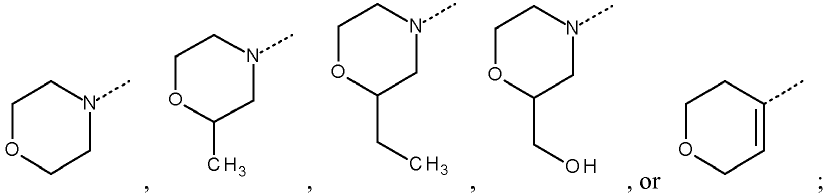
R³ represents C₁₋₄alkyl; -CH(OH)-CH₂-R^{q}; C₁₋₄alkyl substituted on the same carbon atom with one -OH and with one Het¹; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 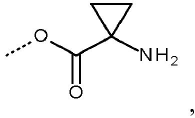 -NH-C₁₋₄alkyl-OH, Het¹, -O-C(=O)-C₁₋₄alkyl-Het¹, -C(=O)-Het¹, and -NH-C(=O)-Het¹;
R^{q} represents Het¹, fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -O-(C=O)-CH(NH₂)-C₁₋₄alkyl, -O-(C=O)-CH(NH₂)-C₁₋₄alkyl-Ar, 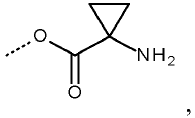 or -NH-C₁₋₄alkyl-OH;
Ar represents phenyl optionally substituted with one hydroxy;
R^{4a} represents hydrogen, C₁₋₄alkyl, Het^{a}, or C₁₋₄alkyl substituted with one or more substituents each independently selected from the group consisting of -OH, -NR⁵R⁶ and Het^{a};
R^{4b} represents hydrogen, halo, C₁₋₄alkyl, or C₁₋₄alkyl substituted with one or more halo substituents;
or R^{4a} and R^{4b} are taken together to form together with the phenyl ring to which they are attached a structure of Formula (a-1), (a-2), (a-3), (a-4) or (a-5): 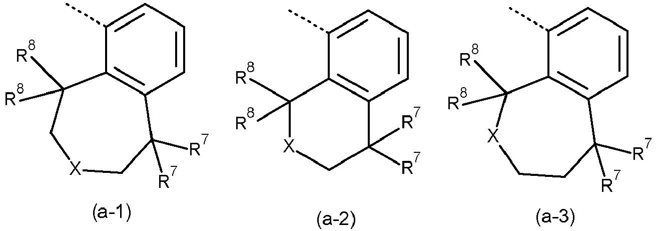 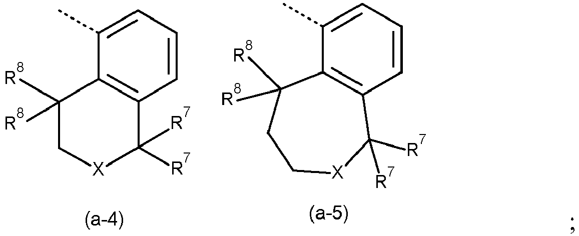
X represents -NH-, -O-, -N(C₁₋₃alkyl)-, or -N(hydroxyC₁₋₃alkyl)-;
both R⁷ substituents are the same and are selected from the group consisting of hydrogen, fluoro and methyl; or both R⁷ substituents are taken together to form together with the common carbon atom to which they are attached a cyclopropyl, cyclobutyl or oxetanyl;
both R⁸ substituents are the same and are selected from the group consisting of hydrogen and methyl; or both R⁸ substituents are taken together to form together with the common carbon atom to which they are attached a cyclopropyl, cyclobutyl or oxetanyl;
R⁵ represents hydrogen, C₁₋₆alkyl, or C₁₋₆alkyl substituted with one -OH;
R⁶ represents hydrogen, C₁₋₆alkyl, or C₁₋₆alkyl substituted with one -OH;
Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of halo, -NH₂, C₁₋₄alkyl, -S(=O)₂-C₁₋₆alkyl, -C₁₋₄alkyl-S(=O)₂-C₁₋₆alkyl, hydroxyl, C₁₋₄alkyloxy, fluoro, cyano and C₁₋₄alkyl substituted with one hydroxy; or two substituents on the same carbon atom of said 4-, 5- or 6-membered saturated heterocyclyl are taken together to form together with the common carbon atom to which they are attached Ring A;
Ring A represents cyclobutyl, cyclopentyl, cyclohexyl or a 4-, 5- or 6-membered saturated heterocyclyl containing at least one heteroatom each independently selected from O, S, S(=O)ₚ and N; said cyclobutyl, cyclopentyl, cyclohexyl or 4-, 5- or 6-membered saturated heterocyclyl is optionally substituted with one or two C₁₋₄alkyl substituents, with one C₁₋₄alkyl and one hydroxy substituent, or with one hydroxy substituent;
each Het^{a} independently represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of C₁₋₄alkyl, -S(=O)₂-C₁₋₆alkyl, hydroxy, -C₁₋₄alkyl-S(=O)₂-C₁₋₆alkyl, and C₁₋₄alkyl substituted with one hydroxy; or two substituents on the same carbon atom of said 4-, 5- or 6-membered saturated heterocyclyl are taken together to form together with the common carbon atom to which they are attached Ring B;
Ring B represents cyclobutyl, cyclopentyl, cyclohexyl or a 4-, 5- or 6-membered saturated heterocyclyl containing at least one heteroatom each independently selected from O, S, S(=O)ₚ and N; said cyclobutyl, cyclopentyl, cyclohexyl or 4-, 5- or 6-membered saturated heterocyclyl is optionally substituted with one or two C₁₋₄alkyl substituents, with one C₁₋₄alkyl and one hydroxy substituent, or with one hydroxy substituent;
p represents 1 or 2;
and the N-oxides, the pharmaceutically acceptable addition salts, and the solvates thereof.

The present invention also concerns methods for the preparation of compounds of the present invention and pharmaceutical compositions comprising them.

The compounds of the present invention were found to inhibit PI3Kβ per se or can undergo metabolism to a (more) active form in vivo (prodrugs), and therefore may be useful in the treatment or prevention, in particular in the treatment, of diseases such as cancer, autoimmune disorders, cardiovascular diseases, inflammatory diseases, neurodegenerative diseases, allergy, pancreatitis, asthma, multiorgan failure, kidney diseases, platelet aggregation, sperm motility, transplantation rejection, graft rejection, lung injuries and the like.

In view of the aforementioned pharmacology of the compounds of Formula (I) and N-oxides, pharmaceutically acceptable addition salts, and solvates thereof, it follows that they may be suitable for use as a medicament.

In particular the compounds of Formula (I) and N-oxides, pharmaceutically acceptable addition salts, and solvates thereof, may be suitable in the treatment or prevention, in particular in the treatment, of cancer.

The present invention also concerns the use of compounds of Formula (I) and N-oxides, pharmaceutically acceptable addition salts, and solvates thereof, for the manufacture of a medicament for the inhibition of PI3Kβ, for the treatment or prevention of cancer. The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Detailed description

When describing the compounds of the invention, the terms used are to be construed in accordance with the following definitions, unless a context dictates otherwise.

When any variable occurs more than one time in any constituent or in any formula (e.g. Formula (I)), its definition in each occurence is independent of its definition at every other occurrence.

Whenever the term "substituted" is used in the present invention, it is meant, unless otherwise is indicated or is clear from the context, to indicate that one or more hydrogens, in particular from 1 to 3 hydrogens, preferably 1 or 2 hydrogens, more preferably 1 hydrogen, on the atom or radical indicated in the expression using "substituted" are replaced with a selection from the indicated group, provided that the normal valency is not exceeded, and that the substitution results in a chemically stable compound, i.e. a compound that is sufficiently robust to survive isolation to a useful degree of purity from a reaction mixture, and formulation into a therapeutic agent.

When two or more substituents are present on a moiety they may, unless otherwise is indicated or is clear from the context, replace hydrogens on the same atom or they may replace hydrogen atoms on different atoms in the moiety.

It will be clear for the skilled person that, unless otherwise is indicated or is clear from the context, a substituent on a heterocyclyl group may replace any hydrogen atom on a ring carbon atom or on a ring heteroatom.

The prefix "C_{x-y}" (where x and y are integers) as used herein refers to the number of carbon atoms in a given group. Thus, a C₁₋₆alkyl group contains from 1 to 6 carbon atoms, a C₁₋₄alkyl group contains from 1 to 4 carbon atoms, a C₁₋₃alkyl group contains from 1 to 3 carbon atoms, a C₃₋₆cycloalkyl group contains from 3 to 6 carbon atoms, and so on.

The term "halo" as a group or part of a group is generic for fluoro, chloro, bromo, iodo unless otherwise is indicated or is clear from the context.

The term "C₁₋₆alkyl" as a group or part of a group refers to a hydrocarbyl radical of Formula CₙH₂ₙ₊₁ wherein n is a number ranging from 1 to 6. C₁₋₆alkyl groups comprise from 1 to 6 carbon atoms, preferably from 1 to 4 carbon atoms, more preferably from 1 to 3 carbon atoms, still more preferably 1 to 2 carbon atoms. Alkyl groups may be linear or branched and may be substituted as indicated herein. When a subscript is used herein following a carbon atom, the subscript refers to the number of carbon atoms that the named group may contain. Thus, for example, C₁₋₆alkyl includes all linear, or branched alkyl groups with between 1 and 6 carbon atoms, and thus includes such as for example methyl, ethyl, *n*-propyl, *i*-propyl, 2-methyl-ethyl, butyl and its isomers (e.g. *n*-butyl, *iso*butyl and *tert*-butyl), pentyl and its isomers, hexyl and its isomers, and the like.

The term "C₁₋₄alkyl" as a group or part of a group refers to a hydrocarbyl radical of Formula CₙH₂ₙ₊₁ wherein n is a number ranging from 1 to 4. C₁₋₄alkyl groups comprise from 1 to 4 carbon atoms, preferably from 1 to 3 carbon atoms, more preferably 1 to 2 carbon atoms. C₁₋₄alkyl groups may be linear or branched and may be substituted as indicated herein. When a subscript is used herein following a carbon atom, the subscript refers to the number of carbon atoms that the named group may contain. C₁₋₄alkyl includes all linear, or branched alkyl groups with between 1 and 4 carbon atoms, and thus includes methyl, ethyl, *n*-propyl, *i*-propyl, 2-methyl-ethyl, butyl and its isomers (e.g. *n*-butyl, *iso*butyl and *tert*-butyl), and the like.

The term "C₁₋₃alkyl" as a group or part of a group refers to a hydrocarbyl radical of Formula CₙH₂ₙ₊₁ wherein n is a number ranging from 1 to 3. C₁₋₃alkyl groups comprise from 1 to 3 carbon atoms, preferably 1 to 2 carbon atoms. C₁₋₃alkyl groups may be linear or branched and may be substituted as indicated herein. When a subscript is used herein following a carbon atom, the subscript refers to the number of carbon atoms that the named group may contain. C₁₋₃alkyl includes all linear, or branched alkyl groups with between 1 and 3 carbon atoms, and thus includes methyl, ethyl, *n*-propyl, *i*-propyl, 2-methyl-ethyl, and the like.

In an embodiment the expression 'at least one heteroatom' is restricted to '1, 2 or 3 heteroatoms', in a particular embodiment to '1 or 2 heteroatoms', in a more particular embodiment to '1 heteroatom'.

A 4-, 5- or 6-membered saturated heterocyclyl containing at least one heteroatom each independently selected from O, S, S(=O)ₚ and N (as occuring for example in the definitions of Het¹, Het^{a}, Ring A and Ring B); in a particular embodiment is a 4-, 5- or 6-membered saturated heterocyclyl containing 1, 2 or 3 heteroatoms selected from O, S, S(=O)ₚ and N; in a more particular embodiment a 4-, 5- or 6-membered saturated heterocyclyl containing 1 or 2 heteroatoms selected from O, S, S(=O)ₚ and N.

Examples of a 4-, 5- or 6-membered saturated heterocyclyl containing at least one heteroatom each independently selected from O, S, S(=O)ₚ and N, include, but are not limited to azetidinyl, morpholinyl, piperidinyl, pyrrolidinyl, 1,1-dioxido-thietanyl, 1,1-dioxido-thiomorpholinyl, piperazinyl, dioxolanyl, oxazolidinyl, oxetanyl, tetrahydrofuranyl, and the like.

Het¹ and Het^{a} may be attached to the remainder of the molecule of Formula (I) through any available ring carbon atom or ring heteroatom as appropriate, if not otherwise specified.

It will be clear that when two substituents on the same carbon atom in the Het¹ or Het^{a} definition are taken together to form together with the common carbon atom to which they are attached Ring A or Ring B respectively, a spiro moiety is formed.

For example, when Het¹ represents 1-piperidinyl wherein two substituents on the carbon atom in position β are taken together to form together with the common carbon atom to which they are attached ring A, the following spiro moiety is formed: 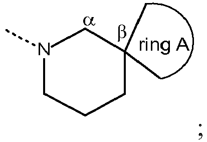 in particular if in the above example ring A represents 3-azetidinyl, the following spiro moiety is formed:

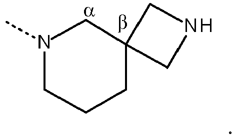

Examples of such spiro moieties, include, but are not limited to 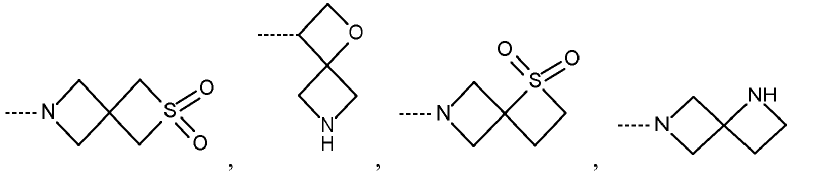 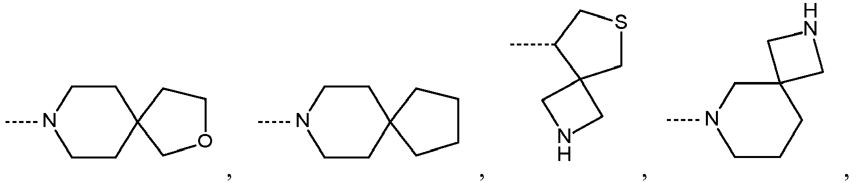 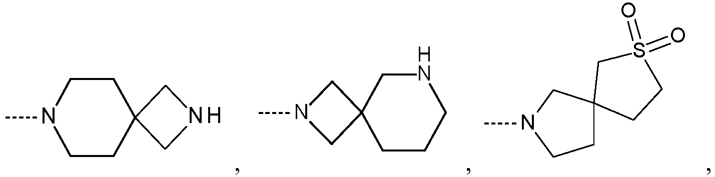 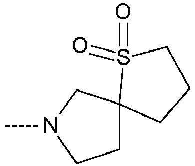 and the like.

Whenever substituents are represented by chemical structure, "---" represents the bond of attachment to the remainder of the molecule of Formula (I).

Whenever one of the ring systems, is substituted with one or more substituents, those substituents may replace, unless otherwise is indicated or is clear from the context, any hydrogen atom bound to a carbon or nitrogen atom of the ring system.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compounds of the invention" as used herein, is meant to include the compounds of Formula (I) and N-oxides, pharmaceutically acceptable addition salts, and solvates thereof.

As used herein, any chemical formula with bonds shown only as solid lines and not as solid wedged or hashed wedged bonds, or otherwise indicated as having a particular configuration (e.g. *R*, *S*) around one or more atoms, contemplates each possible stereoisomer, or mixture of two or more stereoisomers.

Hereinbefore and hereinafter, the term "compound of Formula (I)" is meant to include the stereoisomers thereof and the tautomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present invention.

Diastereomers (or diastereoisomers) are stereoisomers that are not enantiomers, i.e. they are not related as mirror images. If a compound contains a double bond, the substituents may be in the E or the Z configuration. Substituents on bivalent cyclic (partially) saturated radicals may have either the cis- or trans-configuration; for example if a compound contains a disubstituted cycloalkyl group, the substituents may be in the cis or trans configuration. Therefore, the invention includes enantiomers, atropisomers, diastereomers, racemates, E isomers, Z isomers, cis isomers, trans isomers and mixtures thereof, whenever chemically possible.

The meaning of all those terms, i.e. enantiomers, atropisomers, diastereomers, racemates, E isomers, Z isomers, cis isomers, trans isomers and mixtures thereof are known to the skilled person.

The absolute configuration is specified according to the Cahn-Ingold-Prelog system. The configuration at an asymmetric atom is specified by either R or S. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (R), this means that the compound is substantially free of the (S) isomer; when a compound of Formula (I) is for instance specified as E, this means that the compound is substantially free of the Z isomer; when a compound of Formula (I) is for instance specified as cis, this means that the compound is substantially free of the trans isomer.

Some of the compounds of Formula (I) may also exist in their tautomeric form. Such forms in so far as they may exist, are intended to be included within the scope of the present invention. It follows that a single compound may exist in both stereoisomeric and tautomeric form.

For example, it will be clear for the skilled person that when R¹ represents 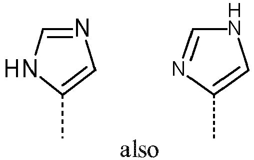 is included in the scope of the invention.

For therapeutic use, salts of the compounds of Formula (I), N-oxides and solvates thereof, are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable addition salts as mentioned hereinabove or hereinafter are meant to comprise the therapeutically active non-toxic acid and base addition salt forms which the compounds of Formula (I), N-oxides and solvates thereof, are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I), N-oxides and solvates thereof containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the hydrates and solvent addition forms which the compounds of Formula (I) are able to form, as well as N-oxides and pharmaceutically acceptable addition salts thereof. Examples of such forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and N-oxides, pharmaceutically acceptable addition salts, and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

In the framework of this application, an element, in particular when mentioned in relation to a compound of Formula (I), comprises all isotopes and isotopic mixtures of this element, either naturally occurring or synthetically produced, either with natural abundance or in an isotopically enriched form. Radiolabelled compounds of Formula (I) may comprise a radioactive isotope selected from the group of ²H, ³H, ¹¹C, ¹⁸F, ¹²²I, ¹²³I, ¹²⁵I, ¹³¹I, ⁷⁵Br, ⁷⁶Br, ⁷⁷Br and ⁸²Br. Preferably, the radioactive isotope is selected from the group of ²H, ³H, ¹¹C and ¹⁸F. More preferably, the radioactive isotope is ²H. In particular, deuterated compounds are intended to be included within the scope of the present invention.

In an embodiment, the present invention concerns novel compounds of Formula (I), tautomers and stereoisomeric forms thereof, wherein
R¹ represents hydrogen, -C(=O)OH, -C(=O)NH₂, -NH2, 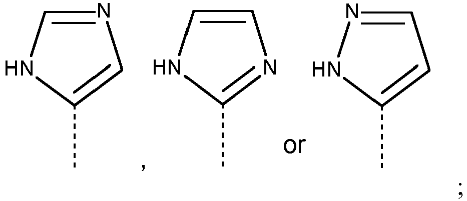
R² represents 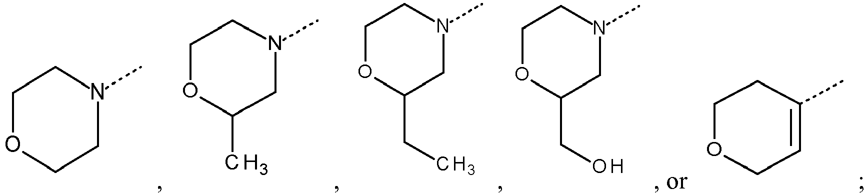
R³ represents C₁₋₄alkyl; C₁₋₄alkyl substituted on the same carbon atom with one -OH and with one Het¹; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 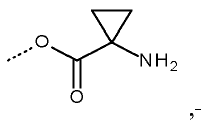 -NH-C₁₋₄alkyl-OH, Het¹, -O-C(=O)-C₁₋₄alkyl-Het¹, -C(=O)-Het¹, and -NH-C(=O)-Het¹;
R^{4a} represents hydrogen, C₁₋₄alkyl, or C₁₋₄alkyl substituted with one or more substituents each independently selected from the group consisting of -OH, and -NR⁵R⁶;
R^{4b} represents hydrogen, halo, C₁₋₄alkyl, or C₁₋₄alkyl substituted with one or more halo substituents;
or R^{4a} and R^{4b} are taken together to form together with the phenyl ring to which they are attached a structure of Formula (a-1), (a-2), (a-3), (a-4) or (a-5);
X represents -NH-, -O-, -N(C₁₋₃alkyl)-, or -N(hydroxyC₁₋₃alkyl)-;
both R⁷ substituents are hydrogen;
both R⁸ substituents are hydrogen;
R⁵ represents hydrogen, C₁₋₆alkyl, or C₁₋₆alkyl substituted with one -OH;
R⁶ represents hydrogen, C₁₋₆alkyl, or C₁₋₆alkyl substituted with one -OH;
Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of halo, -NH₂, C₁₋₄alkyl, -S(=O)₂-C₁₋₆alkyl, -C₁₋₄alkyl-S(=O)₂-C₁₋₆alkyl, hydroxyl, C₁₋₄alkyloxy, fluoro, cyano and C₁₋₄alkyl substituted with one hydroxy; or two substituents on the same carbon atom of said 4-, 5- or 6-membered saturated heterocyclyl are taken together to form together with the common carbon atom to which they are attached Ring A;
Ring A represents cyclobutyl, cyclopentyl, cyclohexyl or a 4-, 5- or 6-membered saturated heterocyclyl containing at least one heteroatom each independently selected from O, S, S(=O)ₚ and N; said cyclobutyl, cyclopentyl, cyclohexyl or 4-, 5- or 6-membered saturated heterocyclyl is optionally substituted with one or two C₁₋₄alkyl substituents, with one C₁₋₄alkyl and one hydroxy substituent, or with one hydroxy substituent;
p represents 1 or 2;
and the N-oxides, the pharmaceutically acceptable addition salts, and the solvates thereof.

In an embodiment, the present invention concerns novel compounds of Formula (I), tautomers and stereoisomeric forms thereof, wherein
R¹ represents hydrogen or -NH₂;
R² represents 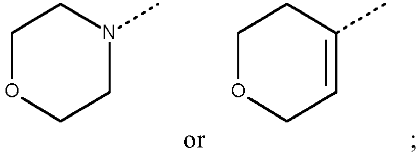
R³ represents C₁₋₄alkyl; or C₁₋₄alkyl substituted with one substituent selected from the group consisting of -OH and Het¹;
R^{4a} represents C₁₋₄alkyl;
R^{4b} represents halo, C₁₋₄alkyl, or C₁₋₄alkyl substituted with one or more halo substituents;
or R^{4a} and R^{4b} are taken together to form together with the phenyl ring to which they are attached a structure of Formula (a-2);
X represents -N(C₁₋₃alkyl)-, or -N(hydroxyC₁₋₃alkyl)-;
both R⁷ substituents are hydrogen;
both R⁸ substituents are hydrogen;
Het¹ represents a 4-, 5- or 6-membered saturated heterocyclyl containing at least one heteroatom each independently selected from S(=O)ₚ and N; said 4-, 5- or 6-membered saturated heterocyclyl is optionally substituted with one or two hydroxyl substituents; or two substituents on the same carbon atom of said 4-, 5- or 6-membered saturated heterocyclyl are taken together to form together with the common carbon atom to which they are attached Ring A;
Ring A represents cyclobutyl optionally substituted with one hydroxy substituent;
p represents 2;
and the N-oxides, the pharmaceutically acceptable addition salts, and the solvates thereof.

In an embodiment, the present invention concerns novel compounds of Formula (I), tautomers and stereoisomeric forms thereof, wherein
R¹ represents hydrogen or -NH₂;
R² represents 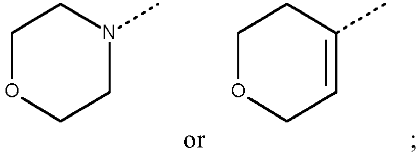
R³ represents C₁₋₄alkyl; or C₁₋₄alkyl substituted with one substituent selected from the group consisting of -OH and Het^{1a};
R^{4a} represents C₁₋₄alkyl;
R^{4b} represents halo, C₁₋₄alkyl, or C₁₋₄alkyl substituted with one or more halo substituents;
or R^{4a} and R^{4b} are taken together to form together with the phenyl ring to which they are attached a structure of Formula (a-2);
X represents -N(C₁₋₃alkyl)-, or -N(hydroxyC₁₋₃alkyl)-;
both R⁷ substituents are hydrogen;
both R⁸ substituents are hydrogen;
Het^{1a} is attached to the remainder of R³ through a ring nitrogen atom, and represents a 4-, 5- or 6-membered saturated heterocyclyl containing at least one heteroatom each independently selected from S(=O)ₚ and N; said 4-, 5- or 6-membered saturated heterocyclyl is optionally substituted with one or two hydroxyl substituents; or two substituents on the same carbon atom of said 4-, 5- or 6-membered saturated heterocyclyl are taken together to form together with the common carbon atom to which they are attached Ring A;
Ring A represents cyclobutyl optionally substituted with one hydroxy substituent;
p represents 2;
and the N-oxides, the pharmaceutically acceptable addition salts, and the solvates thereof.

Another embodiment of the present invention relates to those compounds of Formula (I) and the N-oxides, the pharmaceutically acceptable addition salts, and the solvates thereof, or any subgroup thereof as mentioned in any of the other embodiments wherein one or more of the following restrictions apply:
(i) R¹ represents hydrogen or -NH₂;
(ii) R² represents 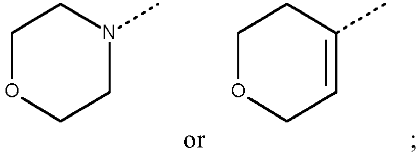
(iii) R³ represents C₁₋₄alkyl; or C₁₋₄alkyl substituted with one substituent selected from the group consisting of -OH and Het¹;
(iv)
   R^{4a} represents C₁₋₄alkyl;
   R^{4b} represents halo, C₁₋₄alkyl, or C₁₋₄alkyl substituted with one or more halo substituents;
   or R^{4a} and R^{4b} are taken together to form together with the phenyl ring to which they are attached a structure of Formula (a-2);
(v) X represents -N(C₁₋₃alkyl)-, or -N(hydroxyC₁₋₃alkyl)-;
(vi) both R⁷ substituents are hydrogen;
(vii) both R⁸ substituents are hydrogen;
(viii) Het¹ represents a 4-, 5- or 6-membered saturated heterocyclyl containing at least one heteroatom each independently selected from S(=O)ₚ and N; said 4-, 5- or 6-membered saturated heterocyclyl is optionally substituted with one or two hydroxyl substituents; or two substituents on the same carbon atom of said 4-, 5- or 6-membered saturated heterocyclyl are taken together to form together with the common carbon atom to which they are attached Ring A;
(ix) Ring A represents cyclobutyl optionally substituted with one hydroxy substituent;
(x) p represents 2.

In an embodiment, the present invention relates to those compounds of Formula (I) and the N-oxides, the pharmaceutically acceptable addition salts, and the solvates thereof, or any subgroup thereof as mentioned in any of the other embodiments, wherein R¹ represents -NH₂; R² represents

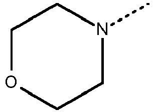

In an embodiment, the present invention relates to those compounds of Formula (I) and the N-oxides, the pharmaceutically acceptable addition salts, and the solvates thereof, or any subgroup thereof as mentioned in any of the other embodiments, wherein R¹ represents hydrogen.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl; or C₁₋₄alkyl substituted with one -OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl substituted with one Het¹; in particular wherein Het¹ is attached to the remainder of R³ through a ring nitrogen atom (Het^{1a}).

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4a} represents hydrogen, C₁₋₄alkyl, or C₁₋₄alkyl substituted with one or more substituents each independently selected from the group consisting of -NR⁵R⁶ and Het^{a};
R^{4b} represents hydrogen, halo, C₁₋₄alkyl, or C₁₋₄alkyl substituted with one or more halo substituents.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4a} represents hydrogen, C₁₋₄alkyl, Het^{a}, or C₁₋₄alkyl substituted with one or more substituents each independently selected from the group consisting of -OH, -NR⁵R⁶ and Het^{a};
R^{4b} represents hydrogen, halo, C₁₋₄alkyl, or C₁₋₄alkyl substituted with one or more halo substituents.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4a} represents C₁₋₄alkyl; in particular R^{4a} represents methyl;
R^{4b} represents C₁₋₄alkyl substituted with one or more halo substituents; in particular R^{4b} represents CF₃.

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{4a} represents C₁₋₄alkyl; in particular R^{4a} represents methyl.

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{4a} represents hydrogen, C₁₋₄alkyl, Het^{a}, or C₁₋₄alkyl substituted with one substituent selected from the group consisting of -OH, -NR⁵R⁶ and Het^{a}.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4b} represents C₁₋₄alkyl substituted with one or more halo substituents; in particular R^{4b} represents CF₃.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4a} and R^{4b} are other than hydrogen.

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{4a} and R^{4b} are taken together to form together with the phenyl ring to which they are attached a structure of Formula (a-1), (a-2), (a-3), (a-4) or (a-5); in particular a structure of Formula (a-2) or (a-4); more in particular a structure of Formula (a-2).

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4a} represents C₁₋₄alkyl, Het^{a}, or C₁₋₄alkyl substituted with one or more substituents each independently selected from the group consisting of -OH, -NR⁵R⁶ and Het^{a};
R^{4b} represents hydrogen, halo, C₁₋₄alkyl, or C₁₋₄alkyl substituted with one or more halo substituents;
or R^{4a} and R^{4b} are taken together to form together with the phenyl ring to which they are attached a structure of Formula (a-2).

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{4a} represents C₁₋₄alkyl, Het^{a}, or C₁₋₄alkyl substituted with one or more substituents each independently selected from the group consisting of -OH, -NR⁵R⁶ and Het^{a}; R^{4b} represents C₁₋₄alkyl, or C₁₋₄alkyl substituted with one or more halo substituents.

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{4a} represents C₁₋₄alkyl, Het^{a}, or C₁₋₄alkyl substituted with one or more substituents each independently selected from the group consisting of -OH, -NR⁵R⁶ and Het^{a}; R^{4b} represents C₁₋₄alkyl.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R^{4a} represents C₁₋₄alkyl, Het^{a}, or C₁₋₄alkyl substituted with one or more substituents each independently selected from the group consisting of -OH, -NR⁵R⁶ and Het^{a};
R^{4b} represents C₁₋₄alkyl, or C₁₋₄alkyl substituted with one or more halo substituents;
or R^{4a} and R^{4b} are taken together to form together with the phenyl ring to which they are attached a structure of Formula (a-2).

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl; or C₁₋₄alkyl substituted with one substituent selected from the group consisting of fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 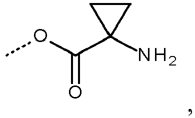 and -NH-C₁₋₄alkyl-OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl; -CH(OH)-CH₂-R^{q}; or C₁₋₄alkyl substituted with one substituent selected from the group consisting of fluoro, -OH, -NH₂, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, and -NH-C₁₋₄alkyl-OH;
R^{q} represents -OH, or -NH₂.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl substituted with one substituent selected from the group consisting of fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 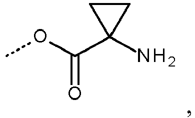 and -NH-C₁₋₄alkyl-OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl; or C₁₋₄alkyl substituted with one substituent selected from the group consisting of fluoro, -OH, -NH₂, -O-(C=O)-C₁₋₄alkyl, -NH-(C=O)-C₁₋₄alkyl, -N(CH₃)-C₁₋₄alkyl-SO₂-CH₃, -NH-C₁₋₄alkyl-SO₂-CH₃, -O-(C=O)-CH(NH₂)-C₁₋₄alkyl, -O-(C=O)-CH(NH₂)-C₁₋₄alkyl-Ar, 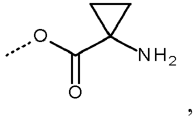 and -NH-C₁₋₄alkyl-OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents -CH(OH)-CH₂-R^{q}; or C₁₋₄alkyl substituted with one substituent selected from the group consisting of fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-(CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents -CH(OH)-CH₂-R^{q}; or C₁₋₄alkyl substitued with one substituent selected from the group consisting of fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -C-(C=O)-CH(NH₂)-C₁₋₄alkyl, -O-(C=O)-CH(NH₂)-C₁₋₄alkyl-Ar, 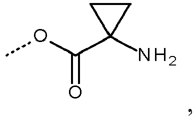 and -NH-C₁₋₄alkyl-OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one substituent as defined in any of the other embodiments.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R² represents

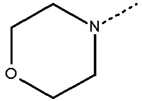

In an embodiment, the present invention relates to those compounds of Formula (I) and the N-oxides, the pharmaceutically acceptable addition salts, and the solvates thereof, or any subgroup thereof as mentioned in any of the other embodiments, wherein
R² represents

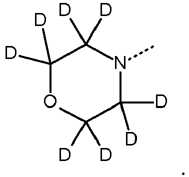

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one -OH substituent; in particular R³ represents -CH₂-OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R¹ represents -C(=O)NH₂, -NH₂,

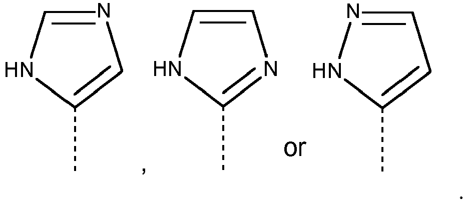

In an embodiment, the present invention relates to those compounds of Formula (I) and the N-oxides, the pharmaceutically acceptable addition salts, and the solvates thereof, or any subgroup thereof as mentioned in any of the other embodiments, wherein
R¹ represents -C(=O)NH₂, -NH₂,

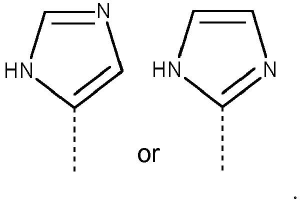

In an embodiment, the present invention relates to those compounds of Formula (I) and the N-oxides, the pharmaceutically acceptable addition salts, and the solvates thereof, or any subgroup thereof as mentioned in any of the other embodiments, wherein
R¹ represents

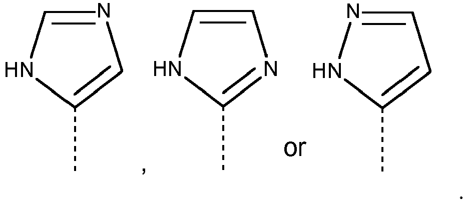

In an embodiment, the present invention relates to those compounds of Formula (I) and the N-oxides, the pharmaceutically acceptable addition salts, and the solvates thereof, or any subgroup thereof as mentioned in any of the other embodiments, wherein R¹ represents -C(=O)OH, -C(=O)NH₂, or -NH₂.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R¹ represents -C(=O)NH₂ or -NH₂.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R¹ represents -NH₂.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R¹ represents hydrogen.

In an embodiment, the present invention relates to those compounds of Formula (I) and the N-oxides, the pharmaceutically acceptable addition salts, and the solvates thereof, or any subgroup thereof as mentioned in any of the other embodiments, wherein R¹ represents other than hydrogen.

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{q} represents fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -O-(C=O)-CH(NH₂)-C₁₋₄alkyl, or -NH-C₁₋₄alkyl-OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein R^{q} represents -OH or -NH₂; in particular wherein R^{q} represents -NH₂.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl; or C₁₋₄alkyl substituted with one substituent selected from the group consisting of fluoro, -OH, -O-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH and -NH-C₁₋₄alkyl-OH;
in particular wherein R³ represents C₁₋₄alkyl; or C₁₋₄alkyl substituted with one substituent selected from the group consisting of fluoro, -OH, -N(CH₃)-C₁₋₄alkyl-SO₂-CH₃, -NH-C₁₋₄alkyl-SO₂-CH₃, -N(CH₃)-C₁₋₄alkyl-OH, and -NH-C₁₋₄alkyl-OH;
more in particular wherein R³ represents C₁₋₄alkyl; or C₁₋₄alkyl substituted with one substituent selected from the group consisting of fluoro and -OH;
even more in particular wherein R³ represents C₁₋₄alkyl; or C₁₋₄alkyl substituted with one -OH substituent;
still more in particular wherein R³ represents C₁₋₄alkyl.

In an embodiment, the present invention relates to those compounds of Formula (I) and the N-oxides, the pharmaceutically acceptable addition salts, and the solvates thereof, or any subgroup thereof as mentioned in any of the other embodiments, wherein each Het^{a} independently represents

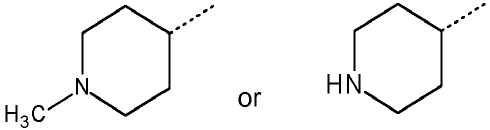

In an embodiment, the present invention relates to those compounds of Formula (I) and the N-oxides, the pharmaceutically acceptable addition salts, and the solvates thereof, or any subgroup thereof as mentioned in any of the other embodiments, wherein each Het^{a} independently represents a 4-, 5- or 6-membered saturated heterocyclyl containing one or two heteroatoms each independently selected from O, S(=O)ₚ and N; said 4-, 5- or 6-membered saturated heterocyclyl is optionally substituted with one or two substituents each independently selected from the group consisting of hydroxy, and C₁₋₄alkyl substituted with one hydroxy;
p represents 1 or 2.

In an embodiment, the present invention relates to those compounds of Formula (I) and the N-oxides, the pharmaceutically acceptable addition salts, and the solvates thereof, or any subgroup thereof as mentioned in any of the other embodiments, wherein both R⁷ substituents are hydrogen; and wherein both R⁸ substituents are hydrogen.

In an embodiment, the present invention relates to those compounds of Formula (I) and the N-oxides, the pharmaceutically acceptable addition salts, and the solvates thereof, or any subgroup thereof as mentioned in any of the other embodiments, wherein both R⁷ substituents are the same and are selected from the group consisting of hydrogen, fluoro and methyl; and wherein
both R⁸ substituents are the same and are selected from the group consisting of hydrogen and methyl.

In an embodiment, the present invention relates to those compounds of Formula (I) and the N-oxides, the pharmaceutically acceptable addition salts, and the solvates thereof, or any subgroup thereof as mentioned in any of the other embodiments, wherein R² represents

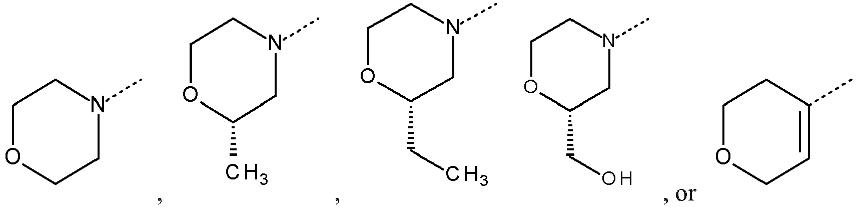

In an embodiment, the present invention relates to those compounds of Formula (I) and the N-oxides, the pharmaceutically acceptable addition salts, and the solvates thereof, or any subgroup thereof as mentioned in any of the other embodiments, wherein R² represents

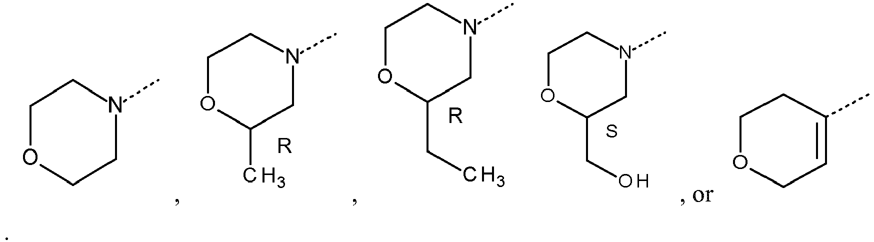

In an embodiment, the present invention relates to those compounds of Formula (I) and the N-oxides, the pharmaceutically acceptable addition salts, and the solvates thereof, or any subgroup thereof as mentioned in any of the other embodiments, wherein
R² representing 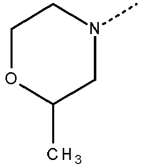 is limited to

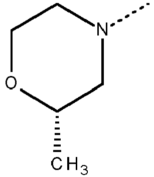

In an embodiment, the present invention relates to those compounds of Formula (I) and the N-oxides, the pharmaceutically acceptable addition salts, and the solvates thereof, or any subgroup thereof as mentioned in any of the other embodiments, wherein R² representing 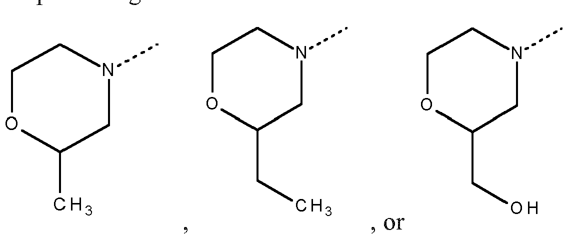 are limited respectively to

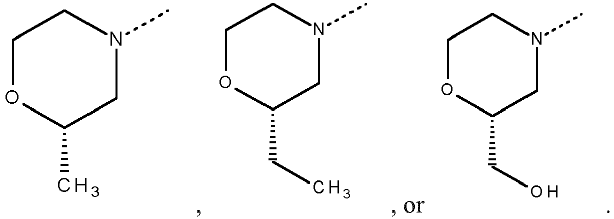

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one substituent selected from the group consisting of Het^{1a}, -C(=O)-Het¹, and -NH-C(=O)-Het^{1b}; or
C₁₋₄alkyl substituted on the same carbon atom with one -OH and with one Het^{1b};
Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of halo, -NH₂, C₁₋₄alkyl, -S(=O)₂-C₁₋₆alkyl, -C₁₋₄alkyl-S(=O)₂-C₁₋₆alkyl, hydroxy and C₁₋₄alkyl substituted with one hydroxy; or two substituents on the same carbon atom of said 4-, 5- or 6-membered saturated heterocyclyl are taken together to form together with the common carbon atom to which they are attached Ring A;
Het^{1a} is defined as Het¹ provided however that Het^{1a} is always attached to the remainder of R³ through a ring nitrogen atom;
Het^{1b} is defined as Het¹ provided however that Het^{1b} is always attached to the remainder of R³ through a ring carbon atom.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one substituent selected from the group consisting of Het^{1a}, -O-C(=O)-C₁₋₄alkyl-Het^{1a}, -C(=O)-Het¹, and -NH-C(=O)-Het^{1b}; -CH(OH)-CH₂-Het^{1a}; or C₁₋₄alkyl substituted on the same carbon atom with one -OH and with one Het^{1b};
Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of halo, -NH₂, C₁₋₄alkyl, -S(=O)₂-C₁₋₆alkyl, -C₁₋₄alkyl-S(=O)₂-C₁₋₆alkyl, hydroxy and C₁₋₄alkyl substituted with one hydroxy; or two substituents on the same carbon atom of said 4-, 5- or 6-membered saturated heterocyclyl are taken together to form together with the common carbon atom to which they are attached Ring A;
Het^{1a} is defined as Het¹ provided however that Het^{1a} is always attached to the remainder of R³ through a ring nitrogen atom;
Het^{1b} is defined as Het¹ provided however that Het^{1b} is always attached to the remainder of R³ through a ring carbon atom.

In an embodiment, the present invention relates to those compounds of Formula (I) and the N-oxides, the pharmaceutically acceptable addition salts, and the solvates thereof, or any subgroup thereof as mentioned in any of the other embodiments, wherein R¹ represents other than -C(=O)OH.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one substituent selected from the group consisting of Het¹, -C(=O)-Het¹, and -NH-C(=O)-Het¹; or
C₁₋₄alkyl substituted on the same carbon atom with one -OH and with one Het¹.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one substituent selected from the group consisting of Het¹, -C(=O)-Het¹, and -NH-C(=O)-Het¹; -CH(OH)-CH₂-Het¹; or
C₁₋₄alkyl substituted on the same carbon atom with one -OH and with one Het¹.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl substituted with one substituent selected from the group consisting of Het¹, -O-C(=O)-C₁₋₄alkyl-Het¹, -C(=O)-Het¹, and -NH-C(=O)-Het¹; or -CH(OH)-CH₂-Het¹.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl substituted with one substituent selected from the group consisting of Het¹, -C(=O)-Het¹, and -NH-C(=O)-Het¹.

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl substituted with one substituent selected from the group consisting of Het¹ and -C(=O)-Het¹;
in particular R³ represents C₁₋₄alkyl substituted with one Het¹.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl substituted with one substituent selected from the group consisting of Het¹, -C(=O)-Het¹, and -NH-C(=O)-Het¹.

In an embodiment, the present invention relates to those compounds of Formula (I) and the N-oxides, the pharmaceutically acceptable addition salts, and the solvates thereof, or any subgroup thereof as mentioned in any of the other embodiments, wherein R³ represents C₁₋₄alkyl substituted with one Het¹ substituent; in particular R³ represents C₁₋₄alkyl substituted with one Het^{1a} substituent wherein Het^{1a} is defined as Het¹ provided however that Het^{1a} is always attached to C₁₋₄alkyl through a ring nitrogen atom.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing at least one heteroatom each independently selected from S(=O)ₚ and N; said 4-, 5- or 6-membered saturated heterocyclyl is optionally substituted with one or two substituents each independently selected from the group consisting of -NH₂, C₁₋₄alkyl, -S(=O)₂-C₁₋₆alkyl, hydroxy and C₁₋₄alkyl substituted with one hydroxy; or two substituents on the same carbon atom of said 4-, 5- or 6-membered saturated heterocyclyl are taken together to form together with the common carbon atom to which they are attached Ring A.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing at least one heteroatom each independently selected from S(=O)ₚ and N; said 4-, 5- or 6-membered saturated heterocyclyl is optionally substituted with one or two substituents each independently selected from the group consisting of -NH₂, C₁₋₄alkyl, -S(=O)₂-C₁₋₆alkyl, hydroxy and C₁₋₄alkyl substituted with one hydroxy.

In an embodiment, the present invention relates to those compounds of Formula (I) and the N-oxides, the pharmaceutically acceptable addition salts, and the solvates thereof, or any subgroup thereof as mentioned in any of the other embodiments, wherein Ring A represents a 4-, 5- or 6-membered saturated heterocyclyl containing at least one heteroatom each independently selected from S(=O)ₚ and N.

In an embodiment, the present invention relates to those compounds of Formula (I) and the N-oxides, the pharmaceutically acceptable addition salts, and the solvates thereof, or any subgroup thereof as mentioned in any of the other embodiments, wherein Ring A represents cyclobutyl.

In an embodiment, the present invention relates to those compounds of Formula (I) and the N-oxides, the pharmaceutically acceptable addition salts, and the solvates thereof, or any subgroup thereof as mentioned in any of the other embodiments, wherein Ring A represents cyclobutyl, cyclopentyl, cyclohexyl or a 4-, 5- or 6-membered saturated heterocyclyl containing at least one heteroatom each independently selected from S(=O)ₚ and N; said cyclobutyl, cyclopentyl, cyclohexyl or 4-, 5- or 6-membered saturated heterocyclyl is optionally substituted with one hydroxy substituent.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing at least one heteroatom each independently selected from O, S, S(=O)ₚ and N; and 2 substituents on the same carbon atom of said 4-, 5- or 6-membered saturated heterocyclyl are taken together to form together with the common carbon atom to which they are attached Ring A.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of -NH₂, C₁₋₄alkyl, -S(=O)₂-C₁₋₆alkyl, -C₁₋₄alkyl-S(=O)₂-C₁₋₆alkyl, hydroxy and C₁₋₄alkyl substituted with one hydroxy.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing at least one heteroatom each independently selected from O, S, S(=O)ₚ and N; p represents 2.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing one S(=O)ₚ and also containing one N; p represents 2.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents a 4-, 5- or 6-membered saturated heterocyclyl containing one S(=O)ₚ and also containing one N; said 4-, 5- or 6-membered saturated heterocyclyl is optionally substituted with one or two substituents each independently selected from the group consisting of -NH₂, C₁₋₄alkyl, -S(=O)₂-C₁₋₆alkyl, -C₁₋₄alkyl-S(=O)₂-C₁₋₆alkyl, hydroxy and C₁₋₄alkyl substituted with one hydroxy;
p represents 2.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents

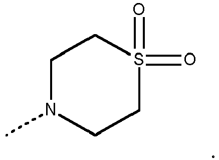

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents 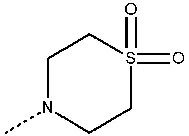 optionally substituted with one or two substituents each independently selected from the group consisting of -NH₂, C₁₋₄alkyl, -S(=O)₂-C₁₋₆alkyl, -C₁₋₄alkyl-S(=O)₂-C₁₋₆alkyl, hydroxy and C₁₋₄alkyl substituted with one hydroxyl; in particular optionally substituted with hydroxyl.

In an embodiment, the present invention relates to those compounds of Formula (I) and the N-oxides, the pharmaceutically acceptable addition salts, and the solvates thereof, or any subgroup thereof as mentioned in any of the other embodiments, wherein Het¹ represents

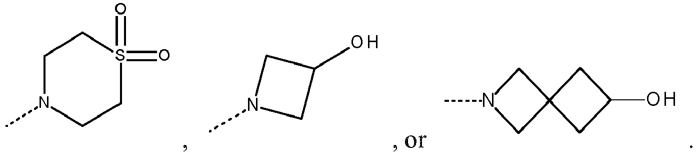

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl; -CH(OH)-CH₂-R^{q}; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 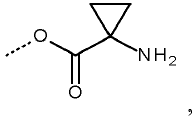 -NH-C₁₋₄alkyl-OH, Het¹, and -C(=O)-Het¹;
and wherein Het¹ represents

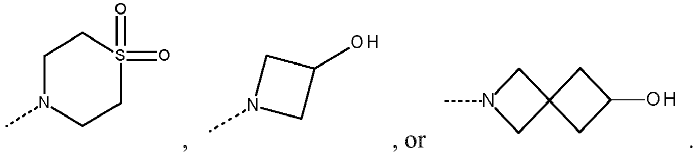

In an embodiment, the present invention relates to those compounds of Formula (I) and the N-oxides, the pharmaceutically acceptable addition salts, and the solvates thereof, or any subgroup thereof as mentioned in any of the other embodiments, wherein
Het¹ represents 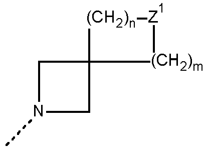
Z¹ represents -NH-, -S-, -O- or -S(O)₂-; in particular Z¹ represents -S(O)₂-;
n represents 0, 1 or 2;
m represents 1, 2 or 3; provided however that m does not have value 1 when n is 0.

In a particular embodiment, the present invention relates to those compounds of Formula (I) and the N-oxides, the pharmaceutically acceptable addition salts, and the solvates thereof, or any subgroup thereof as mentioned in any of the other embodiments, wherein Het¹ is attached to the remainder of the molecule of Formula (I) through a nitrogen atom (Het^{1a}).

In a particular embodiment, the present invention relates to those compounds of Formula (I) and the N-oxides, the pharmaceutically acceptable addition salts, and the solvates thereof, or any subgroup thereof as mentioned in any of the other embodiments, wherein Het¹ is attached to the remainder of the molecule of Formula (I) through a carbon atom (Het^{1b}).

In a particular embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl; C₁₋₄alkyl substituted on the same carbon atom with one -OH and with one Het¹; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl,-N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 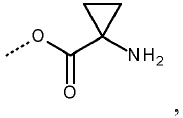 -NH-C₁₋₄alkyl-OH, Het¹, and -C(=O)-Het¹;
wherein Het¹ is attached to the remainder of the molecule of Formula (I) through a nitrogen atom (Het^{1a}).

In an embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl; -CH(OH)-CH₂-R^{q}; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 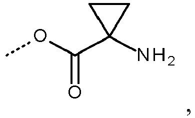 -NH-C₁₋₄alkyl-OH, Het¹, nd -C(=O)-Het¹;
wherein Het¹ is attached to the remainder of the molecule of Formula (I) through a nitrogen atom (Het^{1a}).

In a particular embodiment, the present invention relates to those compounds of Formula (I) and the N-oxides, the pharmaceutically acceptable addition salts, and the solvates thereof, or any subgroup thereof as mentioned in any of the other embodiments, wherein
R³ represents C₁₋₄alkyl; -CH(OH)-CH₂-R^{q}; C₁₋₄alkyl substituted on the same carbon atom with one -OH and with one Het^{1b}; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-C=O-CHNH₂-C₁₋₄alkyl -O-C=O-CH(NH₂)-C₁₋₄alkyl-Ar, 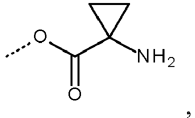 -NH-C₁₋₄alkyl-OH, Het^{1a}, -O-C(=O)-C₁₋₄alkyl-Het^{1a}, -C(=O)-Het¹, and -NH-C(=O)-Het^{1b};
R^{q} represents Het^{1a}, fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -O-(C=O)-CH(NH₂)-C₁₋₄alkyl, -O-(C=O)-CH(NH₂)-C₁₋₄alkyl-Ar, 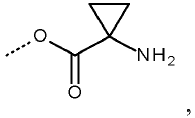 or -NH-C₁₋₄alkyl-OH;
Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of halo, -NH₂, C₁₋₄alkyl, -S(=O)₂-C₁₋₆alkyl, -C₁₋₄alkyl-S(=O)₂-C₁₋₆alkyl, hydroxy and C₁₋₄alkyl substituted with one hydroxy; or two substituents on the same carbon atom of said 4-, 5- or 6-membered saturated heterocyclyl are taken together to form together with the common carbon atom to which they are attached Ring A;
Het^{1a} is defined as Het¹ provided however that Het^{1a} is always attached to the remainder of R³ through a ring nitrogen atom;
Het^{1b} is defined as Het¹ provided however that Het^{1b} is always attached to the remainder of R³ through a ring carbon atom.

All possible combinations of the above-indicated embodiments are considered to be embraced within the scope of this invention.

### Methods for the Preparation of Compounds of Formula (I)

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or prepared by standard synthetic processes commonly used by those skilled in the art. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art of organic chemistry.

The skilled person will realize that in the reactions described in the Schemes, it may be necessary to protect reactive functional groups, for example hydroxy, amino, or carboxy groups, where these are desired in the final product, to avoid their unwanted participation in the reactions. Conventional protecting groups can be used in accordance with standard practice. This is illustrated in the specific examples. The protecting groups may be removed at a convenient subsequent stage using methods known from the art.

The skilled person will realize that in the reactions described in the Schemes, it may be advisable or necessary to perform the reaction under an inert atmosphere, such as for example under N₂-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the schemes below may be further functionalized according to methods well-known by the person skilled in the art.

In general, compounds of Formula (I) wherein R¹ is restricted to hydrogen, and wherein the other variables are as shown in Formula (Ia), can be prepared according to the following reaction Scheme 1, wherein W represent a leaving group such as Cl or Br. All other variables in Scheme 1 are defined according to the scope of the present invention.

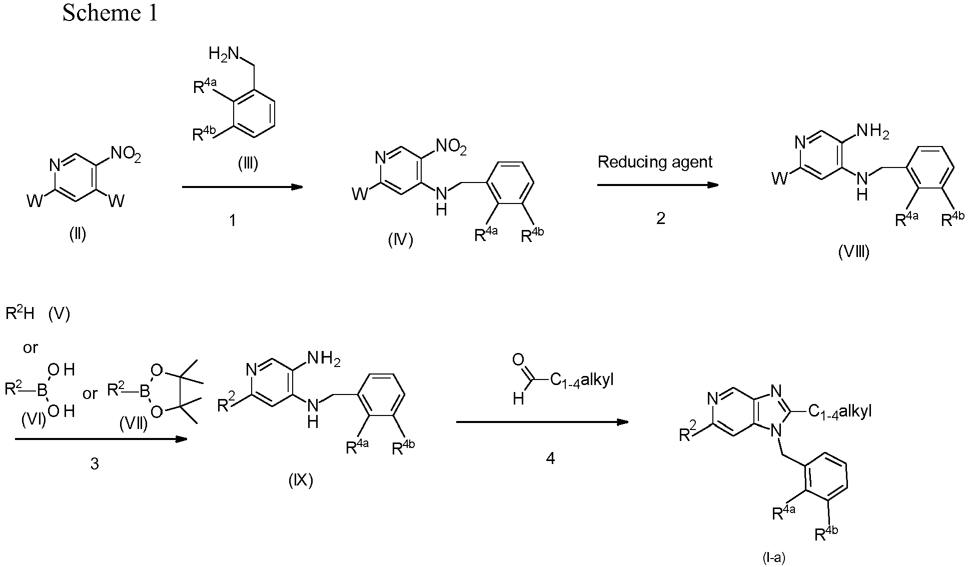

In Scheme 1, the following reaction conditions apply:
1: in the presence of a suitable base such as for example trimethylamine, in the presence of a suitable solvent such as for example tetrahydrofurane;
2: in the presence of a suitable reagent such as for example iron, in the presence of a suitable acid such as for example hydrochloric acid or aceric acid, in a suitable solvent such as for example a mixture of ethanol and water at a suitable temperature such as 100°C;
   Alternatively, in the presence of a suitable catalyst such as Raney® Nickel, under a pression of hydrogen suc as for example 1 atmosphere, in a suitable solvent such as for example methanol;
3: in case of R₂H:
   - Without solvent at a suitable temperature such as 100°C
   - Alternatively in the presence of a suitable ligand such as 2-dicyclohexyl-phosphino-2',6'-diisopropoxybiphenyl (Ruphos) or 2-Dicyclohexylphosphino-2'-(N,N-dimethylamino)biphenyl (DavePhos), a suitable catalyst such as for example tris(dibenzylideneacetone)dipalladium (Pd₂dba₃) or palladium acetate, a suitable base such as for example Cs₂CO₃, and a suitable solvent such as for example 2-methyl-2-butanol or dioxane, at a suitable temperature such as for example between 100 and 120°C;
   in case of R₂B(OH)₂ or R₂(4,4,5,5-tetramethyl-1,3,2-dioxaborolane), in the presence of a suitable catalyst such as for example 1,1'-bis(diphenylphosphino)ferrocene palladium(II)dichloride dichloromethane adduct or RuPhos palladacycle (chloropalladium, dicyclohexyl-[2-[2,6-di(propan-2-yloxy)phenyl]phenyl]phosphane, 2-methoxy-2-methylpropane, 2-phenylethanamine), a suitable base such as for example potassium phosphate, and a suitable solvent such as for example a mixture dioxane and water, at a suitable temperature ranged between 80°C and 105°C;
4: in a suitable solvent such as for example 1-butanol at a suitable temperature such as for example reflux.

In general, compounds of Formula (I) wherein R¹ is restricted to hydrogen, and wherein the other variables are as shown in Formula (I-b), (I-c), (I-d), (I-e) and (I-f) can be prepared according to the following reaction Scheme 2, wherein W¹ represent a leaving group such as Cl, a mesylate or a tosylate and, wherein R^{x} and R^{y} represent C₁₋₄alkyl, and R^{z} represents C₁₋₄alkyl or phenyl, for instance R^{x} and R^{y} represent CH₃ and R^{z} represents C(CH₃)₃ or phenyl. All other variables in Scheme 2 are defined according to the scope of the present invention.

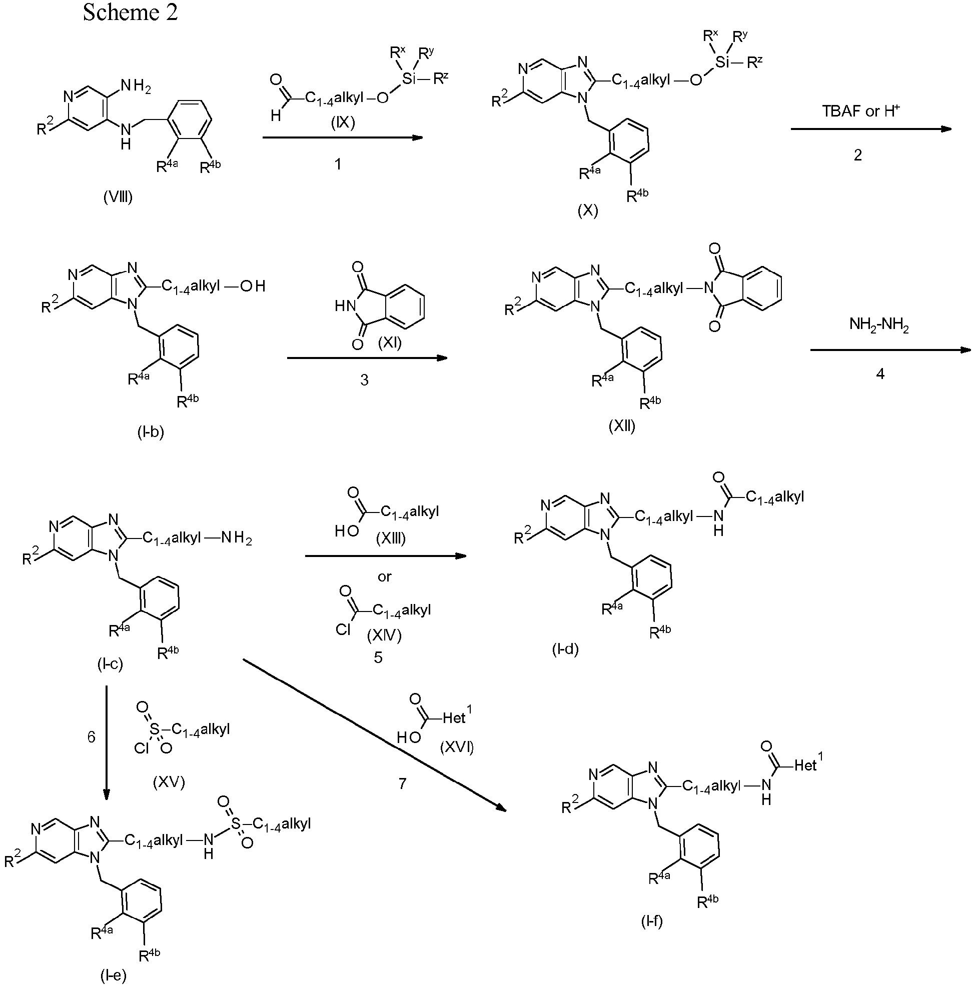

In Scheme 2, the following reaction conditions apply:
1: in a suitable solvent such as for example 1-butanol at a suitable temperature such as for example reflux;
2: in the presence of a suitable reagent such as for example tetrabutylammonium fluoride (TBAF), hydrochloric acid or trifluoroacetic acid in a suitable solvent such as THF, dioxane or dichloromethane;
3: in the presence of a suitable reagent such as for example di-*tert*-butyl azodicarboxylate, a suitable phosphine such as for example triphenylphosphine, and in a suitable solvent such as for example THF;
4: in the presence of suitable solvent such as ethanol at a suitable temperature such as 80°C;
5: in case of an acyl chloride, in the presence of a suitable base such as for example diisopropylethylamine, and in a suitable solvent such as for example dichloromethane in case of a carboxylic acid, in the presence of a suitable coupling reagent such as for example 1-(3-dimethylaminopropyl)-3-ethylcarbodiimide hydrochloride, a suitable additive such as for example 1-hydroxybenzotriazole, a suitable base such as for example triethylamine, and in a suitable solvent such as for example a mixture of tetrahydrofuran (THF) and dichloromethane (DCM);
6: in the presence of a suitable base such as for example triethylamine; in a suitable solvent such as for example dichloromethane;
7: in the presence of a suitable coupling reagent such as for example 1-(3-dimethylaminopropyl)-3-ethylcarbodiimide hydrochloride, a suitable additive such as for example 1-hydroxybenzotriazole, a suitable base such as for example triethylamine, and in a suitable solvent such as for example a mixture of THF and DCM.

In general, compounds of Formula (I) wherein R¹ is restricted to an hydrogen, and wherein the other variables are as shown in Formula (I-g), (I-h) and (I-i) can be prepared according to the following reaction Scheme 3, wherein Het^{1a} is restricted to Het¹ being attached via the nitrogen atom. All other variables in Scheme 3 are defined according to the scope of the present invention.

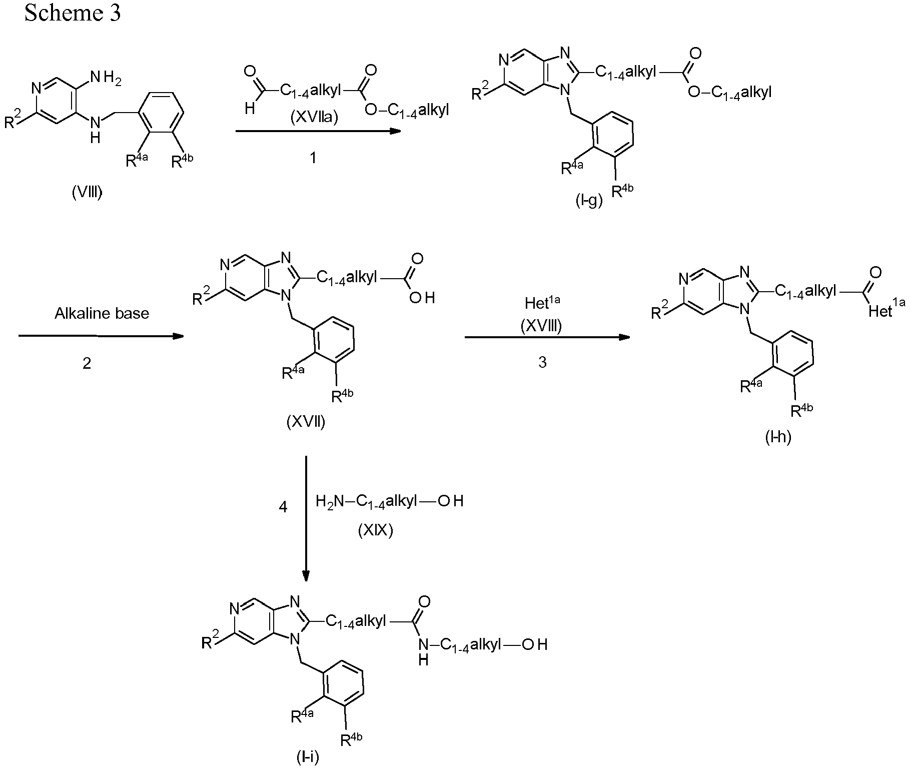

In Scheme 3, the following reaction conditions apply:
1: in a suitable solvent such as for example 1-butanol at a suitable temperature such as for example reflux;
2: in the presence of a suitable base such as for example solium hydroxide or lithium hydroxide, in a suitable solvent such as for example a mixture of tetrhydrofurane/water or a mixture of 2-methyltetrahydrofurane/water at a suitable temperature such as for example room temperature or 60°C.
3: in the presence of a suitable coupling reagent such as for example 1-(3-dimethylaminopropyl)-3-ethylcarbodiimide hydrochloride, a suitable additive such as for example 1-hydroxybenzotriazole, a suitable base such as for example triethylamine, and in a suitable solvent such as for example a mixture of THF and DCM;
4: in the presence of a suitable coupling reagent such as for example 1-(3-dimethylaminopropyl)-3-ethylcarbodiimide hydrochloride, a suitable additive such as for example 1-hydroxybenzotriazole, a suitable base such as for example triethylamine, and in a suitable solvent such as for example a mixture of THF and DCM.

In general, compounds of Formula (I) wherein R¹ is restricted to an hydrogen, and wherein the other variables are as shown in Formula (I-j) and (I-k) can be prepared according to the following reaction Scheme 4, wherein R⁹ is defined as being H or CH₃ and R¹⁰ is defined as being -C₁₋₄alkyl-SO₂-CH₃ or -C₁₋₄alkyl-OH. All other variables in Scheme 4 are defined according to the scope of the present invention.

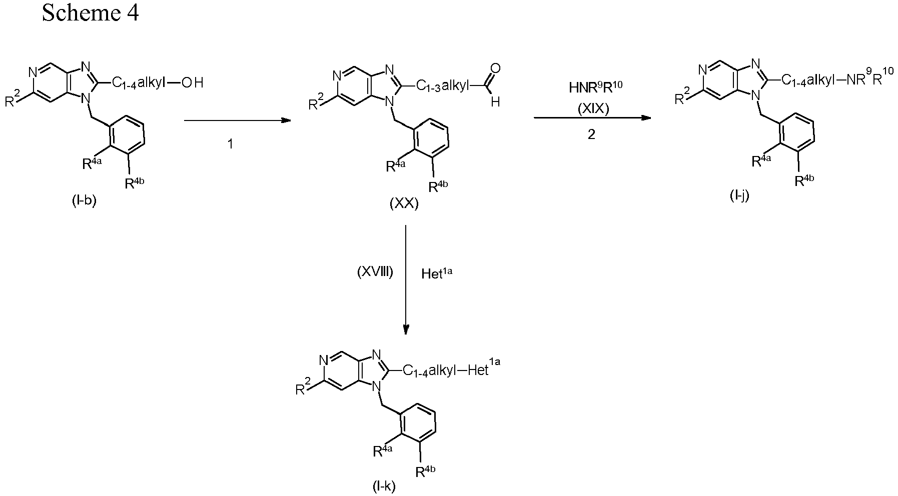

In Scheme 4, the following reaction conditions apply:
1: in the presence of suitable reagents such as for example oxalyl chloride and dimethylsulfoxide, a suitable base such as for example trimethylamine, in a suitable solvent such as for example DCM, at a suitable temperature ranged between -80°C to room temperature;
2: in the presence of a suitable reducing agent such as for example sodium triacetoxyborohydride, in a suitable solvent such as for example DCM;
3: in the presence of a suitable reducing agent such as for example sodium triacetoxyborohydride, in a suitable solvent such as for example DCM.

In general, compounds of Formula (I) wherein R¹ is restricted to an hydrogen, and wherein the other variables are as shown in Formula (I-1) can be prepared according to the following reaction Scheme 5. All other variables in Scheme 5 are defined according to the scope of the present invention.

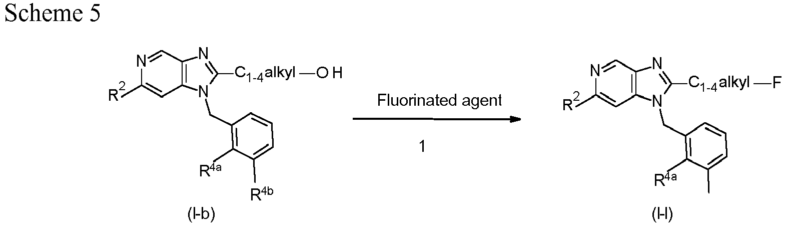

In Scheme 5, the following reaction conditions apply:
1: in the presence of a suitable fluorinated reagent such as for example diethylaminosulfur trifluoride in a suitable solvent such as for example DCM.

In general, compounds of Formula (I) wherein R¹ is restricted to an hydrogen, and wherein the other variables are as shown in Formula (I-m) can be prepared according to the following reaction Scheme 6. In scheme 6, R¹¹ represents -CH(NH₂)-C₁₋₄alkyl, -CH(NH₂)-C₁₋₄alkyl-Ar, 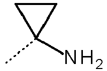 or -C₁₋₄alkyl-Het¹, nd PG¹ represent a protective group such as for example tert-butoxycarbonyl or benzyloxycarbonyl.

All other variables are defined as above or according to the scope of the present invention.

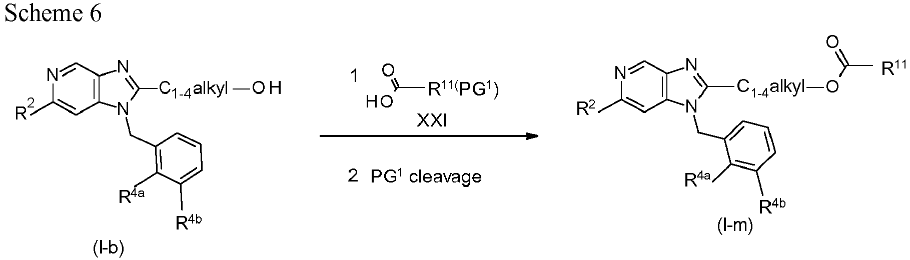

In Scheme 6, the following reaction conditions apply:
1: in the presence of a suitable coupling reagent such as for example 1-[bis(dimethyl-amino)methylene]-1*H*-1,2,3-triazolo[4,5-*b*]pyridinium 3-oxide, a suitable additive such as for example dimethylaminopyridine, a suitable base such as for example diisopropylethylamine, and in a suitable solvent such as for example DMF;
2: in the presence of an acide such as for example trifluoroacetic acid or hydrogen chloride in a suitable solvent such as for exemple dichloromethane or methanol. Alternatively, in the presence of palladium on charcoal, in a suitable solvent such as methanol under an atmosphere of hydrogen.

In general, compounds of Formula (I) wherein R¹ is restricted to hydrogen and R³ is restricted to CH₂OH, CH₂-NR⁹R¹⁰ and CH²-Het^{1a} (Het^{1a} is restricted to Het¹ being attached via the nitrogen atom) and wherein the other variables are as shown in Formula (I-aa), (I-ba), (I-ka) and (I-ja) can be prepared according to the following reaction Scheme 7. All other variables are defined as above or according to the scope of the present invention.

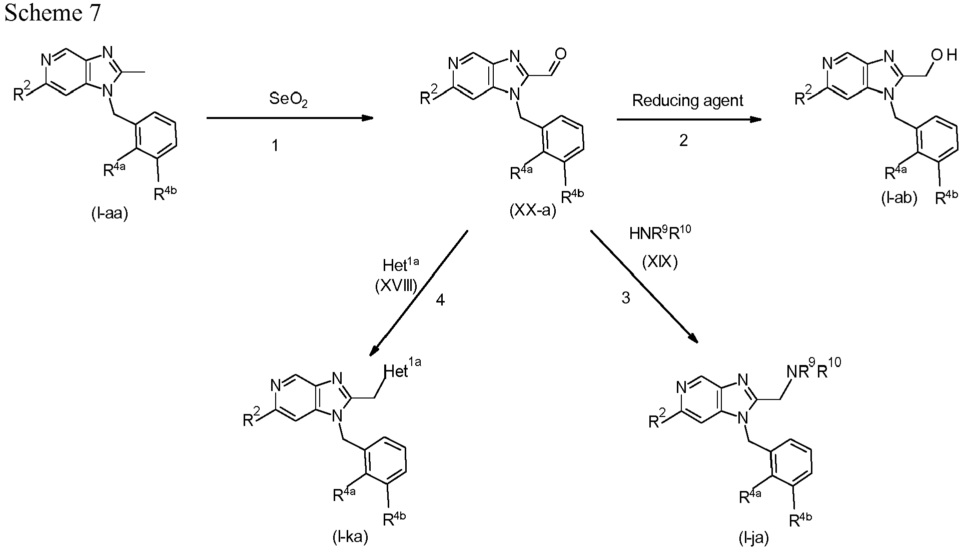

In Scheme 7, the following reaction conditions apply:
1: in a suitable solvent such as for example dioxane at a suitable temperature such as for example reflux;
2: in the presence of a suitable reducing agent such as for example sodium borohydride in a suitable solvent such as for example methanol;
3: in the presence of a suitable reducing agent such as for example sodium triacetoxyborohydride, in a suitable solvent such as for example DCM;
4: in the presence of a suitable reducing agent such as for example sodium triacetoxyborohydride, in a suitable solvent such as for example DCM.

In general, compounds of Formula (I) wherein R¹ is restricted to hydrogen and Het^{1a} is restricted to Het¹ being attached via the nitrogen atom and, herein the other variables are as shown in Formula (I-n) can be prepared according to the following reaction Scheme 8. All other variables are defined as above or according to the scope of the present invention.

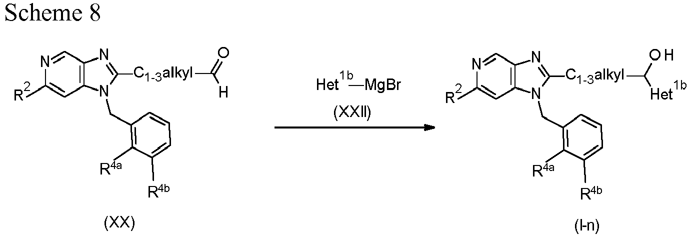

In Scheme 8, the following reaction conditions apply:
1: at a suitable temperature such as for example 0°C or -78°C, in a suitable solvent such as for example THF.

In general, compounds of Formula (I) wherein R¹ is restricted to hydrogen and wherein the other variables are as shown in Formula (I-o), (I-p) and (I-q) can be prepared according to the following reaction Scheme 9. All other variables are defined as above or according to the scope of the present invention.

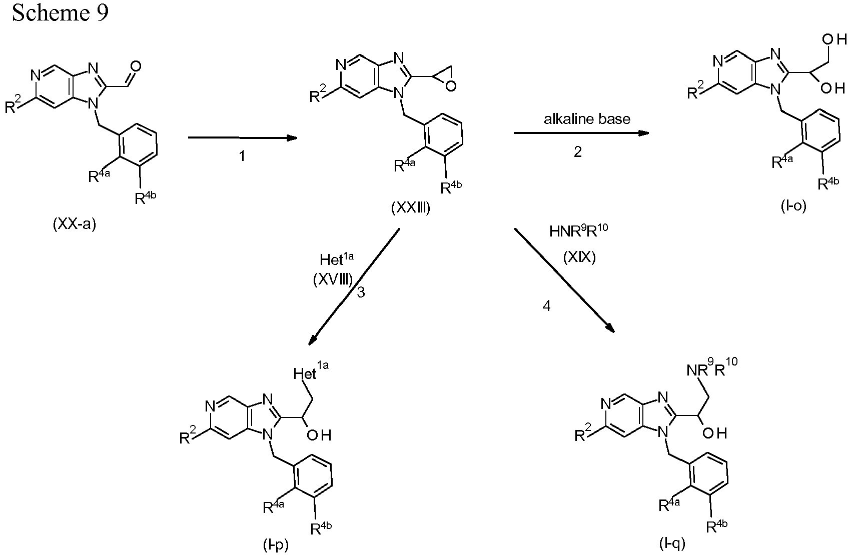

In Scheme 9, the following reaction conditions apply:
1: in the presence of suitable reagent such as for example Trimethylsulfonium iodide, in the presence of a suibale base such as for exmaple potassium hydroxide, in a suitable solvent such as for exmaple a mixture of acetonitrile and water, at a suitable temperature such as for exmaple 60°C;
2: in the presence of a suitable alkaline base such as for example sodium hydroxide, in a suitable solvent such as for example a mixture of dioxane and water at a suitable temperature such as for example 80°C;
3: in a suitable solvent such as for example acetonitrile or dimethylformamide, at a suitable temperature such as for example 80°C, optionally in sealed conditions;
4: in a suitable solvent such as for example acetonitrile or dimethylformamide, at a suitable temperature such as for example 80°C, optionally in sealed conditions.

In general, compounds of Formula (I) wherein R¹ is -NH₂, and wherein the other variables are as shown in Formula (I-r), can be prepared according to the following reaction Scheme 10, wherein W³ represent a leaving group such as Cl, Br or I. All other variables are defined as above or according to the scope of the present invention.

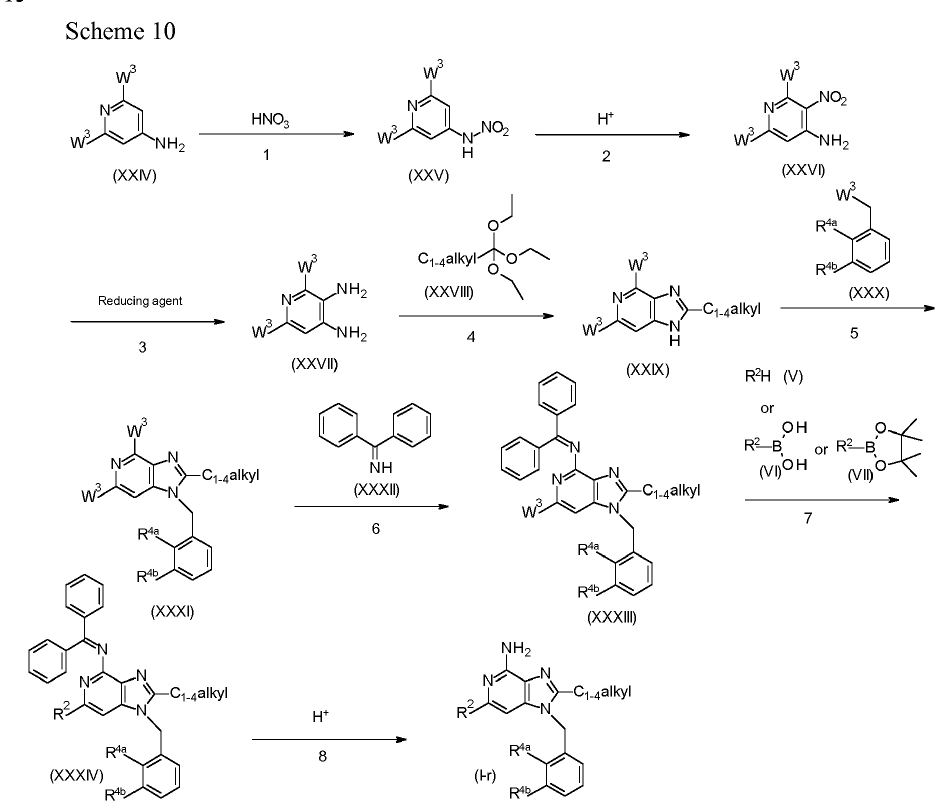

In Scheme 10, the following reaction conditions apply:
1: In the presence of a nitrating agent such as for example nitric acid, in a suitable solvent such as for example sulphuric acid at a suitable temperature such as for example 10°C;
2: in a suitable solvent such as for example sulphuric acid at a suitable temperature such as for example 100°C;
3: in the presence of a suitable reagent such as for example iron, in the presence of a suitable acid such as for example hydrochloric acid or acetic acid, in a suitable solvent such as for example a mixture of ethanol and water at a suitable temperature such as 100°C;
4: in the presence of a suitable acid such as for example hydrochloric acid, in a suitable solvent such as for example ethanol at a suitable temperature such as for example 100°C;
5: in the presence of a suitable base such as for example potassium carbonate, in a suitable solvent such as for example acetonitrile, at a suitable temperature such as for example 85°C;
6: in a sealed tube, in the presence of a suitable catalyst such as for example palladium acetate, in the presence of a suitable ligand such as for example 2,2'-bis(diphenyl-phosphino)-1,1'-binaphthyl, in the presence of a suitable base such as for example cesium carbonate, in a suitable solvent such as for example dioxane at a suitable temperature such as for example 100°C;
7: in case of R₂H, in the presence of a suitable ligand such as 2-Dicyclohexyl-phosphino-2'-(N,N-dimethylamino)biphenyl (DavePhos), a suitable catalyst such as for example palladium acetate, a suitable base such as for example Cs₂CO₃, and a suitable solvent such as for example dioxane, at a suitable temperature such as for example 120°C;
   in case of R₂B(OH)₂ or R₂(4,4,5,5-tetramethyl-1,3,2-dioxaborolane), in the presence of a suitable catalyst such as for example 1,1'-bis(diphenylphosphino)ferrocene palladium(II)dichloride dichloromethane adduct or RuPhos palladacycle, a suitable base such as for example potassium phosphate, and a suitable solvent such as for example a mixture dioxane and water, at a suitable temperature range between 80°C and 105°C;
8: in the presence of a suitable acid such as for example hydrochloric acid, in a suitable solvent such as for example tetrahydrofuran (THF).

In general, compounds of Formula (I) wherein R¹ is restricted to R^{1a} being 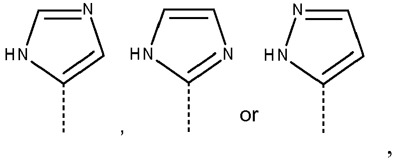 and wherein the other variables are as shown in Formula (I-s) can be prepared according to the following reaction Scheme 11. In scheme 11, PG is defined as a protective group such as for example a *N,N-*dimethyl-sulfonamidyl or 2-tetrahydropyranyl moiety. All other variables in Scheme 11 are defined as above or according to the scope of the present invention.

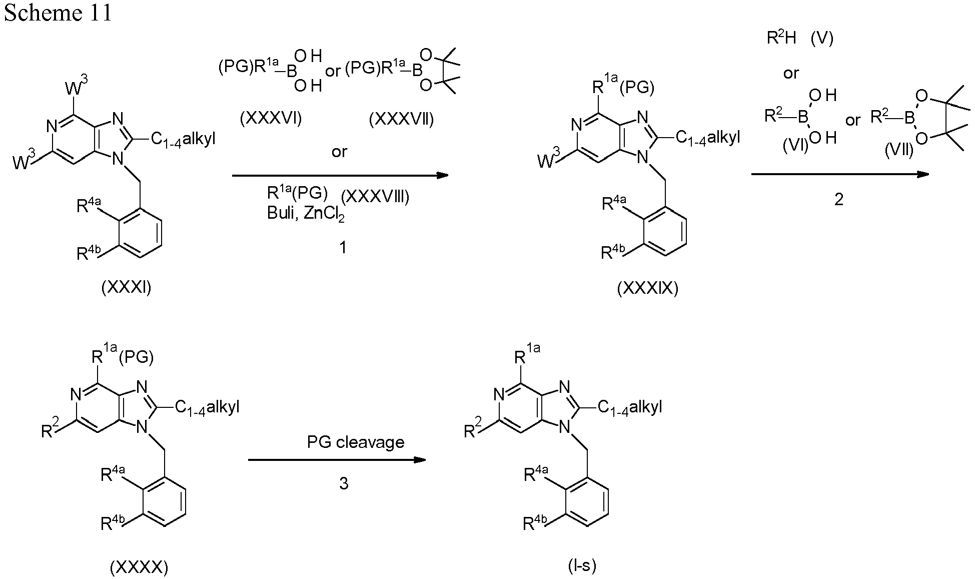

In Scheme 11, the following reaction conditions apply:
1: in case of (PG)R^{1a}B(OH)₂ or (PG)R^{1a} (4,4,5,5-tetramethyl-1,3,2-dioxaborolane), in the presence of a suitable catalyst such as for example 1,1'-bis(diphenylphosphino)-ferrocene palladium(II)dichloride dichloromethane adduct, a suitable base such as for example potassium carbonate, and a suitable solvent such as for example a mixture of dioxane and water, at a suitable temperature such as for example at 100°C;
   In case of R^{1a}(PG), first, n the presence of zinc chloride, a suitable deprotonating agent such as for example butyl lithium, a suitable solvent such as for example THF, at a suitable temperature such as for example -78°C, followed by addition (of/to) this solution (to) a mixture of intermediate (XXXXVI), optionally in solution in THF, and a suitable catalyst such as for example Pd(PPh₃)₄, heating at a suitable temperature ranging from 60 to 100°C;
2: in case of R₂H:
   - Without solvent at a suitable temperature such as 100°C
   - Alternatively in the presence of a suitable ligand such as 2-dicyclohexyl-phosphino-2',6'-diisopropoxybiphenyl (RuPhos), a suitable catalyst such as for example tris(dibenzylideneacetone)dipalladium (Pd₂dba₃), a suitable base such as for example Cs₂CO₃, and a suitable solvent such as for example 2-methyl-2-butanol, at a suitable temperature such as for example between 100 and 120°C;
   in case of R₂B(OH)₂ or R₂(4,4,5,5-tetramethyl-1,3,2-dioxaborolane), in the presence of a suitable catalyst such as for example 1,1'-bis(diphenylphosphino)ferrocene palladium(II)dichloride dichloromethane adduct or RuPhos palladacycle, a suitable base such as for example potassium phosphate, and a suitable solvent such as for example a mixture dioxane and water, at a suitable temperature ranged between 80°C and 105°C;
3: in the presence of a suitable acid such as for example p-toluenesulfonic acid, hydrochloric acid or trifluoroacetic acid, in a suitable solvent such as for example dioxane, methanol or dichloromethane, at a suitable temperature such as for example 50 or 100°C.

In general, compounds of Formula (I) wherein R¹ is -COOH, and wherein the other variables are as shown in Formula (I-u); and compounds of Formula (I) wherein R¹ is -CONH₂, and wherein the other variables are as shown in Formula (I-v) can be prepared according to the following reaction Scheme 12, wherein all other variables are defined as above or according to the scope of the present invention.

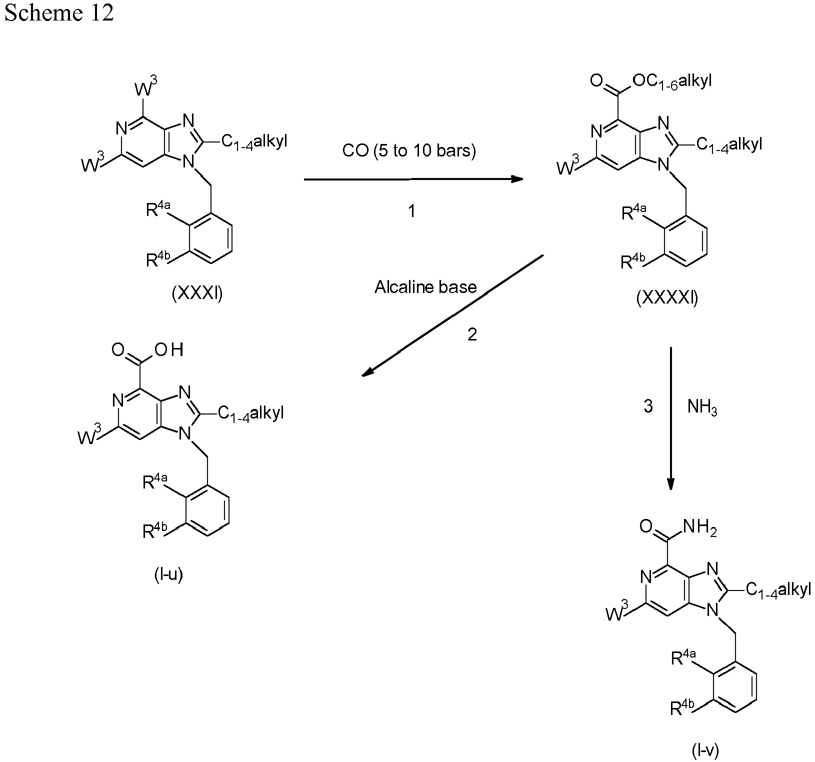

In Scheme 12, the following reaction conditions apply:
1: in the presence of a suitable catalyst such as for example Pd(PPh₃)₄, a suitable base such as for example triethylamine (Et₃N), and a suitable solvent such as for example methanol or ethanol, at a suitable temperature such as for example at 100°C or 120°C;
2: in the presence of a suitable base such as for example lithium hydroxide monohydrate, and a suitable solvent or a mixture of solvents such as for example a mixture of THF/water or MeOH/water;
3: in a suitable solvent such as for example methanol, at a suitable temperature such as for example at 65°C and, in a sealed vessel.

In all these preparations, the reaction products may be isolated from the reaction medium and, if necessary, further purified according to methodologies generally known in the art such as, for example, extraction, crystallization, trituration and chromatography.

The chirally pure forms of the compounds of Formula (I) form a preferred group of compounds. It is therefore that the chirally pure forms of the intermediates and their salt forms are particularly useful in the preparation of chirally pure compounds of Formula (I). Also enantiomeric mixtures of the intermediates are useful in the preparation of compounds of Formula (I) with the corresponding configuration.

### Pharmacology

It has been found that the compounds of the present invention inhibit PI3Kβ kinase activity, and optionally also have PI3Kδ inhibitory activity.

It is therefore anticipated that the compounds according to the present invention or pharmaceutical compositions thereof may be useful for treating or preventing, in particular treating, of diseases such as cancer, autoimmune disorders, cardiovascular diseases, inflammatory diseases, neurodegenerative diseases, allergy, pancreatitis, asthma, multiorgan failure, kidney diseases, platelet aggregation, sperm motility, transplantation rejection, graft rejection, lung injuries and the like; in particular cancer.

Because the pharmaceutically active compounds of the present invention are active as PI3Kβ inhibitors, they exhibit therapeutic utility in treatment or prevention, in particular treatment, of susceptible neoplasms, particularly those neoplasms that exhibit a PTEN deficiency.

As used herein, the phrase "PTEN deficient" or "PTEN deficiency" shall describe tumors with deficiencies of the tumor suppressor function of PTEN (Phosphatase and Tensin Homolog). Such deficiency includes mutation in the PTEN gene, reduction or absence of PTEN proteins when compared to PTEN wild-type, or mutation or absence of other genes that cause suppression of PTEN function.

"Susceptible neoplasm" as used herein refers to neoplasms which are susceptible to treatment by a kinase inhibitor and particularly neoplasms that are susceptible to treatment by a PI3Kβ inhibitor. Neoplasms which have been associated with inappropriate activity of the PTEN phosphatase and particularly neoplasms which exhibit mutation of PTEN, or mutation of an upstream activator of PI3Kβ kinase or overexpression of an upstream activator of PI3Kβ kinase, and are therefore susceptible to treatment with an PI3Kβ inhibitor, are known in the art, and include both primary and metastatic tumors and cancers. According to an embodiment, description of the treatment of a susceptible neoplasm may be used interchangeably with description of the treatment of a cancer.

According to one embodiment, "susceptible neoplasms" include but are not limited to PTEN-deficient neoplasms listed as follows: brain (gliomas), glioblastomas, leukemias, Bannayan-Zonana syndrome, Cowden disease, Lhermitte-Duclos disease, breast cancer, inflammatory breast cancer, colorectal cancer Wilm's tumor, Ewing's sarcoma, Rhabdomyosarcoma, ependymoma, medulloblastoma, colon cancer, head and neck cancer, liver cancer, kidney cancer, lung cancer, melanoma, squamous cell carcinoma, ovarian cancer, pancreatic cancer, prostate cancer, sarcoma cancer, osteosarcoma, giant cell tumor of bone, thyroid cancer, lymphoblastic T cell leukemia, chronic myelogenous leukemia, chronic lymphocytic leukemia, hairy-cell leukemia, acute lymphoblastic leukemia, acute myelogenous leukemia, chronic neutrophilic leukemia, acute lymphoblastic T cell leukemia, Plasmacytoma, Immunoblastic large cell leukemia, Mantle cell leukemia, Multiple myeloma, Megakaryoblastic leukemia, Acute megakaryocytic leukemia, promyelocytic leukemia, Erythro leukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, cervical cancer, vulval cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor), and testicular cancer.

According to an alternative embodiment, the term "susceptible neoplasm" includes and is limited to hormone refractory prostate cancer, non-small-cell lung cancer, endometrial cancer, gastric cancer, melanoma, head and neck cancer, breast cancer, including tripnegative breast cancer, and glioma.

In an embodiment, the term "susceptible neoplasm" includes and is limited to prostate cancer, in particular hormone refractory prostate cancer.

The compounds of the present invention may also have therapeutic applications in sensitising tumour cells for radiotherapy and chemotherapy.

Hence the compounds of the present invention may be used as "radiosensitizer" and/or "chemosensitizer" or can be given in combination with another "radiosensitizer" and/or "chemosensitizer".

The term "radiosensitizer", as used herein, is defined as a molecule, preferably a low molecular weight molecule, administered to animals in therapeutically effective amounts to increase the sensitivity of the cells to ionizing radiation and/or to promote the treatment of diseases which are treatable with ionizing radiation.

The term "chemosensitizer", as used herein, is defined as a molecule, preferably a low molecular weight molecule, administered to animals in therapeutically effective amounts to increase the sensitivity of cells to chemotherapy and/or promote the treatment of diseases which are treatable with chemotherapeutics.

Several mechanisms for the mode of action of radiosensitizers have been suggested in the literature including: hypoxic cell radiosensitizers (e.g., 2-nitroimidazole compounds, and benzotriazine dioxide compounds) mimicking oxygen or alternatively behave like bioreductive agents under hypoxia; non-hypoxic cell radiosensitizers (e.g., halogenated pyrimidines) can be analogoues of DNA bases and preferentially incorporate into the DNA of cancer cells and thereby promote the radiation-induced breaking of DNA molecules and/or prevent the normal DNA repair mechanisms; and various other potential mechanisms of action have been hypothesized for radiosensitizers in the treatment of disease.

Many cancer treatment protocols currently employ radiosensitizers in conjunction with radiation of x-rays. Examples of x-ray activated radiosensitizers include, but are not limited to, the following: metronidazole, misonidazole, desmethylmisonidazole, pimonidazole, etanidazole, nimorazole, mitomycin C, RSU 1069, SR 4233, EO9, RB 6145, nicotinamide, 5-bromodeoxyuridine (BUdR), 5- iododeoxyuridine (IUdR), bromodeoxycytidine, fluorodeoxyuridine (FudR), hydroxyurea, cisplatin, and therapeutically effective analogs and derivatives of the same.

Photodynamic therapy (PDT) of cancers employs visible light as the radiation activator of the sensitizing agent. Examples of photodynamic radiosensitizers include the following, but are not limited to: hematoporphyrin derivatives, Photofrin, benzoporphyrin derivatives, tin etioporphyrin, pheoborbide-a, bacteriochlorophyll-a, naphthalocyanines, phthalocyanines, zinc phthalocyanine, and therapeutically effective analogs and derivatives of the same.

Radiosensitizers may be administered in conjunction with a therapeutically effective amount of one or more other compounds, including but not limited to: compounds which promote the incorporation of radiosensitizers to the target cells; compounds which control the flow of therapeutics, nutrients, and/or oxygen to the target cells; chemotherapeutic agents which act on the tumour with or without additional radiation; or other therapeutically effective compounds for treating cancer or other diseases.

Chemosensitizers may be administered in conjunction with a therapeutically effective amount of one or more other compounds, including but not limited to: compounds which promote the incorporation of chemosensitizers to the target cells; compounds which control the flow of therapeutics, nutrients, and/or oxygen to the target cells; chemotherapeutic agents which act on the tumour or other therapeutically effective compounds for treating cancer or other disease. Calcium antagonists, for example verapamil, are found useful in combination with antineoplastic agents to establish chemo sensitivity in tumor cells resistant to accepted chemotherapeutic agents and to potentiate the efficacy of such compounds in drug-sensitive malignancies.

The invention relates to compounds of Formula (I) and N-oxides, pharmaceutically acceptable addition salts, and solvates thereof, for use as a medicament.

The invention also relates to compounds of Formula (I) and N-oxides, pharmaceutically acceptable addition salts, and solvates thereof, for use in the inhibition of PI3Kβ kinase activity and optionally also for use in the inhibition of PI3Kδ.

The compounds of the present invention can be "anti-cancer agents", which term also encompasses "anti-tumor cell growth agents" and "anti-neoplastic agents".

The invention also relates to compounds of Formula (I) and N-oxides, pharmaceutically acceptable addition salts, and solvates thereof, for use in the treatment of diseases mentioned above.

The invention also relates to compounds of Formula (I) and N-oxides, pharmaceutically acceptable addition salts, and solvates thereof, for the treatment or prevention, in particular for the treatment, of said diseases.

The invention also relates to compounds of Formula (I) and N-oxides, pharmaceutically acceptable addition salts, and solvates thereof, for the treatment or prevention, in particular in the treatment, of PI3Kβ mediated diseases or conditions.

The invention also relates to compounds of Formula (I) and N-oxides, pharmaceutically acceptable addition salts, and solvates thereof, for the treatment or prevention, in particular in the treatment, of PI3Kβ and optionally PI3Kδ mediated diseases or conditions.

The invention also relates to the use of compounds of Formula (I) and N-oxides, pharmaceutically acceptable addition salts, and solvates thereof, for the manufacture of a medicament.

The invention also relates to the use of compounds of Formula (I) and N-oxides, pharmaceutically acceptable addition salts, and solvates thereof, for the manufacture of a medicament for the inhibition of PI3Kβ.

The invention also relates to the use of compounds of Formula (I) and N-oxides, pharmaceutically acceptable addition salts, and solvates thereof, for the manufacture of a medicament for the inhibition of PI3Kβ and optionally also for the inhibition of PI3Kδ.

The invention also relates to the use of compounds of Formula (I) and N-oxides, pharmaceutically acceptable addition salts, and solvates thereof, for the manufacture of a medicament for the treatment or prevention, in particular for the treatment, of any one of the disease conditions mentioned hereinbefore.

The invention also relates to the use of compounds of Formula (I) and N-oxides, pharmaceutically acceptable addition salts, and solvates thereof, for the manufacture of a medicament for the treatment of any one of the disease conditions mentioned hereinbefore.

The compounds of Formula (I) and N-oxides, pharmaceutically acceptable addition salts, and solvates thereof, can be administered to mammals, preferably humans for the treatment or prevention of any one of the diseases mentioned hereinbefore.

In view of the utility of the compounds of Formula (I) and N-oxides, pharmaceutically acceptable addition salts, and solvates thereof, there is provided a method of treating warm-blooded animals, including humans, suffering from or a method of preventing warm-blooded animals, including humans, to suffer from any one of the diseases mentioned hereinbefore.

Said methods comprise the administration, i.e. the systemic or topical administration, preferably oral administration, of an effective amount of a compound of Formula (I) or a N-oxide, a pharmaceutically acceptable addition salt, or a solvate thereof, to warm-blooded animals, including humans.

Those of skill in the treatment of such diseases could determine the effective therapeutic daily amount from the test results presented hereinafter. An effective therapeutic daily amount would be from about 0.005 mg/kg to 50 mg/kg, in particular 0.01 mg/kg to 50 mg/kg body weight, more in particular from 0.01 mg/kg to 25 mg/kg body weight, preferably from about 0.01 mg/kg to about 15 mg/kg, more preferably from about 0.01 mg/kg to about 10 mg/kg, even more preferably from about 0.01 mg/kg to about 1 mg/kg, most preferably from about 0.05 mg/kg to about 1 mg/kg body weight. The amount of a compound according to the present invention, also referred to here as the active ingredient, which is required to achieve a therapeutically effect will of course, vary on case-by-case basis, for example with the particular compound, the route of administration, the age and condition of the recipient, and the particular disorder or disease being treated.

A method of treatment may also include administering the active ingredient on a regimen of between one and four intakes per day. In these methods of treatment the compounds according to the invention are preferably formulated prior to administration. As described herein below, suitable pharmaceutical formulations are prepared by known procedures using well known and readily available ingredients.

The compounds of the present invention, that can be suitable to treat or prevent cancer or cancer-related conditions, may be administered alone or in combination with one or more additional therapeutic agents. Combination therapy includes administration of a single pharmaceutical dosage formulation which contains a compound of Formula (I), a N-oxide, a pharmaceutically acceptable addition salt, or a solvate thereof, and one or more additional therapeutic agents, as well as administration of the compound of Formula (I), a N-oxide, a pharmaceutically acceptable addition salt, or a solvate thereof, and each additional therapeutic agents in its own separate pharmaceutical dosage formulation. For example, a compound of Formula (I), a N-oxide, a pharmaceutically acceptable addition salt, or a solvate thereof, and a therapeutic agent may be administered to the patient together in a single oral dosage composition such as a tablet or capsule, or each agent may be administered in separate oral dosage formulations.

While it is possible for the active ingredient to be administered alone, it is preferable to present it as a pharmaceutical composition.

Accordingly, the present invention further provides a pharmaceutical composition comprising a pharmaceutically acceptable carrier and, as active ingredient, a therapeutically effective amount of a compound of Formula (I), a N-oxide, a pharmaceutically acceptable addition salt, or a solvate thereof.

The carrier or diluent must be "acceptable" in the sense of being compatible with the other ingredients of the composition and not deleterious to the recipients thereof.

For ease of administration, the subject compounds may be formulated into various pharmaceutical forms for administration purposes. The compounds according to the invention, in particular the compounds of Formula (I) and N-oxides, pharmaceutically acceptable addition salts, and solvates thereof, or any subgroup or combination thereof may be formulated into various pharmaceutical forms for administration purposes. As appropriate compositions there may be cited all compositions usually employed for systemically administering drugs.

To prepare the pharmaceutical compositions of this invention, an effective amount of the particular compound as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable solutions containing a compound of Formula (I), a N-oxide, a pharmaceutically acceptable addition salt, or a solvate thereof, may be formulated in an oil for prolonged action. Appropriate oils for this purpose are, for example, peanut oil, sesame oil, cottonseed oil, corn oil, soybean oil, synthetic glycerol esters of long chain fatty acids and mixtures of these and other oils. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment. Acid or base addition salts of compounds of Formula (I) due to their increased water solubility over the corresponding base or acid form, are more suitable in the preparation of aqueous compositions.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

In order to enhance the solubility and/or the stability of the compounds of Formula (I) and N-oxides, pharmaceutically acceptable addition salts, and solvates thereof, in pharmaceutical compositions, it can be advantageous to employ α-, β- or γ-cyclodextrins or their derivatives, in particular hydroxyalkyl substituted cyclodextrins, e.g. 2-hydroxypropyl-β-cyclodextrin or sulfobutyl-β-cyclodextrin. Also co-solvents such as alcohols may improve the solubility and/or the stability of the compounds according to the invention in pharmaceutical compositions.

Depending on the mode of administration, the pharmaceutical composition will preferably comprise from 0.05 to 99 % by weight, more preferably from 0.1 to 70 % by weight, even more preferably from 0.1 to 50 % by weight of the compound of Formula (I), a N-oxide, a pharmaceutically acceptable addition salt, or a solvate thereof, and from 1 to 99.95 % by weight, more preferably from 30 to 99.9 % by weight, even more preferably from 50 to 99.9 % by weight of a pharmaceutically acceptable carrier, all percentages being based on the total weight of the composition.

As another aspect of the present invention, a combination of a compound of the present invention with another anticancer agent is envisaged, especially for use as a medicine, more specifically for use in the treatment of cancer or related diseases.

For the treatment of the above conditions, the compounds of the invention may be advantageously employed in combination with one or more other medicinal agents, more particularly, with other anti-cancer agents or adjuvants in cancer therapy. Examples of anti-cancer agents or adjuvants (supporting agents in the therapy) include but are not limited to:
- platinum coordination compounds for example cisplatin optionally combined with amifostine, carboplatin or oxaliplatin;
- taxane compounds for example paclitaxel, paclitaxel protein bound particles (Abraxane™) or docetaxel;
- topoisomerase I inhibitors such as camptothecin compounds for example irinotecan, SN-38, topotecan, topotecan hcl;
- topoisomerase II inhibitors such as anti-tumour epipodophyllotoxins or podophyllotoxin derivatives for example etoposide, etoposide phosphate or teniposide;
- anti-tumour vinca alkaloids for example vinblastine, vincristine or vinorelbine;
- anti-tumour nucleoside derivatives for example 5-fluorouracil, leucovorin, gemcitabine, gemcitabine hcl, capecitabine, cladribine, fludarabine, nelarabine;
- alkylating agents such as nitrogen mustard or nitrosourea for example cyclophosphamide, chlorambucil, carmustine, thiotepa, mephalan (melphalan), lomustine, altretamine, busulfan, dacarbazine, estramustine, ifosfamide optionally in combination with mesna, pipobroman, procarbazine, streptozocin, temozolomide, uracil;
- anti-tumour anthracycline derivatives for example daunorubicin, doxorubicin optionally in combination with dexrazoxane, doxil, idarubicin, mitoxantrone, epirubicin, epirubicin hcl, valrubicin;
- molecules that target the IGF-1 receptor for example picropodophilin;
- tetracarcin derivatives for example tetrocarcin A;
- glucocorticoïden for example prednisone;
- antibodies for example trastuzumab (HER2 antibody), rituximab (CD20 antibody), gemtuzumab, gemtuzumab ozogamicin, cetuximab, pertuzumab, bevacizumab, alemtuzumab, eculizumab, ibritumomab tiuxetan, nofetumomab, panitumumab, tositumomab, CNTO 328;
- estrogen receptor antagonists or selective estrogen receptor modulators or inhibitors of estrogen synthesis for example tamoxifen, fulvestrant, toremifene, droloxifene, faslodex, raloxifene or letrozole;
- aromatase inhibitors such as exemestane, anastrozole, letrazole, testolactone and vorozole;
- differentiating agents such as retinoids, vitamin D or retinoic acid and retinoic acid metabolism blocking agents (RAMBA) for example accutane;
- DNA methyl transferase inhibitors for example azacytidine or decitabine;
- antifolates for example pemetrexed disodium;
- antibiotics for example antinomycin D, bleomycin, mitomycin C, dactinomycin, carminomycin, daunomycin, levamisole, plicamycin, mithramycin;
- antimetabolites for example clofarabine, aminopterin, cytosine arabinoside or methotrexate, azacitidine, cytarabine, floxuridine, pentostatin, thioguanine;
- apoptosis inducing agents and antiangiogenic agents such as Bcl-2 inhibitors for example YC 137, BH 312, ABT 737, gossypol, HA 14-1, TW 37 or decanoic acid;
- tubuline-binding agents for example combrestatin, colchicines or nocodazole;
- kinase inhibitors (e.g. EGFR (epithelial growth factor receptor) inhibitors, MTKI (multi target kinase inhibitors), mTOR inhibitors) for example flavoperidol, imatinib mesylate, erlotinib, gefitinib, dasatinib, lapatinib, lapatinib ditosylate, sorafenib, sunitinib, sunitinib maleate, temsirolimus;
- farnesyltransferase inhibitors for example tipifarnib;
- histone deacetylase (HDAC) inhibitors for example sodium butyrate, suberoylanilide hydroxamic acid (SAHA), depsipeptide (FR 901228), NVP-LAQ824, R306465, JNJ-26481585, trichostatin A, vorinostat;
- Inhibitors of the ubiquitin-proteasome pathway for example PS-341, MLN .41 or bortezomib;
- Yondelis;
- Telomerase inhibitors for example telomestatin;
- Matrix metalloproteinase inhibitors for example batimastat, marimastat, prinostat or metastat;
- Recombinant interleukins for example aldesleukin, denileukin diftitox, interferon alfa 2a, interferon alfa 2b, peginterferon alfa 2b;
- MAPK inhibitors;
- Retinoids for example alitretinoin, bexarotene, tretinoin;
- Arsenic trioxide;
- Asparaginase;
- Steroids for example dromostanolone propionate, megestrol acetate, nandrolone (decanoate, phenpropionate), dexamethasone;
- Gonadotropin releasing hormone agonists or antagonists for example abarelix, goserelin acetate, histrelin acetate, leuprolide acetate;
- Thalidomide, lenalidomide;
- Mercaptopurine, mitotane, pamidronate, pegademase, pegaspargase, rasburicase;
- BH3 mimetics for example ABT-737;
- MEK inhibitors for example PD98059, AZD6244, CI-1040;
- colony-stimulating factor analogs for example filgrastim, pegfilgrastim, sargramostim; erythropoietin or analogues thereof (e.g. darbepoetin alfa); interleukin 11; oprelvekin; zoledronate, zoledronic acid; fentanyl; bisphosphonate; palifermin;
- a steroidal cytochrome P450 17alpha-hydroxylase-17,20-lyase inhibitor (CYP17), e.g. abiraterone, abiraterone acetate;
- Glycolysis inhibitors, such as 2-deoxyglucose;
- mTOR inhibitors such as rapamycins and rapalogs, and mTOR kinase inhibitors;
- PI3K inhibitors and dual mTOR/PI3K inhibitors;
- autophagy inhibitors, such as chloroquine and hydroxy-chloroquine;
- antibodies that re-activate the immune response to tumors, for example nivolumab (anti-PD-1), lambrolizumab (anti-PD-1), ipilimumab (anti-CTLA4), and MPDL3280A (anti-PD-L1).

The compounds of the invention can also be advantageously combined with anti-androgen therapies including androgen receptor antagonists and inhibitors of androgen biosynthesis in PTEN-negative prostate cancers.

The present invention further relates to a product containing as first active ingredient a compound according to the invention and as further active ingredient one or more anticancer agents, as a combined preparation for simultaneous, separate or sequential use in the treatment of patients suffering from cancer.

The one or more other medicinal agents and the compound according to the present invention may be administered simultaneously (e.g. in separate or unitary compositions) or sequentially in either order. In the latter case, the two or more compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular other medicinal agent and compound of the present invention being administered, their route of administration, the particular tumour being treated and the particular host being treated. The optimum method and order of administration and the dosage amounts and regime can be readily determined by those skilled in the art using conventional methods and in view of the information set out herein.

The weight ratio of the compound according to the present invention and the one or more other anticancer agent(s) when given as a combination may be determined by the person skilled in the art. Said ratio and the exact dosage and frequency of administration depends on the particular compound according to the invention and the other anticancer agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of Formula (I) and another anticancer agent may range from 1/10 to 10/1, more in particular from 1/5 to 5/1, even more in particular from 1/3 to 3/1.

The platinum coordination compound is advantageously administered in a dosage of 1 to 500mg per square meter (mg/m²) of body surface area, for example 50 to 400 mg/m², particularly for cisplatin in a dosage of about 75 mg/m² and for carboplatin in about 300mg/m² per course of treatment.

The taxane compound is advantageously administered in a dosage of 50 to 400 mg per square meter (mg/m²) of body surface area, for example 75 to 250 mg/m², particularly for paclitaxel in a dosage of about 175 to 250 mg/m² and for docetaxel in about 75 to 150 mg/m² per course of treatment.

The camptothecin compound is advantageously administered in a dosage of 0.1 to 400 mg per square meter (mg/m²) of body surface area, for example 1 to 300 mg/m², particularly for irinotecan in a dosage of about 100 to 350 mg/m² and for topotecan in about 1 to 2 mg/m² per course of treatment.

The anti-tumour podophyllotoxin derivative is advantageously administered in a dosage of 30 to 300 mg per square meter (mg/m²) of body surface area, for example 50 to 250mg/m², particularly for etoposide in a dosage of about 35 to 100 mg/m² and for teniposide in about 50 to 250 mg/m² per course of treatment.

The anti-tumour vinca alkaloid is advantageously administered in a dosage of 2 to 30 mg per square meter (mg/m²) of body surface area, particularly for vinblastine in a dosage of about 3 to 12 mg/m² , for vincristine in a dosage of about 1 to 2 mg/m², and for vinorelbine in dosage of about 10 to 30 mg/m² per course of treatment.

The anti-tumour nucleoside derivative is advantageously administered in a dosage of 200 to 2500 mg per square meter (mg/m²) of body surface area, for example 700 to 1500 mg/m², particularly for 5-FU in a dosage of 200 to 500mg/m², for gemcitabine in a dosage of about 800 to 1200 mg/m² and for capecitabine in about 1000 to 2500 mg/m² per course of treatment.

The alkylating agents such as nitrogen mustard or nitrosourea is advantageously administered in a dosage of 100 to 500 mg per square meter (mg/m²) of body surface area, for example 120 to 200 mg/m², particularly for cyclophosphamide in a dosage of about 100 to 500 mg/m² , for chlorambucil in a dosage of about 0.1 to 0.2 mg/kg, for carmustine in a dosage of about 150 to 200 mg/m² , and for lomustine in a dosage of about 100 to 150 mg/m² per course of treatment.

The anti-tumour anthracycline derivative is advantageously administered in a dosage of 10 to 75 mg per square meter (mg/m²) of body surface area, for example 15 to 60 mg/m², particularly for doxorubicin in a dosage of about 40 to 75 mg/m², for daunorubicin in a dosage of about 25 to 45mg/m², and for idarubicin in a dosage of about 10 to 15 mg/m² per course of treatment.

The antiestrogen agent is advantageously administered in a dosage of about 1 to 100 mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

Antibodies are advantageously administered in a dosage of about 1 to 5 mg per square meter (mg/m²) of body surface area, or as known in the art, if different. Trastuzumab is advantageously administered in a dosage of 1 to 5 mg per square meter (mg/m²) of body surface area, particularly 2 to 4mg/m² per course of treatment.

These dosages may be administered for example once, twice or more per course of treatment, which may be repeated for example every 7, 14, 21 or 28 days.

### Examples

The following examples illustrate the present invention.

When a stereocenter is indicated with 'RS' this means that a racemic mixture was obtained.

Hereinafter, the term 'ACN' means acetonitrile, 'AcOH' means acetic acid, 'aq.' means aqueous, 'Ar' means Argon, 'BINAP' means 2,2'-bis(diphenylphosphino)-1,1'-bi-naphthyl, 'BOC' means tert-butyloxycarbonyl, 'Boc₂O' means di-tert-butyl dicarbonate, 'celite®' means diatomaceous earth, 'DavePhos' means 2-Dicyclohexyl-phosphino-2'-(N,N-dimethylamino)biphenyl, 'DCM' means dichloromethane, 'DIPE' means diisopropyl ether, 'DIPEA' means diisopropylethylamine, 'DMF' means dimethylformamide, 'DPPP' means 1,3-bis(diphenylphosphino)propane, 'Et₂O' means diethyl ether, 'EtOAc' means ethyl acetate, 'EtOH' means ethanol, 'h' means hours(s), 'HPLC' means High-performance Liquid Chromatography, 'LC/MS' means Liquid Chromatography/Mass Spectrometry, 'MeOH' means methanol, 'min' means minute(s), 'M.P.' or 'm.p.' means melting point, 'MsCl' means methanesulfonyl chloride, 'NaBH(OAc)₃' means sodium triacetoxyborohydride, 'NaRi' means Raney Nickel, 'NMR' means Nuclear Magnetic Resonance, 'Pd(OAC)₂' means palladium (II) acetate, 'Quant.' means quantitative, 'rt' means room temperature, 'Rt' means retention time, 'RuPhos palladacycle' means chloropalladium, dicyclohexyl-[2-[2,6-di(propan-2-yloxy)phenyl]phenyl]phosphane, 2-methoxy-2-methylpropane, 2-phenylethanamine, 'sat.' means saturated, 'SeO₂' means selenium dioxide, 'TBAF' means tetrabutylammonium fluoride, 'TBDMS' or 'SMDBT' means tert-butyldimethylsilyl, 'TEA' means triethylamine, 'TFA' means trifluoroacetic acid, 'THF' means tetrahydrofuran, 'TLC' means thin layer chromatography.

### A. Preparation of the intermediates

### Example A1

### Preparation of intermediate 1:

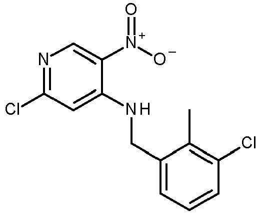

2-methyl-3-chlorobenzylamine (1.33 g, 8.55 mmol) was added to a solution of 2,4-dichloro-5-nitropyridine (1.50 g, 7.77 mmol) and TEA (3.2 mL, 23.32 mmol) in THF (30 mL). The reaction mixture was stirred overnight at room temperature, diluted with DCM and washed with water. The organic layer was decanted, dried over MgSO₄, filtered and evaporated to dryness. The residue was taken up with Et₂O and the precipitate was filtered and dried under vacuum to give 2 g of intermediate 1 (82% yield).

### Preparation of intermediate 2:

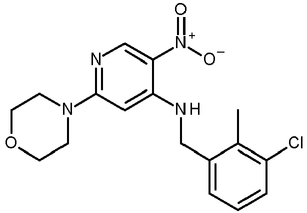

A mixture of intermediate 1 (2.00 g, 6.41 mmol) in morpholine (15 mL) was heated at 100 °C for 1 h. The reaction mixture was cooled to room temperature and CH₃CN was added. The precipitate was filtered, washed with Et₂O and dried under vacuo to give a first batch of intermediate 2. The filtrate was evaporated to dryness and gathered with the first batch. The residue was dissolved in DCM and washed with a 10% aqueous solution of K₂CO₃. The organic layer was decanted, dried over MgSO₄, filtered and evaporated to dryness. The residue was taken up with CH₃CN and the precipitate was filtered, washed with Et₂O and dried to give 2.18 g of intermediate 2 (94% yield). The intermediate below was prepared following the same method than intermediate 2

| Intermediate number | Structure |
|---|---|
| Intermediate 30 (from intermediate 29 and morpholine) | 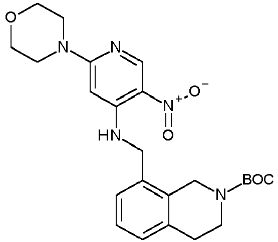 |

Preparation of intermediate 3:

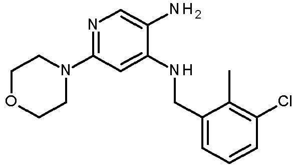

A suspension of intermediate 2 (500.00 mg, 1.26 mmol) and RaNi (547.24 mg, 9.32 mmol) in MeOH (30 mL) was hydrogenated at room temperature under H₂ (1 atmosphere) for 3 h. The catalyst was removed by filtration over a pad of celite® and the filtrate was evaporated to dryness (azeotrope with toluene to remove traces of water) to give 432 mg of intermediate 3 (94% yield) which was used immediately in the next reaction step.

### Example A2

Preparation of intermediate 4:

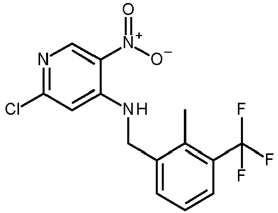

2-methyl-3-(trifluoromethyl)benzylamine (1.00 g, 5.29 mmol) was added to a solution of 2,4-dichloro-5-nitropyridine (927.39 mg, 4.81 mmol) and TEA (2 mL, 14.42 mmol) in THF (20 mL). The reaction mixture was stirred overnight at room temperature, diluted with DCM and washed with water. The organic layer was filtered through Chromabond® and evaporated to dryness. The residue was taken up with Et₂O and the precipitate was filtered and dried under vacuum to give 1.65 g of intermediate 4 (99% yield).

Preparation of intermediate 5:

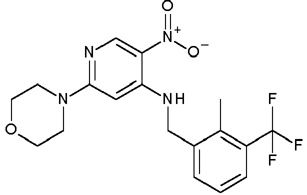

A mixture of intermediate 4 (1.65 g, 4.77 mmol) in morpholine (10 mL) was heated at 100 °C for 1 h. The reaction mixture was cooled to room temperature and DCM and water were added. The organic layer was decanted, dried over MgSO₄, filtered and evaporated to dryness. The residue was taken up with CH₃CN and the precipitate was filtered, washed with Et₂O and dried under vacuum to give 1.6 g of intermediate 5 (85% yield).

Preparation of intermediate 6:

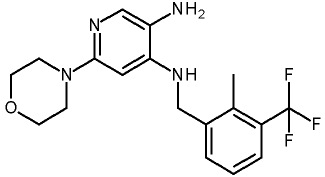

A suspension of intermediate 5 (1.12 g, 2.83 mmol) and RaNi (1.00 g, 17.04 mmol) in MeOH (70 mL) was hydrogenated at room temperature under H₂ (1 atmosphere) for 3 h. The catalyst was removed by filtration over a pad of celite and the filtrate was evaporated to dryness (azeotrope with toluene to remove traces of water) to give 949 mg of intermediate 6 (92% yield) which was used immediately in the next reaction step.

### Example A3

Preparation of intermediate 6:

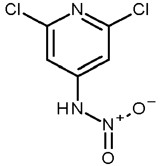

4-amino-2,6-dichloropyridine (40.00 g, 245.39 mmol) was slowly added to H₂SO₄ (280 mL) at rt and the reaction mixture was cooled to 5 °C. HNO₃ (53.00 g, 841.10 mmol) was added and the reaction mixture was stirred at 10 °C for 1 hours. The reaction mixture was slowly poured into ice water and the precipitated solid was filtered and dried under vacuum to give 50 g of intermediate 11 (98% yield, light yellow solid).

Preparation of intermediate 12:

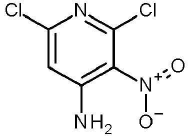

Sulfuric acid (32 mL) was placed in a 250 mL round-bottomed flask fitted with a magnetic stir bar and an internal thermometer. Intermediate 11 (6.19 g, 29.7 mmol) was added portionwise (the internal temperature must remain below 40 °C). The mixture was heated to 100 °C for 1 h, poured into ice water (300 mL) and the pH of the solution was adjusted to 9.5 by addition of 6 N aq. NaOH solution (250 mL). The suspension was stirred for 30 min at rt. The precipitate was collected by filtration, suspended in water (150 mL) and stirred at rt for 30 min. The solid was collected by filtration and dried under high vacuum overnight to give 5.47 g of intermediate 12 (88 % yield, off-white solid).

Preparation of intermediate 13:

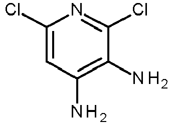

To a solution of intermediate 12 (37.00 g, 177.88 mmol) in HCl (60 mL) and EtOH/H₂O solution (400 mL, 1:1, v/v) was added Iron powder (30.00 g, 537.20 mmol). The reaction mixture was stirred at 100 °C for 3 hours. The reaction mixture was concentrated and diluted with water. The suspended solution was basified by aqueous NaHCO₃ solution until pH=9. The precipitate was filtered and diluted in a mixture of EtOAc/MeOH (8:1, v/v). The remaining solid was filtered and the filtrate was concentrated under vacuum to afford a first batch of intermediate 13.

The basic filtrate was extracted with EtOAc (3 times) and the combined organic layers were concentrated under vacuum to give a second batch of intermediate 13.

The two batches were combined and the resulting solid residue was triturated with ether, filtered and dried under vacuum to give 30 g of intermediate 13 (95% yield, light yellow solid).

The intermediate in the Table below was prepared by using an analogous method as described for the preparation of intermediate 13, starting from the respective starting materials.

| Intermediate number | Structure |
|---|---|
| Intermediate 31 (from intermediate 30) | 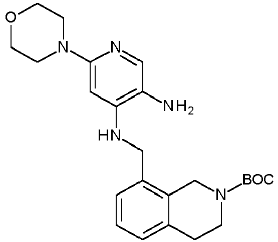 |

Preparation of intermediate 14:

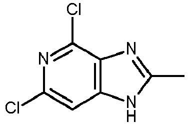

To a solution of intermediate 13 (16.00 g, 89.88 mmol) in EtOH (60 mL) was added triethyl orthoacetate (50 mL) and conc. HCl (4 mL) and the mixture was stirred at 100 °C for 16 hours. The mixture was concentrated and diluted with water and ethyl acetate. The layers were separated and the organic layer was dried over MgSO₄, filtered and concentrated under vacuum. The residue was purified by column chromatography over silica gel (mobile phase gradient: from 100% petrol, 0% EtOAc to 0% petrol, 100% EtOAc). The product containing fractions were collected and the solvent was evaporated to give 11 g of intermediate 14 (57% yield, yellow solid).

The intermediate in the Table below was prepared by using an analogous method as described for the preparation of intermediate 14, starting from the respective starting materials.

| Intermediate number | Structure |
|---|---|
| Intermediate 32 (from intermediate 31) | 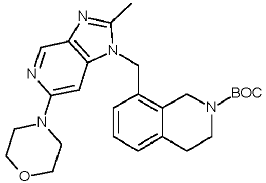 |

Preparation of intermediate 15:

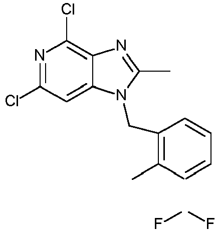

2-methyl-3-(trifluoromethyl)benzyl bromide (6.26 g, 24.7 mmol) was added to a mixture of intermediate 14 (5.00 g, 24.7 mmol) and K₂CO₃ (6.84 g, 49.5 mmol) in CH₃CN (500 mL). The reaction mixture was stirred for 2 h at 85 °C. Water and EtOAc were added to the mixture. The layers were separated and the organic layer was washed with water (twice), brine and water. The organic layer was dried over MgSO₄, filtered and evaporated to dryness. The residue was taken up with DIPE to give 9.23 g of intermediate 15 (quant, yield).

### Example A4

Preparation of intermediate 7:

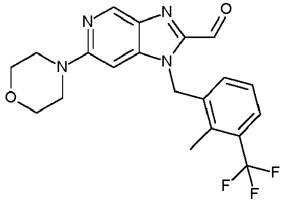

A mixture of compound 2 (1.00 g, 2.56 mmol) and SeO₂ (426.00 mg, 3.84 mmol) in 1,4-dioxane (15 mL) was refluxed for 3 h. The reaction mixture was cooled to room temperature, diluted with DCM and a 10% aqueous solution of K₂CO₃ was added. The organic layer was retrieved, filtered through Chromabond® and evaporated to dryness to give 1.04 g of intermediate 7 (quant, yield) which was used in the next step without further purification.

The intermediate in the Table below was prepared by using an analogous method as described for the preparation of intermediate 7, starting from the respective starting materials.

| Intermediate number | Structure |
|---|---|
| Intermediate 18 (from compound 1) | 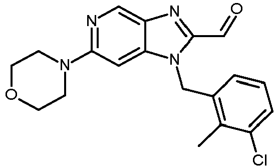 |

### Example A5

Preparation of intermediate 8:

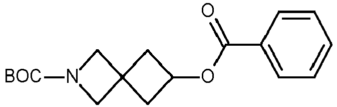

Benzoyl chloride (0.56 mL, 4.78 mmol) was added to a solution of 2-boc-6-hydroxy-2-aza-spiro[3,3]heptane (850.00 mg, 3.99 mmol) and TEA (0.77 mL, 5.58 mmol) in DCM (17 mL) at room temperature. The reaction mixture was stirred overnight at room temperature, diluted with DCM and washed with a 10% aqueous solution of K₂CO₃. The organic layer was decanted, dried over MgSO₄, filtered and evaporated to dryness. The crude residue was purified by chromatography over silica gel (irregular SiOH, 24 g, mobile phase gradient: from 0% MeOH, 100% DCM to 3% MeOH, 97% DCM). The pure fractions were collected and evaporated to dryness to give 1.08 g of intermediate 8 (85% yield).

Preparation of intermediate 9:

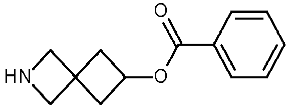

TFA (5 mL, 65.34 mmol) was added dropwise to a solution of intermediate 8 (1.08 g, 3.40 mmol) in DCM (50 mL) at 0 °C and the reaction mixture was stirred at room temperature for 2 hours. A 10% aqueous solution of K₂CO₃ was added and the organic layer was decanted, dried over MgSO₄, filtered and evaporated to dryness to give 739 mg of intermediate 9 which was used in the next step without further purification.

Preparation of intermediate 10:

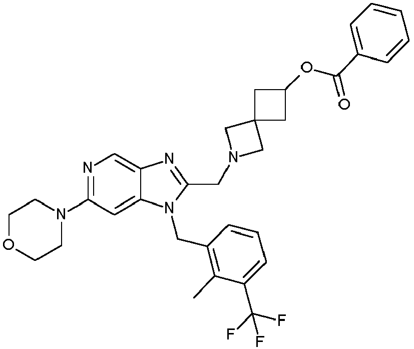

A mixture of intermediate 7 (260.00 mg, 0.64 mmol) and intermediate 9 (279.38 mg, 1.29 mmol) in DCM (6 mL) was stirred at room temperature for 18 hours. Sodium triacetoxyborohydride (272.53 mg, 1.29 mmol) was added and the reaction mixture was stirred at room temperature for 24 hours. The reaction mixture was diluted with DCM and poured onto a 10% aqueous solution of K₂CO₃. The organic layer was decanted, dried over MgSO₄, filtered and evaporated to dryness. The residue was purified by chromatography over silica gel (irregular SiOH, 24 g, mobile phase gradient: from 3% MeOH, 97% DCM to 5% MeOH, 95% DCM). The product containing fractions were collected and evaporated to dryness to give 250 mg of intermediate 10 (64% yield). The intermediate in the Table below was prepared by using an analogous method as described for the preparation of intermediate 10, starting from the respective starting materials.

| Intermediate number | Structure |
|---|---|
| Intermediate 20 (from intermediates 7 and 19) | 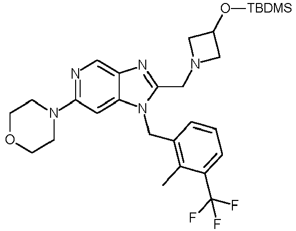 |

### Example A6

Preparation of intermediate 16:

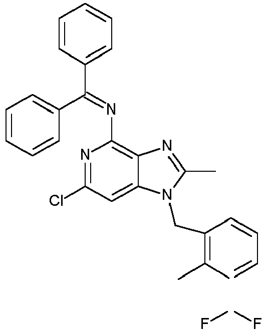

In a sealed tube, a mixture of intermediate 15 (1.70 g, 4.54 mmol), benzhydrylideneamine (1.14 mL, 6.82 mmol) and Cs₂CO₃ (4.44 g, 13.63 mmol) in 1,4-dioxane (50 mL) was degased with N₂. BINAP (141.00 mg, 0.23 mmol) and Pd(OAc)₂ (51.00 mg, 0.23 mmol) were added. The reaction mixture was heated at 100 °C for 48 h. The reaction mixture was cooled to room temperature, water and EtOAc were added and the mixture was filtered through a pad of celite. The organic layer was decanted, washed with water and brine, dried over MgSO₄, filtered and evaporated to dryness. The crude residue was purified via preparative LC (Stationary phase: irregular SiOH 40 µm, 120 g, mobile phase gradient: from 90% heptane, 10% EtOAc to 60% heptane, 40% EtOAc) to give 779 mg of intermediate 16 (33% yield).

Preparation of intermediate 37:

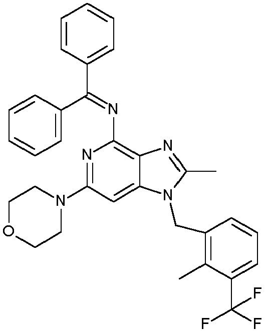

The experiment was repeated 9 times on 50 mg of intermediate 16. Morpholine (10.17 µL, 0.12 mmol) was added to a solution of intermediate 16 (50.00 mg, 0.096 mmol) and Cs₂CO₃ (94.17 mg, 0.29 mmol) in 1,4-dioxane (1 mL). The solution was degased with N₂ and Pd(OAc)₂ (1.1 mg, 0.005 mmol) and DavePhos (1.90 mg, 0.005 mmol) were added. The reaction mixture was heated at 120 °C for 24 h. Water and EtOAc were added and the reaction mixture was filtered over a pad of celite. The organic layer was extracted, washed with water and brine, dried over MgSO₄, filtered and evaporated to dryness. The nine reactions were combined and the resulting crude residue was purified by preparative LC (Stationary phase: irregular SiOH 15-40 µm, 80 g MERCK, mobile phase gradient: from 80% heptane, 20% EtOAc to 40% heptane, 60% EtOAc) to give 200 mg of intermediate 37 (40% yield).

### Example A7

Preparation of intermediate 17:

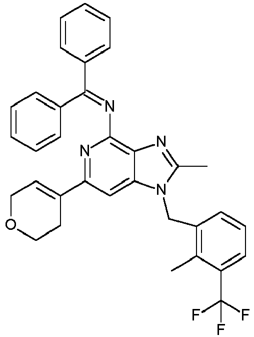

To a solution of intermediate 16 (250.00 mg, 0.48 mmol) in H₂O (730 µL) and 1,4-dioxane (37.5 mL) were added 2-(3,6-Dihydro-2H-pyran-4-yl)-4,4,5,5-tetramethyl-1,3,2-dioxaborolane (303.60 mg, 1.45 mmol) and K₃PO₄ (306.76 mg, 1.45 mmol). The reaction mixture was degased with N₂ and RuPhos palladacycle (9.84 mg, 0.012 mmol) was added to the mixture. The reaction mixture was heated at 105 °C overnight. Water and EtOAc were added and the mixture was filtered over a pad of celite. The organic layer was decanted, washed with water and brine, dried over MgSO₄, filtered and concentrated under vacuum. A purification was performed via preparative LC (Stationary phase: irregular SiOH 40 µm, 40 g, mobile phase gradient: from 100% DCM, 0% MeOH to 97% DCM, 3% MeOH) to give 125 mg of intermediate 17 (46% yield).

The intermediate in the Table below was prepared by using an analogous method as described for the preparation of intermediate 17, starting from the respective starting materials.

| Intermediate number | Structure |
|---|---|
| Intermediate 35 (from intermediate 4) | 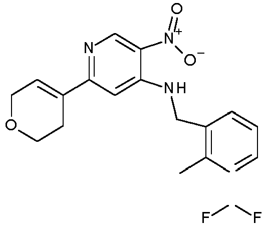 |

### Example A8

Preparation of intermediate 36:

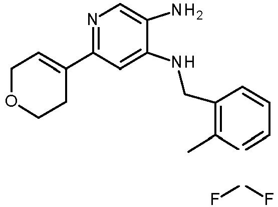

Iron powder (198.05 mg, 1.18 mmol) and AcOH (1.35 mL, 23.64 mmol) were added to a solution of intermediate 35 (465.00 mg, 1.18 mmol) in MeOH (6.2 mL) at rt. The reaction mixture was heated at 80 °C overnight. Water and EtOAc were added and the mixture was filtered through a pad of celite®. The organic layer was extracted, washed with water and brine, dried over MgSO₄, filtered and evaporated to give 289 mg of intermediate 36 (67% yield).

### Example A9

Preparation of intermediate 19:

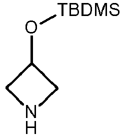

TBDMSCl (3.30 g, 21.91 mmol) was added to a solution of azetidin-3-ol (2.00 g, 18.26 mmol) and TEA (7.61 mL, 54.77 mmol) in DCM (60 mL) at room temperature. The reaction mixture was stirred overnight at room temperature and washed with aqueous saturated Na₂CO₃ (100 mL). The organic layer was decanted, dried over MgSO₄, filtered and evaporated to dryness. The crude residue was purified by chromatography over silica gel (irregular SiOH, 40 g, mobile phase gradient: from 0.5% NH₄OH, 5% MeOH, 95% DCM to 1.5% NH₄OH, 15% MeOH, 85% DCM). The product containing fractions were collected and evaporated to dryness to give 2.9 g of intermediate 19 (85% yield).

Example A10

Preparation of intermediate 21:

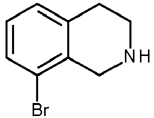

NaBH3CN (30.20 g, 480.64 mmol) was added to a mixture of 8-bromoisoquinoline (20.00 g, 96.13 mmol) in MeOH (300 mL) at 0 °C. The resulting mixture was stirred for 10 minutes and Boron trifluoride diethyl etherate (68.22 g, 480.64 mmol) was added dropwise at 0 °C. The resulting mixture was stirred for 1 hour at 0 °C and then refluxed for 4 hours. Sat. Na₂CO₃ (5 mL) was added and solvent was concentrated under reduced pressure. The remaining liquid was poured into water and extracted with CH₂Cl₂. The organic layer was washed with brine, dried over MgSO₄, filtered and evaporated in vacuo to give 20 g of intermediate 21 (98% yield) which was used in the next step without further purification.

Preparation of intermediate 22:

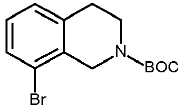

Boc₂O (25.73 g, 117.88 mmol) was added dropwise to a solution of intermediate 21 (25.00 g, 117.88 mmol) and TEA (32.83 mL, 236.00 mmol) in DCM (300 mL) at 0 °C. The resulting mixture was stirred at room temperature for 30 minutes. Sat. citric acid was added to quench the reaction and layers were separated. The organic layer was washed with brine, dried over MgSO₄, filtered and evaporated in vacuo. The crude residue was purified by silica gel column (mobile phase: Petroleum ether/EtOAc, 3/1, v/v) to give 35 g of intermediate 22 (95% yield).

Preparation of intermediate 24:

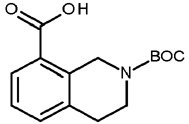

1,2,3,4-tetrahydroisoquinoline-8-carboxylic acid (2.13 g, 9.97 mmol) was dissolved in 50% aqueous 1,4-dioxane solution (25 mL) and Na₂CO₃ (2.11 g, 19.94 mmol) was added followed by Boc₂O (2.61 g, 11.96 mmol). The mixture was stirred at rt for 14 h. Boc₂O (500.00 mg, 2.30 mmol) was added and the reaction mixture was stirred for 1 day. The reaction mixture was concentrated and water (20 mL) was added. The solution was acidified to pH=2 by the addition of 2M HCl. The resulting solid was collected by filtration and dried overnight to give 2.65 g of intermediate 24 (96% yield).

Preparation of intermediate 23:

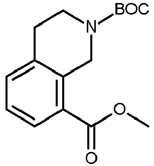

Pd(OAc)₂ (1.58 g, 7.05 mmol) was added to a mixture of intermediate 22 (22.00 g, 70.47 mmol), DPPP (2.91 g, 7.05 mmol) and TEA (49.11 mL, 352.34 mmol) in MeOH/DMF solution (300 mL, 2:1, v/v). The resulting solution was stirred and pressurized to 40 psi with CO at 70 °C for 4 hours. The mixture was cooled to room temperature, diluted with water and extracted with ethyl acetate. The organic layer was washed with water and brine, dried over Na₂SO₄, filtered and concentrated in vacuo. The crude residue was purified by column chromatography on silica gel (mobile phase gradient: from 91% petroleum ether, 9% EtOAc to 83% petroleum ether, 17% EtOAc) to give 12 g of intermediate 23 (59% yield).

Preparation of intermediate 25:

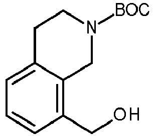

LiAlH₄ (493.00 mg, 13.00 mmol) was added portionwise to a solution of intermediate 23 (5.00 g, 12.01 mmol) in THF (100 mL) at 0 °C. The mixture was stirred at 0 °C for 1 hour. H₂O (500 µL) and a 2N aqueous solution of NaOH (500 µL) were added dropwise to quench the reaction. The mixture was filtered over celite and washed with THF. The filtrate was evaporated and the crude residue was purified by column (mobile phase gradient: from 91% petroleum ether, 9% EtOAc to 80% petroleum ether, 20% EtOAc) to give 3.1 g of intermediate 25 (97% yield).

Alternative preparation of intermediate 25:

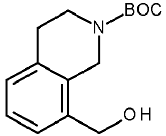

To a solution of intermediate 24 (500.00 mg, 1.80 mmol) in THF (18 mL) at 0 °C was added BH₃.THF complex (1M in THF) (1.80 mL, 1.80 mmol). The solution was allowed to warm to rt and then heated at 40 °C for 2 h. The mixture was then cooled down to 0 °C and BH₃.THF (1.80 mL, 1.80 mmol) was added. The solution was allowed to warm to rt and then heated at 50 °C for 18 h. The crude was then cooled down to 0 °C and BH₃.THF (5.41 mL, 5.41 mmol) was added. The solution was allowed to warm to rt and then refluxed for 18 h. The mixture was cooled to 0 °C and a 3N aqueous solution of HCl was carefully added. The mixture was stirred at rt for 1 h and extracted with EtOAc (three times). The combined organic layers were washed with brine, dried over MgSO₄, filtered and evaporated in vacuo. The crude residue was purified by preparative LC (Irregular SiOH 15-40 µm, 30 g Merck, mobile phase gradient: from DCM 100% to DCM 95%, MeOH 5%) to give 449 mg of intermediate 25 (95% yield, colorless oil).

Preparation of intermediate 26:

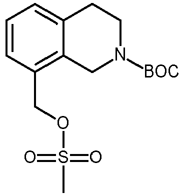

MsCl (5.22 g, 45.6 mmol) was added to a solution of intermediate 25 (10.00 g, 37.98 mmol) and TEA (10.59 mL, 76 mmol) in DCM (100 mL) at 0 °C. The resulting solution was stirred at 0 °C for 2 hours. The resulting mixture was poured into water and extracted with CH₂Cl₂ (3 x 100 mL). The organic layer was washed with sat. NaHCO₃ solution and brine, dried over Na₂SO₄, filtered and evaporated in vacuo to give 12.97 g of intermediate 26 (quant. yield) which was used in the next step without further purification.

Preparation of intermediate 27:

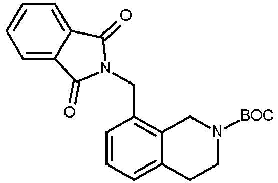

A mixture of intermediate 26 (12.96 g, 37.96 mmol) and phthalimide potassium salt (10.55 g, 56.96 mmol) in 1,4-dioxane (150 mL) was stirred at room temperature overnight. Solvent was removed under vacuo and the residue was poured into water and extracted with EtOAc (3 x 100 mL). The organic layers were combined, washed with brine, dried over Na₂SO₄, filtered and evaporated in vacuo. The crude residue was purified by silica gel column (mobile phase gradient: from 83% petroleum ether, 17% EtOAc to 75% petroleum ether, 25% EtOAc) to give 6 g of intermediate 27 (40% yield, white solid).

Preparation of intermediate 28:

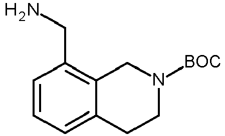

Hydrazine monohydrate (1.21 g, 22.93 mmol based on 95% purity determined by LC/MS) was added to a solution of intermediate 27 (6.00 g, 15.29 mmol) in EtOH (100 mL) at rt. The resulting mixture was refluxed for 3 h. Solvent was evaporated under vacuum and the crude residue was purified by column (eluent: 100% EtOAc) to give 3.5 g of intermediate 28 (92% yield).

Preparation of intermediate 29:

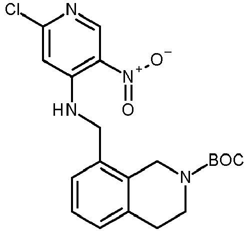

A mixture of intermediate 12 (579.00 mg, 3.00 mmol), intermediate 28 (866.00 mg, 3.30 mmol) and TEA (1.25 mL, 9 mmol) in THF (20 mL) was stirred at room temperature for 3 hours. Water was added and the product was extracted with EtOAc. Layers were separated and the organic layer was washed with water and brine, dried over Na₂SO₄, filtered and evaporated in vacuo. The crude residue was washed with petroleum ether and dried under vacuo to give 1 g of intermediate 29 (75 % yield, 95% purity based on LC/MS).

### Example A11

Preparation of intermediate 33:

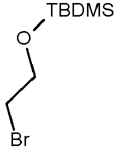

In a round bottom flask, TBDMSCl (10.61 g, 70.42 mmol) and imidazole (6.23 g, 91.55 mmol) were dissolved in DMF (12.5 mL) and the solution was stirred for 30 min at rt. Then, 2-bromoethanol (5 mL, 70.42 mmol) was added and the mixture was stirred at rt for 16 hours. The reaction mixture was partitioned between Et₂O and water. The organic layer was dried over MgSO₄, filtered and concentrated under vacuum to give 17 g of intermediate 33 (quant. yield, colorless oil).

Preparation of intermediate 34:

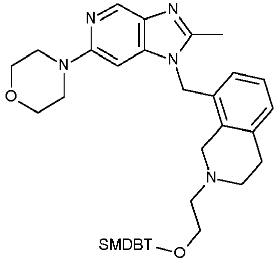

A mixture of compound 9a (200.00 mg, 0.49 mmol), intermediate 33 (351.66 mg, 1.47 mmol) and DIPEA (316.65 mg, 2.45 mmol) in ACN (10 mL) was stirred at rt for 14 h. The solvent was removed under reduced pressure to give 339 mg of intermediate 34 (quant. yield, 75% purity based on LC/MS) which was used in the next step without further purification.

### B. Preparation of the compounds

### Example B1

Preparation of compound 1:

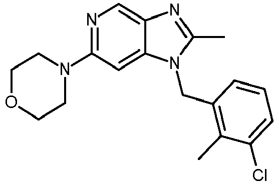

A mixture of intermediate 3 (432.00 mg, 1.30 mmol) and acetaldehyde (87.63 µL, 1.56 mmol) in 1-butanol (10 mL) was refluxed overnight. Acetaldehyde (175.26 µL, 3.12 mmol) was added and the mixture was further refluxed for 6 hours, then stirred at rt for 13 h. The reaction mixture was cooled to room temperature, quenched with water and extracted with EtOAc. The organic layer was decanted, dried over MgSO₄, filtered and evaporated to dryness. The residue was purified by chromatography over silica gel (irregular SiOH, 30 g, mobile phase gradient: from 0.3% NH₄OH, 97% DCM, 3% MeOH to 0.6% NH₄OH, 6% DCM, 94% MeOH). The product containing fractions were collected and evaporated to dryness. The residue was then crystallized from Et₂O, filtrated, and the solid was purified by reverse phase chromatography (X-Bridge-C18, 5 µm, 30^{∗}150 mm, mobile phase gradient: from 75% aq. NH₄HCO₃ (0.5%), 25% ACN to 0% aq. NH₄HCO₃ (0.5%), 100% ACN). The pure fractions were collected and evaporated to dryness to give 50 mg of compound 1 (11% yield).

Preparation of compound 2:

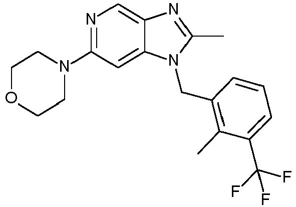

A mixture of intermediate 6 (451.00 mg, 1.23 mmol) and acetaldehyde (83.11 µL, 1.48 mmol) in 1-butanol (10 mL) was refluxed overnight. The solution was cooled to rt and acetaldehyde (166.22 µL, 2.95 mmol) was added and the mixture was further refluxed for 6 hours, then stirred at rt for 13 h. The reaction mixture was cooled to room temperature, quenched with water and extracted with EtOAc. The organic layer was decanted, dried over MgSO₄, filtered and evaporated to dryness. The residue was purified by chromatography over silica gel (irregular SiOH, 30 g, mobile phase gradient: from 0.3% NH₄OH, 97% DCM, 3% MeOH to 0.6% NH₄OH, 6% DCM, 94% MeOH). The pure fractions were collected and evaporated to give 190 mg of compound 2 (39% yield).

The compound in the Table below was prepared by using an analogous method as described for the preparation of compound 2, starting from the respective starting materials.

| Compound number | Structure |
|---|---|
| Compound 13 (from intermediate 36) | 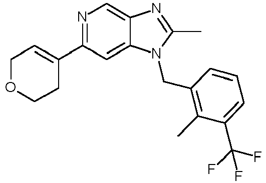 |

### Example B2

Preparation of compound 3:

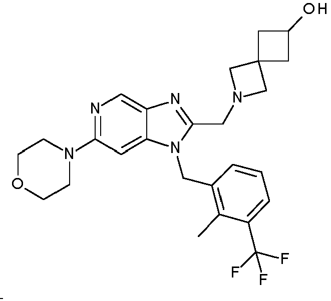

A mixture of intermediate 10 (234.00 mg, 0.39 mmol) and LiOH (64.85 mg, 1.55 mmol) in MeOH (5.6 mL) was stirred at room temperature overnight. The reaction mixture was diluted with DCM and water was added. The mixture was extracted with DCM (six times). The combined organic layers were dried over MgSO₄, filtered and the solvent was evaporated to dryness. The crude residue was purified by chromatography over silica gel (Stationary phase: Spherical bare silica 5 µm, 150x30.0 mm, mobile phase gradient: from 0.3% NH₄OH, 97% DCM, 3% MeOH to 1.5% NH₄OH, 85% DCM, 15% MeOH). The pure fractions were collected and the solvent was evaporated to dryness to give colorless oil which was taken up with few DCM and pentane. The solvent was evaporated under vacuo to give 92 mg of compound 3 (47% yield, pale yellow foam).

### Example B3

Preparation of compound 4:

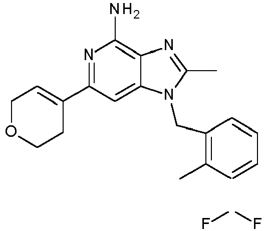

HCl (1M in H₂O) (2.12 mL, 2.12 mmol) was added to a solution of intermediate 17 (120.00 mg, 0.21 mmol) in THF (5 mL). The reaction mixture was stirred at room temperature for 1 h. An aqueous solution of K₂CO₃ (10 %) was added into the mixture until basic pH. EtOAc was added and the organic layer was extracted, washed with water, filtered and concentrated under reduced pressure. The crude residue was crystallized from CH₃CN and the precipitate was filtered, washed with Et₂O and dried under vacuo to give 25 mg of compound 4 (35% yield).

The compounds in the Table below were prepared by using an analogous method as described for the preparation of compound 4, starting from the respective starting materials.

| Compound number | Structure |
|---|---|
| Compound 12 | 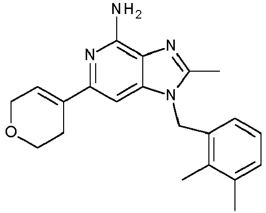 |
| Compound 14 | 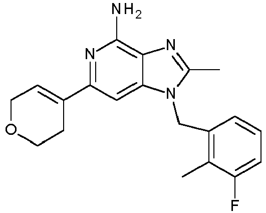 |
| Compound 15 (from intermediate 37) | 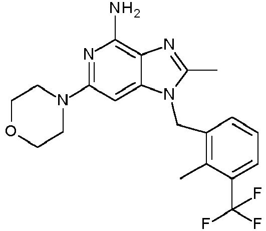 |
| Compound 16 | 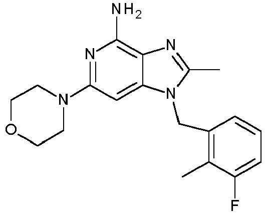 |
| Compound 17 | 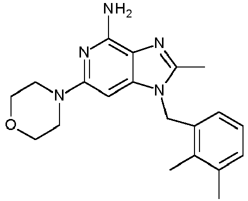 |

### Example B4

Preparation of compound 5:

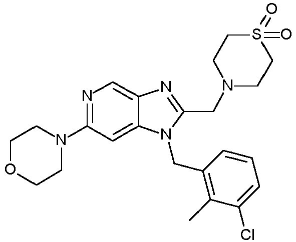

A mixture of intermediate 18 (176.52 mg, 0.48 mmol) and thiomorpholine 1,1-dioxide (96.52 mg, 0.71 mmol) in DCM (6 mL) was stirred overnight at room temperature. Sodium triacetoxyborohydride (201.77 mg, 0.95 mmol) was added and the reaction mixture was stirred at room temperature for 18 hours. The reaction mixture was diluted with DCM and poured onto a 10% aqueous solution of K₂CO₃. The organic layer was decanted, dried over MgSO₄, filtered and evaporated to dryness. The residue was purified by chromatography over silica gel (irregular bare silica 150 g, mobile phase gradient: from 98% DCM, 2% MeOH (+10% NH₄OH) to 87% DCM, 13% MeOH (+10% NH₄OH)). The pure fractions were collected and evaporated to dryness. The residue was further purified by reverse phase chromatography (X-Bridge-C18 5 µm 30^{∗}150 mm, mobile phase gradient: from 75% aq. NH₄HCO₃ (0.5%), 25% ACN to 35% aq. NH₄HCO₃ (0.5%), 65% ACN). The pure fractions were collected and evaporated to dryness to give 49 mg of compound 5 (21% yield).

The compound in the Table below was prepared by using an analogous method as described for the preparation of compound 5, starting from the respective starting materials.

| Compound number | Structure |
|---|---|
| Compound 8 | 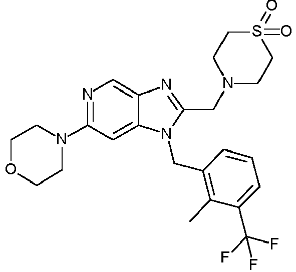 |

### Example B5

Preparation of compound 6:

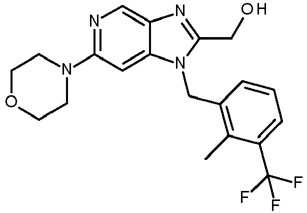

NaBH₄ (28.07 mg, 0.74 mmol) was added portionwise at 5 °C to a solution of intermediate 7 (250.00 mg, 0.62 mmol) in MeOH (5 mL). The reaction mixture was stirred at room temperature for 3 hours, quenched with water and extracted with DCM. The organic layer was decanted, filtered through Chromabond® and evaporated to dryness. The residue was crystallized from CH₃CN and the precipitate was filtered, washed with Et₂O and dried under vacuo to give 229 mg of compound 6 (91% yield).

### Example B6

Preparation of compound 7:

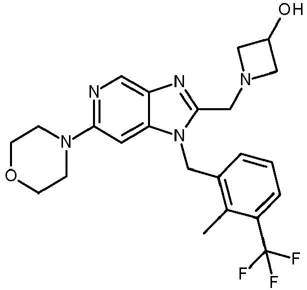

TBAF (1M in THF) (955.00 µL, 0.96 mmol) was added at 5 °C to a solution of intermediate 20 (275.00 mg, 0.48 mmol) in 1,4-dioxane (5 mL). The reaction mixture was stirred at room temperature for 4 hours, diluted with DCM and poured onto a 10% aqueous solution of K₂CO₃. The organic layer was decanted, dried over MgSO₄, filtered and concentrated until precipitation. The precipitate was filtered, washed with CH₃CN and Et₂O and dried under vacuo to give 130 mg of compound 7 (59% yield).

Preparation of compound 11:

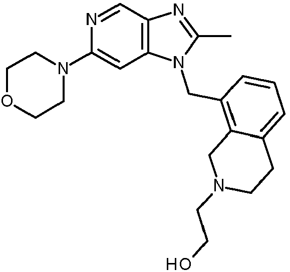

A mixture of intermediate 34 (339.00 mg, 0.49 mmol based on 75% purity determined by LC/MS) and TBAF (1M in THF) (2.45 mL, 2.45 mmol) in THF (10 mL) was stirred at rt overnight. The reaction was poured into water (50 mL) and diluted with EtOAc (100 mL). The aqueous layer was extracted with EtOAc (twice) and the combined organic layers were dried over MgSO₄, filtered and concentrated to dryness. The crude product was purified by high performance liquid chromatography (Column: Phenomenex Gemini 150^{∗}25 mm^{∗}10 µm, flow rate: 25 mL/min, mobile phase gradient: from 85% water (containing 0.05% ammonia), 15% CH₃CN to 55% water (containing 0.05% ammonia), 45% CH₃CN, from 0 to 10 min). Product containing fractions were concentrated in vacuum to give 53 mg of compound 11 (30% yield).

### Example B7

Preparation of compound 9a and compound 9b 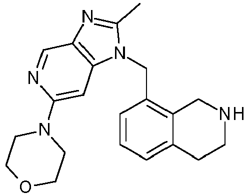 (1.HCl):

A mixture of intermediate 32 (800.00 mg, 1.73 mmol) in TFA/DCM solution (10 mL, 1:3, v/v) was stirred at room temperature for 5 hours. Solvent was removed and the residue was poured into water and pH was adjusted (pH > 7). The product was extracted with DCM and the organic layer was collected, dried over Na₂SO₄, filtered and evaporated in vacuo to give compound 9a (450 mg; 64% based on a LC/MS purity of 89%). 150 mg of compound 9a were purified by HPLC (Column: Gemini 150^{∗}25 mm, 5 µm, flow rate: 25 mL/min, mobile phase gradient: from 100% water (containing 0.1% HCl), 0% CH₃CN to 75% water, 25% CH₃CN, from 0 to 16 min). Product containing fractions were collected and the solvent was concentrated in vacuum to give 68 mg of compound 9b (1.HCl) (46% yield).

### Example B8

Preparation of compound 10: 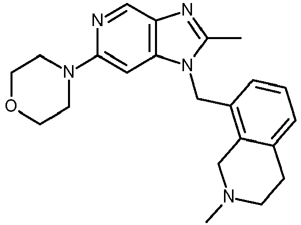 1.HCl

A mixture of compound 9a (163.35 mg, 0.40 mmol) and paraformaldehyde (1.80 g, 20.00 mmol) in MeOH (20 mL) was stirred at room temperature for 1 hour. NaBH(OAc)₃ (4.24 g, 20 mmol)was added and the resulting mixture was stirred for another 24 h. The mixture was filtered and the filtrate was evaporated in vacuo. The crude product was dissolved in ethyl acetate and the mixture was washed with saturated NaHCO₃ solution and brine. The organic layer was dried over Na₂SO₄, filtered and concentrated under reduced pressure. The crude residue was purified by high performance liquid chromatography (Column: Gemini 150^{∗}25 mm, 5 µm, flow rate: 25 mL/min, mobile phase gradient: from 100% water (containing 0.1% HCl), 0% CH₃CN to 75% water, 25% CH₃CN, from 0 to 16 min). Product containing fractions were collected and the solvent was concentrated under vacuum to give 96 mg of compound 10 (1.HCl) (57% yield).

### C. Analytical Part

### LCMS (liquid chromatography/Mass spectrometry)

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rₜ) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl ...), the reported value is the one obtained for the lowest isotope mass. All results were obtained with experimental uncertainties that are commonly associated with the method used.

Hereinafter, "SQD" means Single Quadrupole Detector, "RT" room temperature, "BEH" bridged ethylsiloxane/silica hybrid, "HSS" High Strength Silica, "DAD" Diode Array Detector, "MSD" Mass Selective Detector.

**Table: LCMS Method codes (Flow expressed in mL/min; column temperature (T) in °C; Run time in minutes).**

| Method code | Instrument | Column | Mobile phase | gradient | Flow (mL/min) | Run time |
|---|---|---|---|---|---|---|
| | | | | | T (°C) | |
| 1 | Waters: Acquity UPLC®-DAD and Quattro Micro™ | Waters: BEHC18 (1.7 µm, 2.1 x 100 mm) | A: 95% CH₃COONH₄ 7mM / 5% CH₃CN, B: CH₃CN | From 84.2% A for 0.49 min, to 10.5% A in 2.18 min, held for 1.94 min, back to 84.2% A in 0.73 min, held for 0.73 min. | 0.343 | 6.2 |
| | | | | | 40 | |
| 2 | Agilent 1200, MSD 6110 | Agilent TC-C18 (5 µm, 2.1 x 50 mm) | A: water (+ 0.1% TFA), B: CH₃CN (+ 0.1% TFA) | From 100% A for 1 min, to 40% A in 4 min, to 15% A in 2.5 min, back to 100% A in 2 min, held for 0.5 min. | 0.8 | 10 |
| | | | | | 50 | |
| 3 | Agilent 1200, MSD 6110 | XBridge ShieldRP18 (5 µm, 2.1 x 50 mm) | A: water (+ 0.05% NH₃.H₂O), B: CH₃CN | From 100% A for 1 min, to 40% A in 4 min, to 5% A in 2.5 min, back to 100% A in 2 min, held for 0.5 min. | 0.8 | 10 |
| | | | | | 40 | |

### Melting point (DSC or K)

For a number of compounds, melting points (MP) were determined with a DSC1 (Mettler-Toledo). Melting points were measured with a temperature gradient of 10 °C/minute. Maximum temperature was 350 °C. Values are peak values.

For a number of compounds, melting points were obtained with a Kofler (K) hot bench, consisting of a heated plate with linear temperature gradient, a sliding pointer and a temperature scale in degrees Celsius.

**Table: N° means compound number, MP means melting point (°C), Rt means retention time (min)**

| N° | Compound | MP (°C) | Kofler (K) or DSC | Rt | [M+H]⁺ | LC/MS Method |
|---|---|---|---|---|---|---|
| 1 | 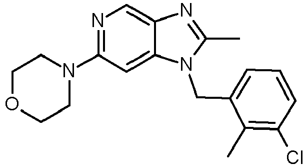 | 177 | K | 2.62 | 357 | 1 |
| 2 | 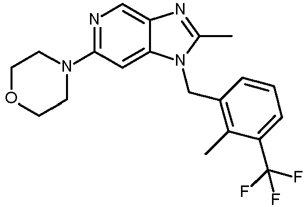 | 136 | K | 2.70 | 391 | 1 |
| 3 | 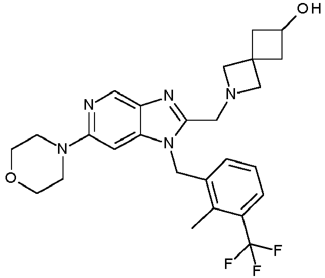 | 78 | K | 2.51 | 502 | 1 |
| 4 | 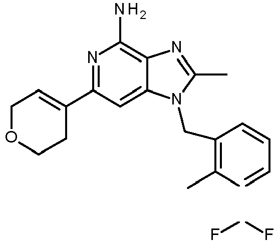 | 225 | DSC | 2.73 | 403 | 1 |
| 5 | 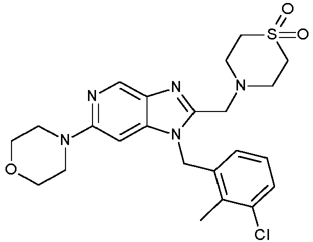 | 238 | K | 2.47 | 490 | 1 |
| 6 | 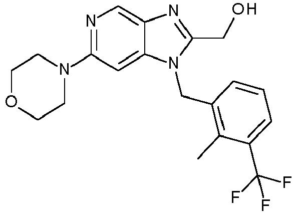 | 219 | K | 2.49 | 407 | 1 |
| 7 | 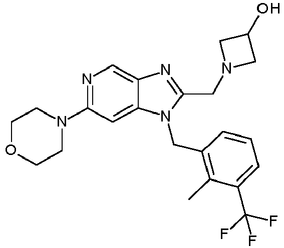 | 255 | K | 2.44 | 462 | 1 |
| 8 | 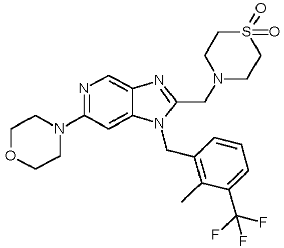 | 260 | K | 2.54 | 524 | 1 |
| 9a | 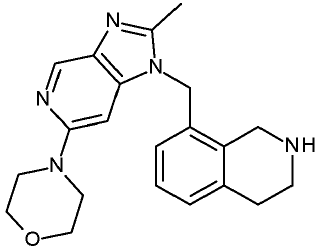 | - | - | - | - | - |
| 9b | 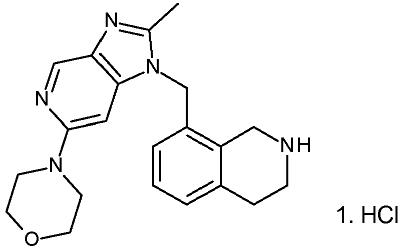 | - | - | 2.69 | 364 | 2 |
| 10 | 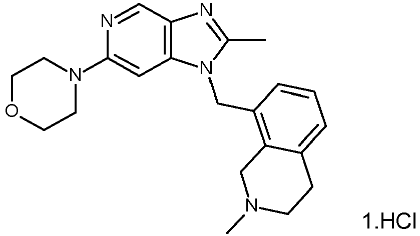 | - | - | 2.68 | 378 | 2 |
| 11 | 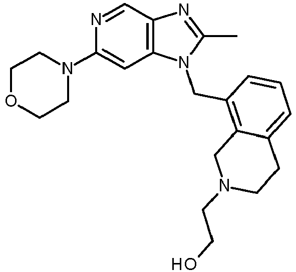 | - | - | 3.58 | 408 | 3 |
| 12 | 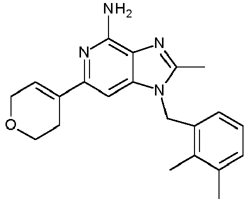 | 219 | DSC | 2.59 | 349 | 1 |
| 13 | 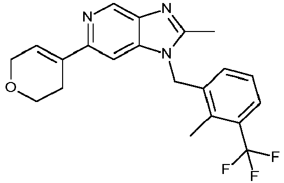 | 172 | DSC | 2.73 | 388 | 1 |
| 14 | 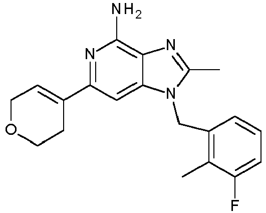 | 248 | K | 2.49 | 353 | 1 |
| 15 | 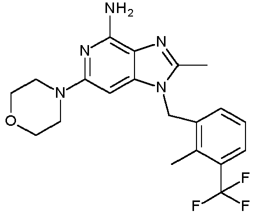 | 234 | DSC | 2.66 | 406 | 1 |
| 16 | 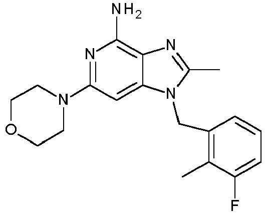 | 253 | DSC | 2.42 | 356 | 1 |
| 17 | 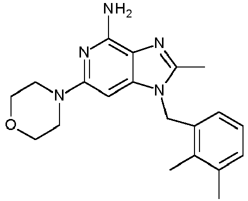 | 222 | DSC | 2.46 | 352 | 1 |

### NMR

The NMR experiments were carried out using a Bruker Avance 500 III using internal deuterium lock and equipped with reverse triple-resonance (¹H, ¹³C, ¹⁵N TXI) probe head. Chemical shifts (δ) are reported in parts per million (ppm). J values are expressed in Hz.
Compound 1: ¹H NMR (500 MHz, DMSO-*d*₆): δ ppm 8.47 (s, 1 H) 7.36 (d, *J*=7.9 Hz, 1 H) 7.08 (t, *J*=7.9 Hz, 1 H) 6.80 (s, 1 H) 6.08 (d, *J*=7.9 Hz, 1 H) 5.45 (s, 2 H) 3.65 - 3.72 (m, 4 H) 3.33 - 3.36 (m, 4 H, partially obscured by solvent peak) 2.45 (s, 3 H) 2.35 (s, 3 H).
Compound 3:
   ¹H NMR (500 MHz, DMSO-*d*₆) δ ppm 8.53 (s, 1 H) 7.58 (d, *J*=7.9 Hz, 1 H) 7.24 (t, *J*=7.7 Hz, 1 H) 6.77 (s, 1 H) 6.40 (d, *J*=7.9 Hz, 1 H) 5.53 (s, 2 H) 4.87 (d, *J*=6.3 Hz, 1 H) 3.77 - 3.85 (m, 1 H) 3.66 - 3.70 (m, 4 H) 3.65 (s, 2 H) 3.34 - 3.36 (m, 4 H, partially obscured by solvent peak) 2.96 - 3.06 (m, 4 H) 2.48 - 2.49 (m, 3 H, partially obscured by solvent peak) 2.03 - 2.13 (m, 2 H) 1.61 - 1.73 (m, 2 H)
Compound 4: ¹H NMR (500 MHz, DMSO-*d*₆): δ ppm 7.61 (d, *J*=7.6 Hz, 1 H) 7.27 (t, *J*=7.9 Hz, 1 H) 6.78 (s, 1 H) 6.56 - 6.60 (m, 1 H) 6.39 (d, *J*=7.9 Hz, 1 H) 6.06 (s, 2 H) 5.51 (s, 2 H) 4.18 - 4.22 (m, 2 H) 3.75 (t, *J*=5.4 Hz, 2 H) 2.51 (s, 3 H, partially obscured by solvent peak) 2.38 - 2.43 (m, 2 H) 2.36 (s, 3 H).

### Pharmacology

### Enzyme Binding Assays (KINOMEscan®)

Kinase enzyme binding affinities of compounds disclosed herein were determined using the KINOMEscan technology performed by DiscoveRx Corporation, San Diego, California, USA (www.kinomescan.com). Table A reports the obtained Kd values (nM), with the Kd being the inhibitor binding constant ('n.d.' means not determined):

| Co. No. | Kd PIK3Cα_h (µM) | Kd PIK3Cβ_h (µM) | Kd PIK3Cδ_h (µM) | Kd PIK3Cγ_h (µM) | Kd MTOR_h (µM) |
|---|---|---|---|---|---|
| 2 | 3.7 | 0.002 | 1.5 | 22.4 | >30.2 |
| 1 | 2.1 | 0.002 | 0.6 | 9.5 | 30.2 |
| 5 | 2.6 | 0.004 | 1.5 | >30.2 | >30.2 |
| 6 | 2.8 | 0.001 | 0.8 | 18.2 | 30.2 |
| 7 | 2.2 | 0.003 | 1.1 | >30.2 | 4.6 |
| 8 | 11.0 | 0.011 | 2.6 | >30.2 | >30.2 |
| 3 | 1.6 | 0.001 | 0.2 | >30.2 | >30.2 |
| 9 | >30.2 | 0.079 | 5.1 | >30.2 | >30.2 |
| 10 | 26.9 | 0.100 | 4.2 | >30.2 | >30.2 |
| 11 | 8.7 | 0.027 | 1.3 | 32.4 | >30.2 |
| 4 | 17.0 | 0.009 | 4.3 | >30.2 | >30.2 |
| 12 | 13.2 | 0.003 | 0.7 | >30.2 | >30.2 |
| 13 | 5.9 | 0.003 | 1.3 | 17.0 | >30.2 |
| 14 | >30.2 | 0.011 | 3.3 | 21.9 | >30.2 |
| 16 | >30.2 | 0.132 | 5.6 | >30.2 | >30.2 |
| 15 | 18.2 | 0.398 | 6.3 | >30.2 | >30.2 |
| 17 | 8.3 | 0.019 | 1.5 | >30.2 | >30.2 |

### Cellular assays:

Cellular activity of PI3Kβ inhibitors was determined by quantifying the phosphorylation of Akt in PC-3 cells. Akt phosphorylated at Ser473 and Thr308 were measured using an enzyme-linked immunosorbent assay (ELISA; Meso Scale Discovery (MSD), Gaithersburg, MD) and specific primary antibodies from MSD.

On day 1, PC3 cells (ATCC # CRL-14351) were seeded into PerkinElmer MW96 plates at 25.000 cells per well, in 75 µl complete culture medium (DMEM high glucose, AQmedia™, D0819, Sigma-Aldrich) containing 10% heat inactivated FCS and incubated at 37 °C, 5% CO2 during 24 hours. On day 2, compound or DMSO (0.3%) was added and cells were further incubated for 60 min at 37 °C, 5% CO2 in a total volume of 100 µl of medium.

The phosphoprotein assay was executed according to vendor instructions in the Phospho-Akt (Ser473) Assay Whole Cell Lysate Kit (MSD # K15100D-3) and the Phospho-Akt (Thr308) Assay Whole Cell Lysate Kit (MSD # K151DYD-3) using the lysis, blocking and wash buffer provided.

Briefly, at the end of the cell treatment period, media were removed by aspiration and adherent cells were lysed in 50 µl ice-cold lysis buffer. MSD plates are supplied precoated with capture antibodies for Phospho-Akt (Ser473 and Thr308). After blocking, lysates from tissue culture plates were added and plates were washed. Then, a solution containing the detection antibody (anti-total Akt conjugated with an electrochemiluminescent compound-MSD Sulfo-tag label) was added. The signals were detected using an MSD SECTOR Imager 6000 and are proportional to the phospho-Akt titres.

Data were processed. The percentage of inhibition was plotted against the log concentration of test compounds, and the sigmoidal log concentration-effect curve of best fit was calculated by nonlinear regression analysis. From these concentration-response curves, the IC₅₀ values were calculated. Five concentrations were used for curve fitting.

**Table B reports the obtained IC₅₀ values (nM) ('n.d.' means not determined):**

| Co. No. | IC₅₀ pAkt_S473 (µM) | IC₅₀ pAkt_Thr308 (µM) |
|---|---|---|
| 1 | 0.04 | 0.03 |
| 2 | 0.18 | 0.43 |
| 3 | 0.07 | 0.04 |
| 4 | ∼0.14 | 0.15 |
| 5 | ∼0.1 | 0.13 |
| 6 | 0.05 | 0.04 |
| 7 | 0.48 | 0.39 |
| 8 | n.d. | 0.29 |
| 9 | ∼0.48 | ∼0.46 |
| 10 | >0.51 | >0.51 |
| 11 | >0.51 | >0.51 |
| 12 | 0.31 | 0.11 |
| 13 | 0.17 | 0.08 |
| 14 | 0.32 | 0.22 |
| 15 | 0.12 | 0.09 |
| 16 | 0.41 | 0.18 |
| 17 | ∼0.23 | 0.19 |

### Prophetic Composition examples

"Active ingredient" (a.i.) as used throughout these examples relates to a compound of Formula (I), including any tautomer or stereoisomeric form thereof, or a N-oxide, a pharmaceutically acceptable addition salt or a solvate thereof; in particular to any one of the exemplified compounds.

### Typical examples of recipes for the formulation of the invention are as follows:

### 1. Tablets

| | |
|---|---|
| Active ingredient | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

### 2. Suspension

An aqueous suspension is prepared for oral administration so that each milliliter contains 1 to 5 mg of active ingredient, 50 mg of sodium carboxymethyl cellulose, 1 mg of sodium benzoate, 500 mg of sorbitol and water ad 1 ml.

### 3. Injectable

A parenteral composition is prepared by stirring 1.5 % (weight/volume) of active ingredient in 0.9 % NaCl solution or in 10 % by volume propylene glycol in water.

### 4. Ointment

| | |
|---|---|
| Active ingredient | 5 to 1000 mg |
| Stearyl alcohol | 3 g |
| Lanoline | 5 g |
| White petroleum | 15 g |
| Water | ad 100 g |

In this Example, active ingredient can be replaced with the same amount of any of the compounds according to the present invention, in particular by the same amount of any of the exemplified compounds.

## Claims

1. A compound of Formula (I) 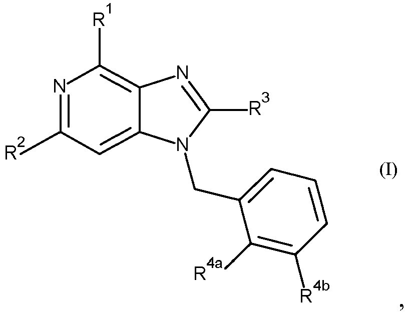 a tautomer or a stereoisomeric form thereof, wherein
R¹ represents hydrogen, -C(=O)OH, -C(=O)NH₂, -NH₂, 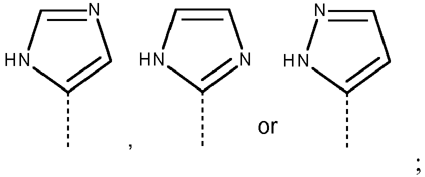
R² represents 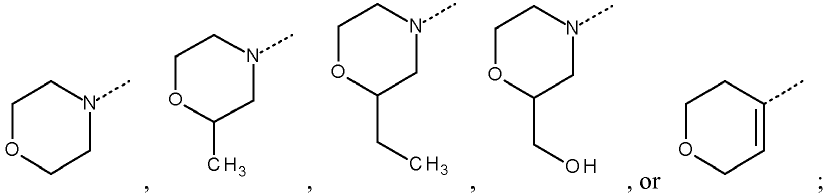
R³ represents C₁₋₄alkyl; -CH(OH)-CH₂-R^{q}; C₁₋₄alkyl substituted on the same carbon atom with one -OH and with one Het¹; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O-(C=O)-CH(NH₂)-C₁₋₄alkyl, -O-(C=O)-CH(NH₂)-C₁₋₄alkyl-Ar, 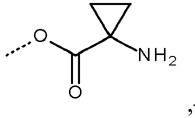 -NH-C₁₋₄alkyl-OH, Het¹, -O-C(=O)-C₁₋₄alkyl-Het¹, -C(=O)-Het¹, and -NH-C(=O)-Het¹;
R^{q} represents Het¹, fluoro, -OH, -NH₂, -O-(C=O)-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -O-(C=O)-CH(NH₂)-C₁₋₄alkyl, -O-(C=O)-CH(NH₂)-C₁₋₄alkyl-Ar, 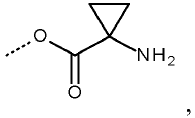 or -NH-C₁₋₄alkyl-OH;
Ar represents phenyl optionally substituted with one hydroxy;
R^{4a} represents hydrogen, C₁₋₄alkyl, Het^{a}, or C₁₋₄alkyl substituted with one or more substituents each independently selected from the group consisting of -OH, -NR⁵R⁶ and Het^{a};
R^{4b} represents hydrogen, halo, C₁₋₄alkyl, or C₁₋₄alkyl substituted with one or more halo substituents;
or R^{4a} and R^{4b} are taken together to form together with the phenyl ring to which they are attached a structure of Formula (a-1), (a-2), (a-3), (a-4) or (a-5): 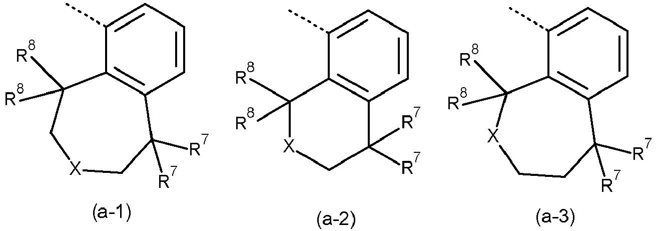 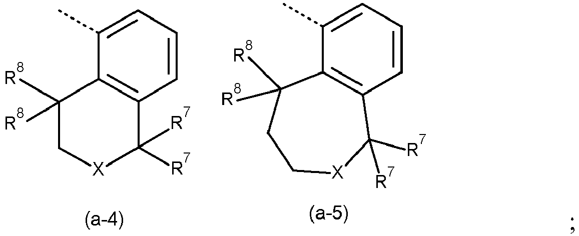
X represents -NH-, -O-, -N(C₁₋₃alkyl)-, or -N(hydroxyC₁₋₃alkyl)-;
both R⁷ substituents are the same and are selected from the group consisting of hydrogen, fluoro and methyl; or both R⁷ substituents are taken together to form together with the common carbon atom to which they are attached a cyclopropyl, cyclobutyl or oxetanyl;
both R⁸ substituents are the same and are selected from the group consisting of hydrogen and methyl; or both R⁸ substituents are taken together to form together with the common carbon atom to which they are attached a cyclopropyl, cyclobutyl or oxetanyl;
R⁵ represents hydrogen, C₁₋₆alkyl, or C₁₋₆alkyl substituted with one -OH;
R⁶ represents hydrogen, C₁₋₆alkyl, or C₁₋₆alkyl substituted with one -OH;
Het¹ represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of halo, -NH₂, C₁₋₄alkyl, -S(=O)₂-C₁₋₆alkyl, -C₁₋₄alkyl-S(=O)₂-C₁₋₆alkyl, hydroxyl, C₁₋₄alkyloxy, fluoro, cyano and C₁₋₄alkyl substituted with one hydroxy; or two substituents on the same carbon atom of said 4-, 5- or 6-membered saturated heterocyclyl are taken together to form together with the common carbon atom to which they are attached Ring A;
Ring A represents cyclobutyl, cyclopentyl, cyclohexyl or a 4-, 5- or 6-membered saturated heterocyclyl containing at least one heteroatom each independently selected from O, S, S(=O)ₚ and N; said cyclobutyl, cyclopentyl, cyclohexyl or 4-, 5- or 6-membered saturated heterocyclyl is optionally substituted with one or two C₁₋₄alkyl substituents, with one C₁₋₄alkyl and one hydroxy substituent, or with one hydroxy substituent;
each Het^{a} independently represents a 4-, 5- or 6-membered saturated heterocyclyl containing at least one heteroatom each independently selected from O, S, S(=O)ₚ and N; said 4-, 5- or 6-membered saturated heterocyclyl is optionally substituted with one or two substituents each independently selected from the group consisting of C₁₋₄alkyl, -S(=O)₂-C₁₋₆alkyl, hydroxy, -C₁₋₄alkyl-S(=O)₂-C₁₋₆alkyl, and C₁₋₄alkyl substituted with one hydroxy; or two substituents on the same carbon atom of said 4-, 5- or 6-membered saturated heterocyclyl are taken together to form together with the common carbon atom to which they are attached Ring B;
Ring B represents cyclobutyl, cyclopentyl, cyclohexyl or a 4-, 5- or 6-membered saturated heterocyclyl containing at least one heteroatom each independently selected from O, S, S(=O)ₚ and N; said cyclobutyl, cyclopentyl, cyclohexyl or 4-, 5- or 6-membered saturated heterocyclyl is optionally substituted with one or two C₁₋₄alkyl substituents, with one C₁₋₄alkyl and one hydroxy substituent, or with one hydroxy substituent;
p represents 1 or 2;
or a N-oxide, a pharmaceutically acceptable addition salt or a solvate thereof.

2. The compound according to claim 1, wherein
R³ represents C₁₋₄alkyl; C₁₋₄alkyl substituted on the same carbon atom with one -OH and with one Het¹; or C₁₋₄alkyl substituted with one substituent selected from the group consisting of fluoro, -OH, -NH₂, -O-(C=O)-C₁₋₄alkyl, -(C=O)-O-C₁₋₄alkyl, -NH-(C=O)-C₁₋₄alkyl, -NH-(SO₂)-C₁₋₄alkyl, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O (C=O)-CH(NH₂)-C₁₋₄alkyl, -O-(C=O)-CH(NH₂)-C₁₋₄alkyl-Ar, 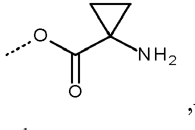 -NH-C₁₋₄alkyl-OH, Het¹, -O-C(=O)-C₁₋₄alkyl-Het¹, -C(=O)-Het¹, and -NH-C(=O)-Het¹;
R^{4a} represents hydrogen, C₁₋₄alkyl, or C₁₋₄alkyl substituted with one or more substituents each independently selected from the group consisting of -OH, and -NR⁵R⁶;
R^{4b} represents hydrogen, halo, C₁₋₄alkyl, or C₁₋₄alkyl substituted with one or more halo substituents;
or R^{4a} and R^{4b} are taken together to form together with the phenyl ring to which they are attached a structure of Formula (a-1), (a-2), (a-3), (a-4) or (a-5);
both R⁷ substituents are hydrogen;
both R⁸ substituents are hydrogen.

3. The compound according to claim 1, wherein
R¹ represents hydrogen or -NH₂;
R² represents 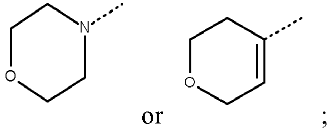
R³ represents C₁₋₄alkyl; or C₁₋₄alkyl substituted with one substituent selected from the group consisting of -OH and Het¹;
R^{4a} represents C₁₋₄alkyl;
R^{4b} represents halo, C₁₋₄alkyl, or C₁₋₄alkyl substituted with one or more halo substituents;
or R^{4a} and R^{4b} are taken together to form together with the phenyl ring to which they are attached a structure of Formula (a-2);
X represents -N(C₁₋₃alkyl)-, or -N(hydroxyC₁₋₃alkyl)-;
both R⁷ substituents are hydrogen;
both R⁸ substituents are hydrogen;
Het¹ represents a 4-, 5- or 6-membered saturated heterocyclyl containing at least one heteroatom each independently selected from S(=O)ₚ and N; said 4-, 5- or 6-membered saturated heterocyclyl is optionally substituted with one or two hydroxyl substituents; or two substituents on the same carbon atom of said 4-, 5- or 6-membered saturated heterocyclyl are taken together to form together with the common carbon atom to which they are attached Ring A;
Ring A represents cyclobutyl optionally substituted with one hydroxy substituent;
p represents 2.

4. The compound according to claim 1, wherein
X represents -N(C₁₋₃alkyl)-, or -N(hydroxyC₁₋₃alkyl)-.

5. The compound according to claim 1, wherein
R³ represents C₁₋₄alkyl; or C₁₋₄alkyl substituted with one substituent selected from the group consisting of -OH and Het¹.

6. The compound according to claim 1, wherein
R^{4a} represents hydrogen, C₁₋₄alkyl, Het^{a}, or C₁₋₄alkyl substituted with one or more substituents each independently selected from the group consisting of -OH, -NR⁵R⁶ and Het^{a};
R^{4b} represents hydrogen, halo, C₁₋₄alkyl, or C₁₋₄alkyl substituted with one or more halo substituents.

7. The compound according to claim 6, wherein
R^{4a} represents C₁₋₄alkyl;
R^{4b} represents C₁₋₄alkyl substituted with one or more halo substituents.

8. The compound according to claim 1, wherein
both R⁷ substituents are hydrogen; and wherein
both R⁸ substituents are hydrogen.

9. The compound according to claim 1, wherein
R² represents

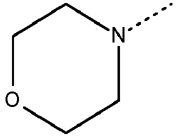

10. The compound according to claim 1, wherein R¹ represents hydrogen.

11. A pharmaceutical composition comprising a pharmaceutically acceptable carrier and, as active ingredient, a therapeutically effective amount of a compound according to any one of claims 1 to 10.

12. A compound as defined in any one of claims 1 to 10 for use as a medicament.

13. A compound as defined in any one of claims 1 to 10 for use in the treatment or prevention of a disease or condition selected from cancer, autoimmune disorders, cardiovascular diseases, inflammatory diseases, neurodegenerative diseases, allergy, pancreatitis, asthma, multiorgan failure, kidney diseases, platelet aggregation, sperm motility, transplantation rejection, graft rejection, and lung injuries.

14. The compound for use according to claim 13 wherein the disease or condition is cancer.

15. The compound for use according to claim 14 wherein the disease or condition is prostate cancer.

## Patentansprüche

1. Verbindung der Formel (I) 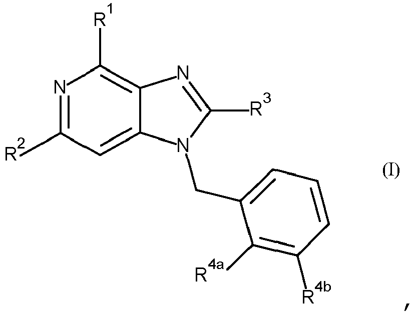 ein Tautomer oder eine stereoisomere Form davon, wobei
R¹ für Wasserstoff, -C(=O)OH, -C(=O)NH₂, -NH₂, 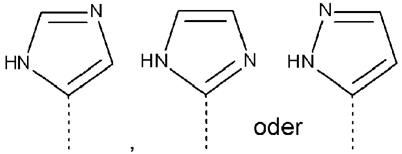 steht;
R² für 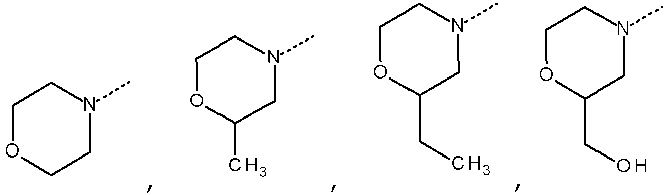 oder 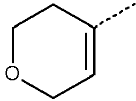 steht;
R³ für C₁₋₄-Alkyl; -CH(OH)-CH₂-R^{q}; C₁₋₄-Alkyl, das an demselben Kohlenstoffatom durch ein -OH und durch ein Het¹ substituiert ist; oder C₁₋₄-Alkyl, das durch einen Substituenten aus der Gruppe bestehend aus Fluor, -OH, -NH₂, -O-(C=O)-C₁-₄-Alkyl, -(C=O)-O-C₁₋₄-Alkyl, -NH-(C=O)-C₁-₄-Alkyl, -NH-(SO₂)-C₁-₄-Alkyl, -N(CH₃)-C₁₋₄-Alkyl-SO₂-CH₃, -NH-C₁₋₄-Alkyl-SO₂-CH₃, -N(CH₃)-C₁₋₄-Alkyl-OH, -(C=O)-NH-C₁₋₄-Alkyl-OH, -O-(C=O)-CH(NH₂)-C₁₋₄-Alkyl, -O-(C=O)-CH(NH₂)-C₁-₄-Alkyl-Ar, 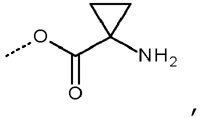 -NH-C₁-₄-Alkyl-OH, Het¹, -O-C(=O)-C₁₋₄-Alkyl-Het¹, -C(=O)-Het-¹ und -NH-C(=O)-Het¹ substituiert ist, steht;
R^{q} für Het¹, Fluor, -OH, -NH₂, -O-(C=O)-C₁₋₄-Alkyl, -NH-(C=O)-C₁₋₄-Alkyl, -NH-(SO₂)-C₁₋₄-Alkyl, -N(CH₃)-C₁₋₄-Alkyl-SO₂-CH₃, -NH-C₁₋₄-Alkyl-SO₂-CH₃, -N(CH₃)-C₁₋₄-Alkyl-OH, -O-(C=O)-CH(NH₂)-C₁₋₄Alkyl, -O-(C=O)-CH(NH₂)-C₁₋₄-Alkyl-Ar, 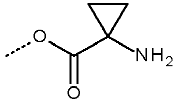 oder -NH-C₁₋₄-Alkyl-OH steht;
Ar für Phenyl, das gegebenenfalls durch ein Hydroxy substituiert ist, steht;
R^{4a} für Wasserstoff, C₁₋₄-Alkyl, Het^{a} oder C₁₋₄-Alkyl, das durch einen oder mehrere Substituenten, die jeweils unabhängig aus der Gruppe bestehend aus -OH, -NR⁵R⁶ und Het^{a} ausgewählt sind, substituiert ist, steht;
R^{4b} für Wasserstoff, Halogen, C₁₋₄-Alkyl oder C₁₋₄-Alkyl, das durch einen oder mehrere Halogensubstituenten substituiert ist, steht;
oder R^{4a} und R^{4b} zusammengenommen zusammen mit dem Phenylring, an den sie gebunden sind, eine Struktur der Formel (a-1), (a-2), (a-3), (a-4) oder (a-5) bilden: 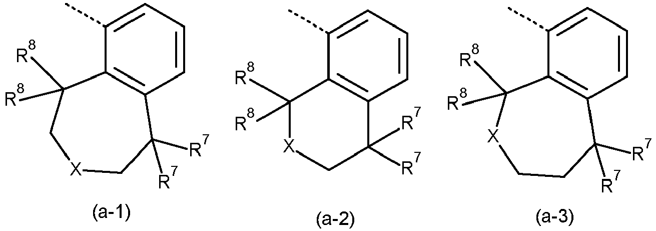 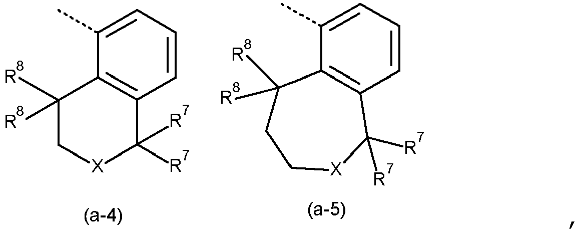
X für -NH-, -O-, -N(C₁₋₃-Alkyl)- oder -N(Hydroxy-C₁₋₃-alkyl) - steht;
beide R⁷-Substituenten gleich sind und aus der Gruppe bestehend aus Wasserstoff, Fluor und Methyl ausgewählt sind oder beide R⁷-Substituenten zusammengenommen zusammen mit dem gemeinsamen Kohlenstoffatom, an das sie gebunden sind, ein Cyclopropyl, Cyclobutyl oder Oxetanyl bilden; beide R⁸-Substituenten gleich sind und aus der Gruppe bestehend aus Wasserstoff und Methyl ausgewählt sind oder beide R⁸-Substituenten zusammengenommen zusammen mit dem gemeinsamen Kohlenstoffatom, an das sie gebunden sind, ein Cyclopropyl, Cyclobutyl oder Oxetanyl bilden;
R⁵ für Wasserstoff, C₁₋₆-Alkyl oder C₁₋₆-Alkyl, das durch ein -OH substituiert ist, steht;
R⁶ für Wasserstoff, C₁₋₆-Alkyl oder C₁₋₆-Alkyl, das durch ein OH substituiert ist, steht;
Het¹ für ein 4-, 5- oder 6-gliedriges gesättigtes Heterocyclyl mit mindestens einem Heteroatom, das jeweils unabhängig aus O, S, S(=O)ₚ und N ausgewählt ist, steht; wobei das 4-, 5- oder 6-gliedrige gesättigte Heterocyclyl gegebenenfalls durch einen oder zwei Substituenten, die jeweils unabhängig aus der Gruppe bestehend aus Halogen, -NH₂, C₁₋₄-Alkyl, -S(=O)₂-C₁₋₆-Alkyl, -C₁₋₄-AlkylS(=O)₂-C₁-₆-alkyl, Hydroxyl, C₁₋₄-Alkyloxy, Fluor, Cyano und C₁₋₄-Alkyl, das durch ein Hydroxy substituiert ist, ausgewählt sind, substituiert ist oder zwei Substituenten an demselben Kohlenstoffatom des 4-, 5- oder 6-gliedrigen gesättigten Heterocyclyls zusammengenommen zusammen mit dem gemeinsamen Kohlenstoffatom, an das sie gebunden sind, Ring A bilden;
Ring A für Cyclobutyl, Cyclopentyl, Cyclohexyl oder ein 4-, 5- oder 6-gliedriges gesättigtes Heterocyclyl mit mindestens einem Heteroatom, das jeweils unabhängig aus O, S, S(=O)ₚ und N ausgewählt ist, steht; wobei das Cyclobutyl, Cyclopentyl, Cyclohexyl oder 4-, 5- oder 6-gliedrige gesättigte Heterocyclyl gegebenenfalls durch einen oder zwei C₁₋₄-Alkylsubstituenten, durch einen C₁₋₄-Alkyl- und einen Hydroxysubstituenten oder durch einen Hydroxysubstituenten substituiert ist;
Het^{a} jeweils unabhängig für ein 4-, 5- oder 6-gliedriges gesättigtes Heterocyclyl mit mindestens einem Heteroatom, das jeweils unabhängig aus O, S, S(=O)ₚ und N ausgewählt ist, steht; wobei das 4-, 5- oder 6-gliedrige gesättigte Heterocyclyl gegebenenfalls durch einen oder zwei Substituenten, die jeweils unabhängig aus der Gruppe bestehend aus C₁₋₄-Alkyl, -S(=O)₂-C₁₋₆-Alkyl, Hydroxy, -C₁₋₄-Alkyl-S(=O)₂-C₁-₆-alkyl und C₁₋₄-Alkyl, das durch ein Hydroxy substituiert ist, ausgewählt sind, substituiert ist oder zwei Substituenten an demselben Kohlenstoffatom des 4-, 5- oder 6-gliedrigen gesättigten Heterocyclyls zusammengenommen zusammen mit dem gemeinsamen Kohlenstoffatom, an das sie gebunden sind, Ring B bilden;
Ring B für Cyclobutyl, Cyclopentyl, Cyclohexyl oder ein 4-, 5- oder 6-gliedriges gesättigtes Heterocyclyl mit mindestens einem Heteroatom, das jeweils unabhängig aus O, S, S(=O)ₚ und N ausgewählt ist, steht; wobei das Cyclobutyl, Cyclopentyl, Cyclohexyl oder 4-, 5- oder 6-gliedrige gesättigte Heterocyclyl gegebenenfalls durch einen oder zwei C₁₋₄-Alkylsubstituenten, durch einen C₁₋₄-Alkyl- und einen Hydroxysubstituenten oder durch einen Hydroxysubstituenten substituiert ist;
p für 1 oder 2 steht;
oder ein N-Oxid, ein pharmazeutisch unbedenkliches Additionssalz oder ein Solvat davon.

2. Verbindung nach Anspruch 1, wobei
R³ für C₁₋₄-Alkyl; C₁₋₄-Alkyl, das an demselben Kohlenstoffatom durch ein -OH und durch ein Het¹ substituiert ist; oder C₁₋₄-Alkyl, das durch einen Substituenten aus der Gruppe bestehend aus Fluor, -OH, -NH₂, -O-(C=O)-C₁₋₄-Alkyl, - (C=O)-O-C₁-₄-Alkyl, -NH-(C=O)-C₁₋₄-Alkyl, -NH-(SO₂)-C₁₋₄-Alkyl, -N(CH₃)-C₁₋₄-Alkyl-SO₂-CH₃, -NH-C₁₋₄-Alkyl-SO₂-CH₃, -N(CH₃)-C₁₋₄-Alkyl-OH, - (C=O)-NH-C₁₋₄-Alkyl-OH, -O-(C=O)-CH(NH₂)-C₁₋₄-Alkyl, -O-(C=O)-CH(NH₂)-C₁₋₄-Alkyl-Ar, 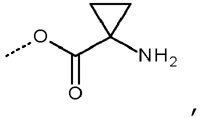 -NH-C₁₋₄-Alkyl-OH, Het¹, -O-C(=O)-C₁₋₄Alkyl-Het¹, -C(=O) -Het¹ und -NH-C (=O) -Het¹ substituiert ist, steht;
R^{4a} für Wasserstoff, C₁₋₄-Alkyl oder C₁₋₄-Alkyl, das durch einen oder mehrere Substituenten, die jeweils unabhängig aus der Gruppe bestehend aus -OH und -NR⁵R⁶ ausgewählt sind, substituiert ist, steht;
R^{4b} für Wasserstoff, Halogen, C₁₋₄-Alkyl oder C₁₋₄-Alkyl, das durch einen oder mehrere Halogensubstituenten substituiert ist, steht;
oder R^{4a} und R^{4b} zusammengenommen zusammen mit dem Phenylring, an den sie gebunden sind, eine Struktur der Formel (a-1), (a-2), (a-3), (a-4) oder (a-5) bilden;
beide R⁷-Substituenten für Wasserstoff stehen; beide R⁸-Substituenten für Wasserstoff stehen.

3. Verbindung nach Anspruch 1, wobei
R¹ für Wasserstoff oder -NH₂ steht;
R² für 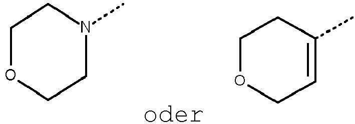 steht;
R³ für C₁₋₄-Alkyl oder C₁₋₄-Alkyl, das durch einen Substituenten aus der Gruppe bestehend aus -OH und
Het¹ substituiert ist, steht;
R^{4a} für C₁₋₄-Alkyl steht;
R^{4b} für Halogen, C₁₋₄-Alkyl oder C₁₋₄-Alkyl, das durch einen oder mehrere Halogensubstituenten substituiert ist, steht;
oder R^{4a} und R^{4b} zusammengenommen zusammen mit dem Phenylring, an den sie gebunden sind, eine Struktur der Formel (a-2) bilden;
X für -N(C₁₋₃-Alkyl) - oder -N(Hydroxy-C₁₋₃-alkyl)-steht;
beide R⁷-Substituenten für Wasserstoff stehen; beide R⁸-Substituenten für Wasserstoff stehen;
Het¹ für ein 4-, 5- oder 6-gliedriges gesättigtes Heterocyclyl mit mindestens einem Heteroatom, das jeweils unabhängig aus S(=O)ₚ und N ausgewählt ist, steht; wobei das 4-, 5- oder 6-gliedrige gesättigte Heterocyclyl gegebenenfalls durch einen oder zwei Hydroxylsubstituenten substituiert ist oder zwei Substituenten an demselben Kohlenstoffatom des 4-, 5- oder 6-gliedrigen gesättigten Heterocyclyls zusammengenommen zusammen mit dem gemeinsamen Kohlenstoffatom, an das sie gebunden sind, Ring A bilden;
Ring A für Cyclobutyl, das gegebenenfalls durch einen Hydroxysubstituenten substituiert ist, steht;
p für 2 steht.

4. Verbindung nach Anspruch 1, wobei
X für -N(C₁₋₃-Alkyl) - oder -N(Hydroxy-C₁₋₃-alkyl)-steht.

5. Verbindung nach Anspruch 1, wobei R³ für C₁₋₄-Alkyl oder C₁₋₄-Alkyl, das durch einen Substituenten aus der Gruppe bestehend aus -OH und Het¹ substituiert ist, steht.

6. Verbindung nach Anspruch 1, wobei
R^{4a} für Wasserstoff, C₁₋₄-Alkyl, Het^{a} oder C₁₋₄-Alkyl, das durch einen oder mehrere Substituenten, die unabhängig aus der Gruppe bestehend aus -OH, -NR⁵R⁶ und Het^{a} ausgewählt sind, substituiert ist, steht;
R^{4b} für Wasserstoff, Halogen, C₁₋₄-Alkyl oder C₁₋₄-Alkyl, das durch einen oder mehrere Halogensubstituenten substituiert ist, steht.

7. Verbindung nach Anspruch 6, wobei
R^{4a} für C₁₋₄-Alkyl steht;
R^{4b} für C₁₋₄-Alkyl, das durch einen oder mehrere Halogensubstituenten substituiert ist, steht.

8. Verbindung nach Anspruch 1, wobei
beide R⁷-Substituenten für Wasserstoff stehen; und wobei
beide R⁸-Substituenten für Wasserstoff stehen.

9. Verbindung nach Anspruch 1, wobei
R² für 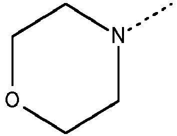 steht.

10. Verbindung nach Anspruch 1, wobei R¹ für Wasserstoff steht.

11. Pharmazeutische Zusammensetzung, umfassend einen pharmazeutisch unbedenklichen Träger und als Wirkstoff eine therapeutisch wirksame Menge einer Verbindung nach einem der Ansprüche 1 bis 10.

12. Verbindung gemäß einem der Ansprüche 1 bis 10 zur Verwendung als Medikament.

13. Verbindung gemäß einem der Ansprüche 1 bis 10 zur Verwendung bei der Behandlung oder Prävention einer Erkrankung oder eines Leidens, die bzw. das aus Krebs, Autoimmunstörungen, Herz-Kreislauf-Erkrankungen, entzündlichen Erkrankungen, neurodegenerativen Erkrankungen, Allergie, Pankreatitis, Asthma, multiplem Organversagen, Nierenerkrankungen, Plättchenaggregation, Spermienmotilität, Transplantatabstoßung und Lungenverletzungen ausgewählt ist.

14. Verbindung zur Verwendung nach Anspruch 13, wobei es sich bei der Erkrankung bzw. dem Leiden um Krebs handelt.

15. Verbindung zur Verwendung nach Anspruch 14, wobei es sich bei der Erkrankung bzw. dem Leiden um Prostatakrebs handelt.

## Revendications

1. Composé de formule (I) 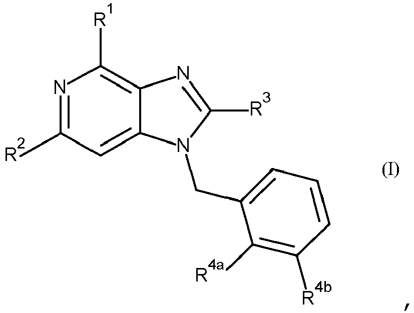 forme tautomère ou forme stéréoisomérique correspondante,
R¹ représentant hydrogène, -C(=O)OH, -C(=O)NH₂,-NH₂, 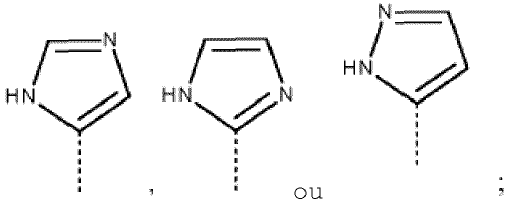
R² représentant 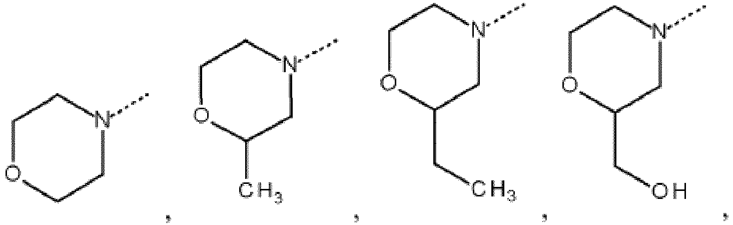 ou 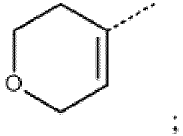
R³ représentant C₁₋₄alkyle ; -CH(OH)-CH₂-R^{q}; C₁₋₄alkyle substitué sur le même atome de carbone par un -OH et par un Het¹ ; ou C₁₋₄alkyle substitué par un substituant choisi dans le groupe constitué par fluoro, -OH, -NH₂, -O-(C=O)-C₁₋₄alkyle, - (C=O)-O-C₁₋₄alkyle, -NH-(C=O)-C₁₋₄alkyle, -NH-(SO₂)-C₁₋₄alkyle, -N(CH₃)-C₁₋₄alkyl-SO₂-CH₃, -NH-C₁₋₄alkyl-SO₂-CH₃,-N(CH₃)-C₁₋₄alkyl-OH, -(C=O)-NH-C₁₋₄alkyl-OH, -O-(C=O)-CH(NH₂)-C₁₋₄alkyle, -O-(C=O)-CH(NH₂)-C₁₋₄alkyl-Ar, 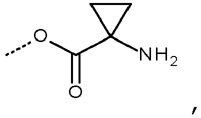 -NH-C₁₋₄alkyl-OH, Het¹, -O-C(=O)-C₁₋₄alkyl-Het¹, -C(=O)-Het¹, et -NH-C(=O)-Het¹ ;
R^{q} représentant Het¹, fluoro, -OH, -NH₂, -O-(C=O)-C₁₋₄alkyle, -NH-(C=O)-C₁₋₄alkyle, -NH-(SO₂)-C₁₋₄alkyle, -N(CH₃)-C₁₋₄alkyl-SO₂-CH₃, -NH-C₁₋₄alkyl-SO₂-CH₃, -N(CH₃)-C₁₋₄alkyl-OH, -O-(C=O)-CH(NH₂)-C₁₋₄alkyle, -O- (C=O)-CH (NH₂)-C₁₋₄alkyl-Ar, 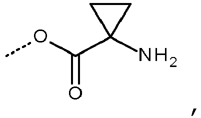 ou-NH-C₁₋₄alkyl-OH ;
Ar représentant phényle éventuellement substitué par un hydroxy ;
R^{4a} représentant hydrogène, C₁₋₄alkyle, Het^{a}, ou C₁₋₄alkyle substitué par un ou plusieurs substituants chacun indépendamment choisi dans le groupe constitué par -OH, -NR⁵R⁶ et Het^{a} ;
R^{4b} représentant hydrogène, halogéno, C₁₋₄alkyle, ou C₁₋₄alkyle substitué par un ou plusieurs substituants halogéno ;
ou R^{4a} et R^{4b} étant pris ensemble pour former, conjointement avec le cycle phényle auquel ils sont fixés, une structure de formule (a-1), (a-2), (a-3), (a-4) ou (a-5): 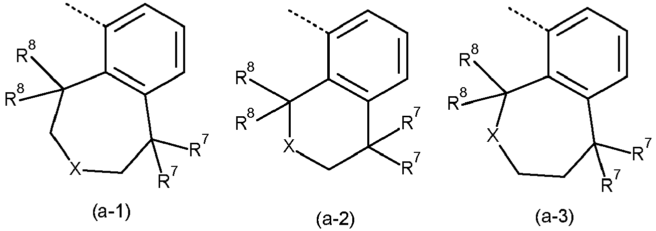 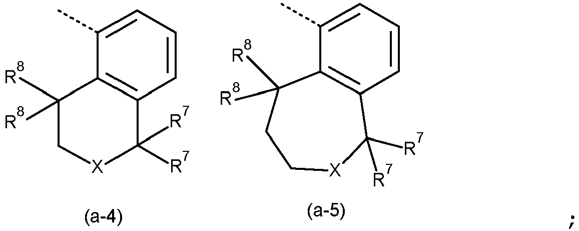
X représentant -NH-, -O-, -N(C₁₋₃alkyle)-, ou-N(hydroxyC₁₋₃alkyle)- ;
les deux substituants R⁷ étant identiques et étant choisis dans le groupe constitué par hydrogène, fluoro et méthyle ; ou les deux substituants R⁷ étant pris ensemble pour former, conjointement avec l'atome de carbone commun auquel ils sont fixés, un cyclopropyle, un cyclobutyle ou un oxétanyle ;
les deux substituants R⁸ étant identiques et étant choisis dans le groupe constitué par hydrogène et méthyle ; ou les deux substituants R⁸ étant pris ensemble pour former, conjointement avec l'atome de carbone commun auquel ils sont fixés, un cyclopropyle, un cyclobutyle ou un oxétanyle ;
R⁵ représentant hydrogène, C₁₋₆alkyle, ou C₁₋₆alkyle substitué par un -OH ;
R⁶ représentant hydrogène, C₁₋₆alkyle, ou C₁₋₆alkyle substitué par un -OH ;
Het¹ représentant un hétérocyclyle saturé à 4, 5 ou 6 chaînons contenant au moins un hétéroatome chacun indépendamment choisi parmi O, S, S(=O)ₚ et N ; ledit hétérocyclyle saturé à 4, 5 ou 6 chaînons étant éventuellement substitué par un ou deux substituants chacun indépendamment choisi dans le groupe constitué par halogéno, -NH₂, C₁₋₄alkyle, -S(=O)₂-C₁₋₆alkyle, -C₁₋₄alkyl-S(=O)₂-C₁₋₆alkyle, hydroxyle, C₁₋₄alkyloxy, fluoro, cyano et C₁₋₄alkyle substitué par un hydroxy ; ou deux substituants sur le même atome de carbone dudit hétérocyclyle saturé à 4, 5 ou 6 chaînons étant pris ensemble pour former, conjointement avec l'atome de carbone commun auquel ils sont fixés, un cycle A ;
le cycle A représentant cyclobutyle, cyclopentyle, cyclohexyle ou un hétérocyclyle saturé à 4, 5 ou 6 chaînons contenant au moins un hétéroatome chacun indépendamment choisi parmi O, S, S(=O)ₚ et N ; ledit cyclobutyle, cyclopentyle, cyclohexyle ou hétérocyclyle saturé à 4, 5 ou 6 chaînons étant éventuellement substitué par un ou deux substituants C₁₋₄alkyle, par un C₁₋₄alkyle et un substituant hydroxy, ou par un substituant hydroxy ;
chaque Het^{a} représentant indépendamment un hétérocyclyle saturé à 4, 5 ou 6 chaînons contenant au moins un hétéroatome chacun indépendamment choisi parmi O, S, S(=O)ₚ et N ; ledit hétérocyclyle saturé à 4, 5 ou 6 chaînons étant éventuellement substitué par un ou deux substituants chacun indépendamment choisi dans le groupe constitué par C₁₋₄alkyle, -S(=O)₂C₁₋₆alkyle, hydroxy, -C₁₋₄alkyl-S(=O)₂-C₁₋₆alkyle, et C₁₋₄alkyle substitué par un hydroxy ; ou deux substituants sur le même atome de carbone dudit hétérocyclyle saturé à 4, 5 ou 6 chaînons étant pris ensemble pour former, conjointement avec l'atome de carbone commun auquel ils sont fixés, un cycle B ;
le cycle B représentant cyclobutyle, cyclopentyle, cyclohexyle ou un hétérocyclyle saturé à 4, 5 ou 6 chaînons contenant au moins un hétéroatome chacun indépendamment choisi parmi O, S, S(=O)ₚ et N ; ledit cyclobutyle, cyclopentyle, cyclohexyle ou hétérocyclyle saturé à 4, 5 ou 6 chaînons étant éventuellement substitué par un ou deux substituants C₁₋₄alkyle, par un C₁₋₄alkyle et un substituant hydroxy, ou par un substituant hydroxy ;
p représentant 1 ou 2 ;
ou N-oxyde, sel d'addition pharmaceutiquement acceptable ou solvate correspondant.

2. Composé selon la revendication 1,
R³ représentant C₁₋₄alkyle ; C₁₋₄alkyle substitué sur le même atome de carbone par un -OH et par un Het¹; ou C₁₋₄alkyle substitué par un substituant choisi dans le groupe constitué par fluoro, -OH,-NH₂, -O-(C=O)-C₁₋₄alkyle, -(C=O)-O-C₁₋₄alkyle, -NH-(C=O)-C₁₋₄alkyle, -NH-(SO₂)-C₁₋₄alkyle, -N(CH₃)-C₁₋₄alkyl-SO₂-CH₃, -NH-C₁₋₄alkyl-SO₂-CH₃, -N(CH₃)-C₁₋₄alkyl-OH, -(C=O)-NH-C₁₋₄alkyl-OH, -O(C=O)-CH(NH₂)-C₁₋₄alkyle, -O-(C=O)-CH(NH₂)-C₁₋₄alkyl-Ar, 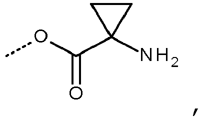 -NH-C₁₋₄alkyl-OH, Het¹, -O-C(=O)-C₁₋₄alkyl-Het¹, -C(=O)-Het¹, et -NH-C(=O)-Het¹ ;
R^{4a} représentant hydrogène, C₁₋₄alkyle, ou C₁₋₄alkyle substitué par un ou plusieurs substituants chacun indépendamment choisi dans le groupe constitué par -OH, et -NR⁵R⁶ ;
R^{4b} représentant hydrogène, halogéno, C₁₋₄alkyle, ou C₁₋₄alkyle substitué par un ou plusieurs substituants halogéno ;
ou R^{4a} et R^{4b} étant pris ensemble pour former, conjointement avec le cycle phényle auquel ils sont fixés, une structure de formule (a-1), (a-2), (a-3), (a-4) ou (a-5) ;
les deux substituants R⁷ étant hydrogène ;
les deux substituants R⁸ étant hydrogène.

3. Composé selon la revendication 1,
R¹ représentant hydrogène ou -NH₂ ;
R² représentant 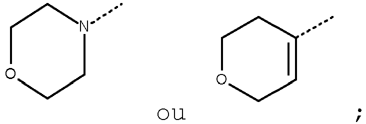
R³ représentant C₁₋₄alkyle ; ou C₁₋₄alkyle substitué par un substituant choisi dans le groupe constitué par -OH et Het¹ ;
R^{4a} représentant C₁₋₄alkyle ;
R^{4b} représentant halogéno, C₁₋₄alkyle, ou C₁₋₄alkyle substitué par un ou plusieurs substituants halogéno ;
ou R^{4a} et R^{4b} étant pris ensemble pour former, conjointement avec le cycle phényle auquel ils sont fixés, une structure de formule (a-2) ;
X représentant -N(C₁₋₃alkyle)-, ou -N(hydroxyC₁₋₃alkyle)- ;
les deux substituants R⁷ étant hydrogène ;
les deux substituants R⁸ étant hydrogène ;
Het¹ représentant un hétérocyclyle saturé à 4, 5 ou 6 chaînons contenant au moins un hétéroatome chacun indépendamment choisi parmi S(=O)ₚ et N ; ledit hétérocyclyle saturé à 4, 5 ou 6 chaînons étant éventuellement substitué par un ou deux substituants hydroxyle ; ou deux substituants sur le même atome de carbone dudit hétérocyclyle saturé à 4, 5 ou 6 chaînons étant pris ensemble pour former, conjointement avec l'atome de carbone commun auquel ils sont fixés, un cycle A ;
le cycle A représentant cyclobutyle éventuellement substitué par un substituant hydroxy ;
p représentant 2.

4. Composé selon la revendication 1,
X représentant -N(C₁₋₃alkyle)-, ou -N(hydroxyC₁₋₃alkyle)-.

5. Composé selon la revendication 1,
R³ représentant C₁₋₄alkyle ; ou C₁₋₄alkyle substitué par un substituant choisi dans le groupe constitué par -OH et Het¹.

6. Composé selon la revendication 1,
R^{4a} représentant hydrogène, C₁₋₄alkyle, Het^{a}, ou C₁₋₄alkyle substitué par un ou plusieurs substituants chacun indépendamment choisi dans le groupe constitué par -OH, -NR⁵R⁶ et Het^{a} ;
R^{4b} représentant hydrogène, halogéno, C₁₋₄alkyle, ou C₁₋₄alkyle substitué par un ou plusieurs substituants halogéno.

7. Composé selon la revendication 6,
R^{4a} représentant C₁₋₄alkyle ;
R^{4b} représentant C₁₋₄alkyle substitué par un ou plusieurs substituants halogéno.

8. Composé selon la revendication 1,
les deux substituants R⁷ étant hydrogène ; et
les deux substituants R⁸ étant hydrogène.

9. Composé selon la revendication 1,
R² représentant

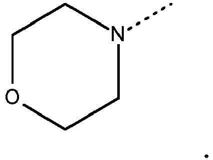

10. Composé selon la revendication 1, R¹ représentant hydrogène.

11. Composition pharmaceutique comprenant un support pharmaceutiquement acceptable et, en tant qu'ingrédient actif, une quantité thérapeutiquement efficace d'un composé selon l'une quelconque des revendications 1 à 10.

12. Composé tel que défini selon l'une quelconque des revendications 1 à 10 pour une utilisation en tant que médicament.

13. Composé tel que défini selon l'une quelconque des revendications 1 à 10 pour une utilisation dans le traitement ou la prévention d'une maladie ou d'une affection choisie parmi un cancer, des troubles auto-immuns, des maladies cardiovasculaires, des maladies inflammatoires, des maladies neurodégénératives, une allergie, une pancréatite, l'asthme, une défaillance multiviscérale, des maladies du rein, une agrégation plaquettaire, la motilité du sperme, un rejet de transplantation, un rejet de greffe, et des lésions pulmonaires.

14. Composé pour une utilisation selon la revendication 13, la maladie ou l'affection étant un cancer.

15. Composé pour une utilisation selon la revendication 14, la maladie ou l'affection étant un cancer de la prostate.
